# FASCICULE DE BREVET EUROPEEN

(11) **EP 0 597 088 B1**
(45) Date de publication et mention de la délivrance du brevet: **06.12.2000**
(21) Numéro de dépôt: 93914482.0
(22) Date de dépôt: 08.01.1993
(51) Int. Cl.: C07D 233/64, C07D 233/84, C07D 403/12, C07D 401/12, C07D 417/12, A61K 31/415

(54) **NOUVEAUX DERIVES DE L'IMIDAZOLE, LEUR PREPARATION ET LEURS APPLICATIONS THERAPEUTIQUES**
NEUE IMIDAZOL DERIVATE, IHRE HERSTELLUNG UND THERAPEUTISCHE VERWENDUNG
NOVEL IMIDAZOLE DERIVATIVES, THEIR PREPARATION AND THERAPEUTIC APPLICATIONS

(30) Priorité: 10.01.1992 FR 9200189
(43) Date de publication de la demande: 18.05.1994
(73) Titulaire: INSTITUT NATIONAL DE LA SANTE ET DE LA RECHERCHE MEDICALE (INSERM), 75654 Paris Cédex 13 (FR); SOCIETE CIVILE BIOPROJET, F-75003 Paris (FR)
(72) Inventeur: SCHWARTZ, Jean-Charles, F-75014 Paris (FR); ARRANG, Jean-Michel, 160, av. du Général-Leclerc, F-91190 Gif-sur-Yvette (FR); GARBARG, Monique, F-75017 Paris (FR); LECOMTE, Jeanne-Marie, F-75003 Paris (FR); GANELLIN, Charon, Robin, Welwyn Hertfordshire AL6 0TD (GB); FKYERAT, Abdellatif, F-74100 ANNEMASSE (FR); TERTIUK, Wasyl 81 The Commons, Hertfordshire AL7 4RZ (GB); SCHUNACK, Walter, D-1000 Berlin 38 (DE); LIPP, Ralph, D-1000 Berlin 30 (DE); STARK, Holger, D-1000 Berlin 30 (DE); PURAND, Katja, D-1000 Berlin 31 (DE)
(74) Mandataire: Bernasconi, Jean
(86) Numéro de dépôt international: FR9300015
(87) Numéro de publication internationale: WO9314070

(56) Documents cités:
- EP-A- 0 041 359
- EP-A- 0 054 865
- EP-A- 0 081 324
- EP-A- 0 199 845
- EP-A- 0 262 448
- EP-A- 0 291 172
- EP-A- 0 302 164
- EP-A- 0 315 316
- EP-A- 0 338 939
- WO-A-87/07891
- AU-B- 514 574
- DE-C- 276 541
- DE-C- 332 955
- FR-A- 1 220 002
- FR-A- 2 100 822
- GB-A- 1 305 547
- GB-A- 1 305 548
- GB-A- 1 305 549
- GB-A- 1 341 375
- GB-A- 1 531 221
- US-A- 2 301 532
- US-A- 2 376 424
- Eur. J. Med. Chem. (1994), Vol. 29, 695-700

## Description

La présente invention concerne de nouveaux dérivés de l'imidazole, leur préparation ainsi que leurs applications thérapeutiques.

Les dérivés de l'imidazole conformes à l'invention présentent d'utiles propriétés antagonistes des récepteurs H₃ de l'histamine contrôlant la libération et la synthèse de l'histamine. Leur activité antagoniste sur les récepteurs H₃ les rend utiles en thérapeutique, en particulier comme médicament à effets sédatifs, régulateur du sommeil, anticonvulsivant, psychostimulant, modulateur de la circulation cérébrale, anti-ulcéreux.

Les dérivés conformes à l'invention répondent à la formule générale IA ou IB 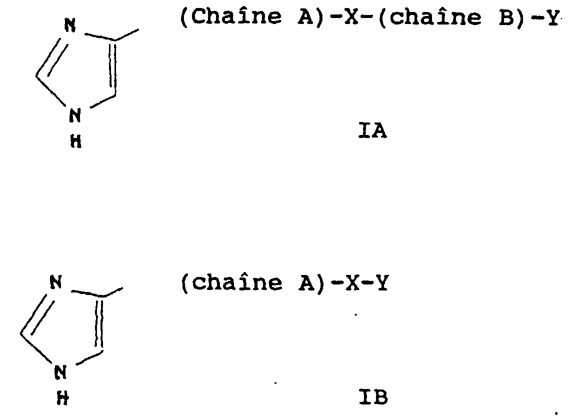 dans laquelle
la chaîne A représente une chaîne hydrocarbonée, droite ou ramifiée, saturée ou insaturée, comportant 1 à 6 atomes de carbone, la chaîne hydrocarbonée saturée pouvant être interrompue par un atome de soufre,
X représente un atome d'oxygène ou de soufre, -O-CO, -CO-O-, -OCONH-, -OCON(alkyle en C₁-C₃)-, -OCONH-CO-, -CONH-, -SO-, -CO-, -CHOH-,
la chaîne B représente une chaîne alkylène droite -(CH₂)ₙ-, n étant un nombre entier pouvant varier entre O et 5 ou une chaîne alkylène ramifiée renfermant de 2 à 8 atomes de carbone, la chaîne alkylène pouvant être interrompue par un ou plusieurs atomes d'oxygène ou de soufre, ou une chaîne -(CH₂)ₘ-O- ou -(CH₂)ₘ-S- où m est un nombre entier égal à 1 ou 2,
Y représente un groupe alkyle droit ou ramifié comportant 1 à 8 atomes de carbone, un cycloalkyle comportant 3 à 6 atomes de carbone, un groupe bicycloalkyle, un groupe cycloalkényle, un groupe naphtyle, un groupe phényle éventuellement substitué, un radical hétérocyclique à 5 ou 6 chaînons comportant un ou deux hétéroatomes choisis parmi les atomes d'azote et de soufre, ledit radical hétérocyclique étant éventuellement substitué, ou encore un radical bicyclique résultant de l'accolement d'un cycle benzénique à un hétérocycle tel que défini précédemment.

La chaîne A peut être une chaîne alkylène droite -(CH₂)ₙ-, n représentant un nombre entier compris entre 1 et 6 atomes de carbone, de préférence entre 1 et 4 atomes de carbone, ou une chaîne alkylène ramifiée, de préférence une chaîne substituée par un ou plusieurs radicaux méthyle ou éthyle.

La chaîne A peut être également une chaîne alkényle droite ou ramifiée, et peut être par exemple le groupe allyle.

Lorsque Y représente un groupe cycloalkyle, celui-ci peut être, par exemple, le cyclopentyle, le cyclohexyle, ou un groupe bicycloalkyle.

Lorsque Y représente un groupe phényle substitué, le groupe phényle peut être mono- ou polysubstitué par un halogène, par un alkyle inférieur, par exemple CH₃, par CF₃, CN, COCH₃, COOR₁, OR₁, R₁ représentant un alkyle inférieur, par exemple COOCH₃, le groupement NO₂, le groupement NR₂R₃, R₂ et R₃ représentant un atome d'hydrogène et/ou un radical alkyle inférieur (par "alkyle inférieur", on entend un radical alkyle comportant au plus 6 atomes de carbone).

Lorsque Y représente un radical hétérocyclique, celui-ci peut être, par exemple, le radical pyridyle, le radical pyridyle N-oxyde ou le radical pyrazinyle, éventuellement mono- ou polysubstitué par NO₂, CF₃, CH₃, NH₂, un halogène tel que Cl, le groupe COOCH₃ ou encore le radical imidazolyle ou le radical thiazyle.

Lorsque Y représente un radical bicyclique résultant de l'accolement d'un cycle benzénique à un hétérocycle, ce radical peut être par exemple le radical benzothiazyle.

Les composés qui répondent à la formule IA ou IB sont des composés nouveaux, à l'exclusion toutefois :
a) des composés dans lesquels X représente un atome d'oxygène, la chaîne A un groupe -CH₂- et Y un groupe phényle substitué (formule IB) ;
b) des composés dans lesquels X représente le groupe -CO-, la chaîne A le groupe -(CH₂)₂- et Y un radical naphtyle ou un phényle éventuellement substitué, un radical hétérocyclique à 5 chaînons comportant le soufre comme hétéroatome et éventuellement substitué, un radical bicyclique résultant de l'accolement d'un cycle hengénique à un hétérocycle à 5 ou 6 chaînons, comportant comme hétéroatomes les atomes d'azote et/ou de soufre (formule IB) ;
c) des composés dans lesquels X représente le groupe -CONH-, la chaîne A le groupe -(CH₂)₂- et Y le groupe phényle éventuellement substitué (formule IB) ;
d) des composés dans lesquels X représente le groupe -CO-, la chaîne A le groupe méthylène et B et Y simultanément -C(CH₃)₂-(CH₂)ₚ- (p = 0 ou 1) et un groupe naphtyle ou un groupe phényle éventuellement substitué.

Les composés énoncés sous a) ont été décrits dans le brevet français n° 1 220 002 ; les composés énoncés sous b) dans la demande de brevet européen EP-A-0 291 172 ; les composés énoncés sous c) dans la demande de brevet européen EP-A-315 316 ; les composés énoncés sous d) dans la demande de brevet européen EP-A-0 054 865. Aucun de ces composés n'est présenté comme ayant des propriétés antagonistes des récepteurs H₃.

La présente invention concerne également les sels d'addition que forment les composés de formule IA ou IB avec des acides pharmaceutiquement acceptables. Les sels pharmaceutiquement acceptables comprennent les sels non toxiques d'acides minéraux ou organiques tels que le chlorhydrate, le bromhydrate ou le maléate.

La présente invention englobe également les hydrates des composés de formule IA ou IB, les sels hydratés de ces composés et les structures cristallines polymorphiques. Il faut, par ailleurs, noter que la structure des composés conformes à l'invention, telle qu'elle est illustrée par les formules IA et IB ne représente que l'une des formes tautomères possibles de ces composés et que ceux-ci peuvent se présenter sous d'autres formes tautomères. La présente invention englobe donc également toutes les formes tautomères possibles des composés en question, que ces tautomères se présentent sous forme isolée ou sous forme de mélanges.

Les composés de formule IA ou IB peuvent exister sous une ou plusieurs formes d'isomères selon le nombre de centres asymétriques de la molécule. L'invention concerne donc aussi bien tous les isomères optiques que leurs mélanges racémiques et les diastéréoisomères correspondants. La séparation des diastéréoisomères et/ou des isomères optiques peut s'effectuer selon des méthodes connues en soi.

Les composés de formule IA ou IB dans lesquelles X représente -OCO-, la chaîne A, la chaîne B et Y ayant les significations précitées, peuvent être obtenus en faisant réagir un chlorure d'acide de formule

ClCO-(chaîne B)-Y

ClCO-Y

sur un alcool, se présentant par exemple sous forme de chlorhydrate, de formule 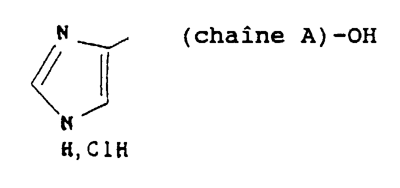 en présence d'un solvant tel que la pyridine.

Les composés de formule IA ou IB dans lesquelles X représente -CO-O-, la chaîne A, la chaîne B et Y ayant les significations précitées, peuvent être obtenus en faisant réagir un acide de formule 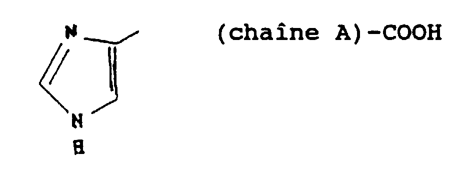 avec un alcool de formule

HO - (chaîne B) - Y

ou

HO - Y,

en présence de chlorure de thionyle.

Les composés de formule IA ou IB dans lesquelles X représente -OCONH-, la chaîne A, la chaîne B et Y ayant les significations précitées, peuvent être obtenus en faisant réagir un alcool, se présentant par exemple sous forme de chlorhydrate, de formule 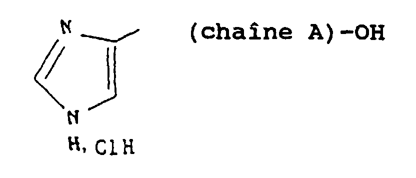 avec un isocyanate de formule

OCN - (chaîne B)-Y

ou

OCN - Y

en présence d'un solvant apolaire.

Les composés de formule IA ou IB dans lesquelles X représente -O-, la chaîne A, la chaîne B et Y ayant les significations précitées, peuvent être obtenus en faisant réagir un alcoolate de formule 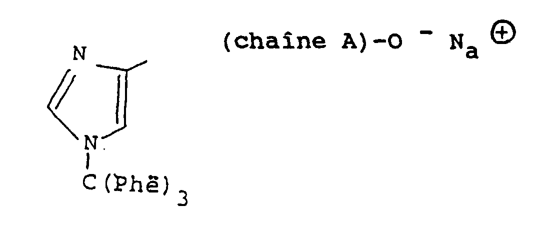 Phe désignant le radical phényle, avec un composé halogéné de formule

Hal - (chaîne B) - Y

ou

Hal - Y

Hal désignant un halogène, en présence d'un solvant neutre tel que le toluène, puis en clivant le groupement -C(Phe)₃ par une solution acide.

Les composés de formule IA ou IB dans lesquelles X représente -O-, la chaîne A, la chaîne B et Y ayant les significations précitées, peuvent être encore obtenus en faisant réagir un composé halogéné, se présentant par exemple sous forme de chlorhydrate, de formule 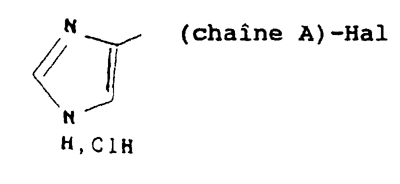 Hal désignant un halogène tel que le chlore, avec un alcool de formule

HO - (chaîne B) - Y

ou

HO - Y,

Les composés de formule IB dans laquelle X représente -O-, la chaîne A a la signification précitée et Y représente un groupe phényle éventuellement substitué peuvent être encore obtenus en faisant réagir un alcool de formule 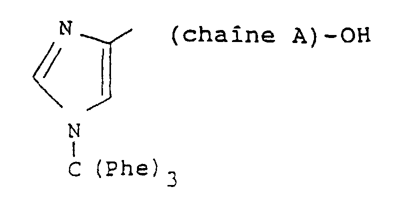 avec un composé phénolique de formule 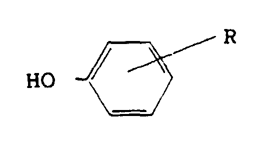 dans laquelle R représente un substituant tel qu'un halogène, un alkyle inférieur, CF₃, CN, COCH₃, en présence de triphénylphosphine et de diéthylazodicarboxylate dans un solvant et en clivant le groupe -C(Phe)₃ par traitement par une solution acide.

Les composés de formule IA ou IB dans lesquelles X représente -S-, la chaîne A, la chaîne B et Y ayant les significations précitées, peuvent être obtenus en faisant réagir une isothiourée de formule 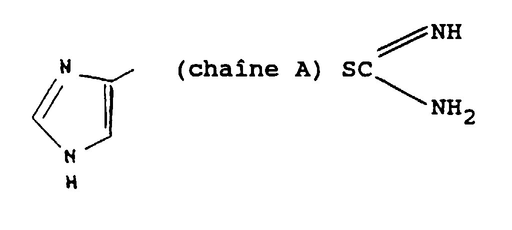 avec un composé halogéné de formule

Hal - (chaîne B) - Y

ou

Hal - Y,

Hal désignant un halogène tel que le chlore, en présence d'un solvant tel que l'éthanol.

Les composés de formule IA ou IB dans lesquelles X représente -S-, la chaîne A, la chaîne B et Y ayant les significations précitées, peuvent être encore obtenus en faisant réagir un alcool de formule 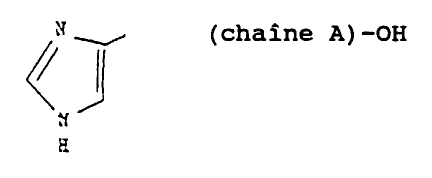 avec un composé de formule

SH - (chaîne B) - Y

ou

SH - Y

en présence d'un hydracide halogéné tel que l'acide bromhydrique ou
en faisant réagir un composé halogéné de formule 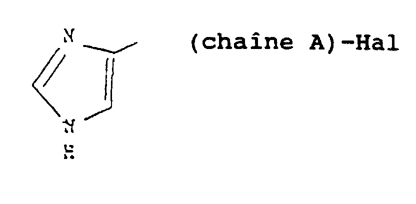 Hal désignant un halogène, avec un composé de formule

SH - (chaîne B) - Y

ou

SH - Y,

en présence d'une base telle qu'un hydroxyde alcalin.

Les composés de formule IA ou IB dans lesquelles X représente -SO-, la chaîne A, la chaîne B et Y ayant les significations précitées, peuvent être obtenus en traitant un composé de formule 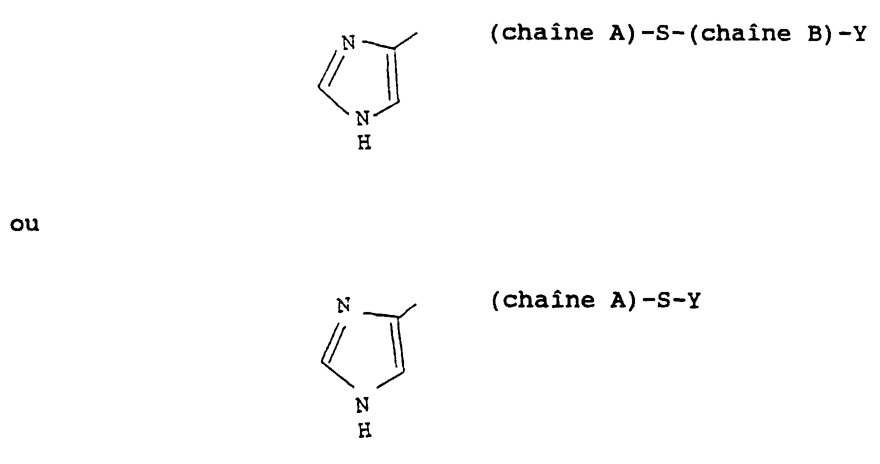 par une base telle qu'un hydroxyde ou un carbonate de métal alcalin ou alcalino-terreux, en présence d'un solvant, puis par un agent d'oxydation.

Les composés de formule IA ou IB dans lesquelles X représente -CO-, la chaîne A, la chaîne B et Y ayant les significations précitées, peuvent être obtenus par réaction d'un composé de formule 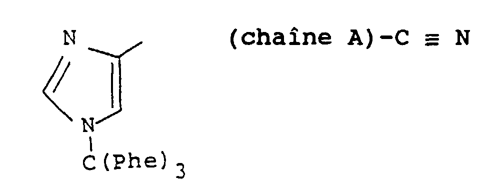 Phe désignant le radical phényle, avec

Hal - Mg - (chaîne B) - Y

ou

Hal - Mg - Y

Hal désignant un halogène, en présence d'un solvant, puis par hydrolyse du produit obtenu.

Les composés de formule IA ou IB dans lesquelles X représente - CHOH -, la chaîne A, la chaîne B et Y ayant les significations précitées, peuvent être obtenus en réduisant le composé de formule 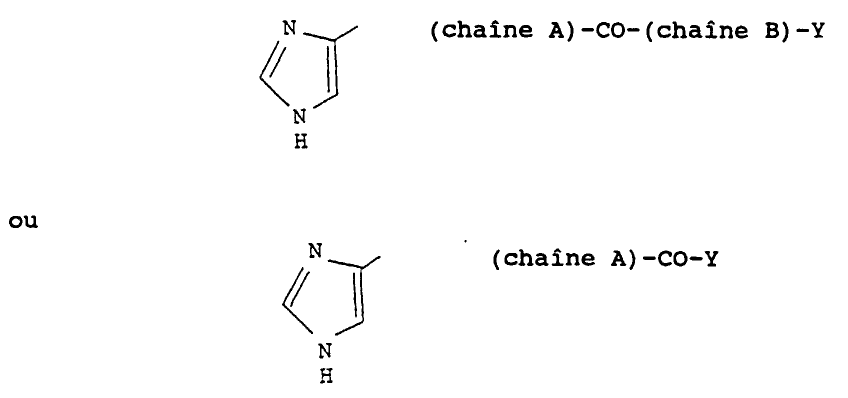 par exemple par un hydrure, en présence d'un solvant, puis par hydrolyse avec une solution basique.

Les exemples qui sont donnés ci-après, à titre non limitatif, illustrent la présente invention.

### Exemple 1 (ne fait pas partie de l'invention)

### N-((1H-imidazol-4-yl) méthyl)-5-phénylpentanamide

On introduit successivement 8 mmol de N, N'-carbonyldiimidazole et 8 mmol d'acide 5-phénylpentanoïque dans 1O ml de THF absolu et, après avoir agité 3O mn, on ajoute au mélange 8 mmol de (1H-imidazol-4-yl) méthanamine, à l'abri de l'humidité en utilisant CaCl₂. Au bout de 14 h, on élimine le solvant par distillation sous vide. On mélange l'huile restante avec un peu d'eau. Le composé du titre est filtré sous vide, séché et cristallisé dans éthanol/diéthyléther.
C₁₅H₁₉N₃O(257,4) F : 125-137°C rendement : 35 %

| Analyse élémentaire : | | | |
|---|---|---|---|
| calculé | C 7O,O | H 7,44 | N 16,3 |
| trouvé | C 7O,O | H 7,52 | N 16,3 |

### Exemple 20

### N-(3-phénylpropyl)-3-(1H-imidazol-4-yl)-propanamide

La préparation s'effectue comme dans l'exemple 1 avec
la 3-phénylpropanamine et l'acide 3-(1H-imidazol-4-yl) propanoïque, l'acide étant ici ajouté après la réaction du composant amine avec le N,N'-carbonyldiimidazole. Le composé du titre est extrait de l'eau par du diéthyléther, purifié par chromatographie et recristallisé sous forme d'hydrogénomaléate dans éthanol/diéthyléther.
C₁₅H₁₉N₃O.C₄H₄O₄.O,5 H₂O (382,4) F : 116°C rendement 45 %

| Analyse élémentaire : | | | |
|---|---|---|---|
| calculé | C 59,7 | H 6,33 | N 11,O |
| trouvé | C 59,8 | H 6,O9 | N 1O,8 |

### Exemple 23 (ne fait pas partie de l'invention)

### N-benzyl-N'-(3-(1H-imidazol-4-yl)propyl) urée

On mélange 5 mmol de dichlorhydrate de 3-(1H-imidazol-4-yl)propanamine et 1O mmol de triéthylamine dans 1O ml d'acétonitrile et, après ajout de 5 mmol d'isocyanate de benzyle, on porte le tout à l'ébullition 1 h au reflux. Après évaporation sous vide, le composé du titre est repris dans un peu d'eau et recristallisé sous forme d'hydrogénomaléate dans éthanol/diéthyléther.
C₁₄H₁₈N₄O.C₄H₄O₄ (374,4) F : 119°C rendement : 8O %

| Analyse élémentaire : | | | |
|---|---|---|---|
| calculé | C 57,7 | H 5,92 | H 15,O |
| trouvé | C 57,7 | H 6,O5 | H 14,6 |

### Exemple 24

### Ester 3-(1H-imidazol-4-yl)propylique de l'acide 3-cyclopentylpropanoïque

On additionne 5 mmol de chlorhydrate de 3-(1H-imidazol-4-yl)propanol dans 3O ml de pyridine avec 1O mg de 4-diméthylaminopyridine à 5 mmol du chlorure d'acide 3-cyclopentylpropanoïque et on agite le tout pendant 16 h à température ambiante. La solution est évaporée sous vide, puis le résidu est repris dans de l'eau et extrait par du diéthyléther.Un hydrogénomaléate est formé à partir de l'huile obtenue, décoloré avec du charbon actif et recristallisé dans éthanol/diéthyléther.
C₁₄H₂₂N₂O₂.C₄H₄O₄ (366,4) F : 88°C rendement : 3O %

| Analyse élémentaire : | | | |
|---|---|---|---|
| calculé | C 59,O | H 7,15 | N 7,65 |
| trouvé | C 59,O | H 7,39 | N 7,65 |

### Exemple 25

### Ester 3-(1H-imidazol-4-yl)propylique de l'acide benzoïque

La préparation est effectuée comme dans l'exemple 24 avec du chlorure de l'acide benzoïque. Le composé du titre est cristallisé sous forme d'hydrogénomaléate dans éthanol/diéthyléther.
C₁₃H₁₄N₂O₂.C₄H₄O₄.0,5H₂O(355,4) F : 165°C rendement : 75 %

| analyse élémentaire : | | | |
|---|---|---|---|
| calculé | C 57,5 | H 5,39 | N 7,88 |
| trouvé | C 57,1 | H 5,07 | N 7,95 |

### Exemple 26

### Ester 3-(1H-imidazol-4-yl)propylique de l'acide 4-iodobenzoïque

La préparation est effectuée comme dans l'exemple 24 avec du chlorure de l'acide 4-iodobenzoïque. Le composé du titre est cristallisé sous forme d'hydrogénomaléate dans éthanol/diéthyléther.
C₁₃H₁₃N₂O₂I.C₄H₄O₄.0,5H₂O(481,2) F : 148°C rendement : 60 %

| analyse élémentaire : | | | |
|---|---|---|---|
| calculé | C 42,4 | H 3,77 | N 5,82 |
| trouvé | C 42,5 | H 3,52 | N 5,89 |

### Exemple 27

### Ester 3-(1H-imidazol-4-yl)propylique de l'acide 3-iodobenzoïque

La préparation est effectuée comme dans l'exemple 24 avec du chlorure de l'acide 3-iodobenzoïque. Le composé du titre est cristallisé sous forme d'hydrogénomaléate dans éthanol/diéthyléther.
C₁₃H₁₃N₂O₂I.C₄H₄O₄.0,25H₂O (481,2) F : 105°C rendement : 7O %

| Analyse élémentaire : | | | |
|---|---|---|---|
| calculé | C 42,4 | H 3,77 | N 5,82 |
| trouvé | C 42,3 | H 3,52 | N 5,78 |

### Exemple 28

### Ester 3-(1H-imidazol-4-yl)propylique de l'acide 3-iodo-4-méthylbenzoïque

La préparation est effectuée comme dans l'exemple 24 avec du chlorure de l'acide 3-iodo-4-méthylbenzoique. Le composé du titre est cristallisé sous forme d'hydrogénomaléate dans éthanol/diéthyléther.
C₁₄H₁₅N₂O₂I.C₄H₄O₄ (486,3) F : 112-133°C rendement : 70 %

| analyse élémentaire : | | | |
|---|---|---|---|
| calculé | C 44,5 | H 3,94 | N 5,76 |
| trouvé | C 44,5 | H 4,09 | N 5,88 |

### Exemple 29

### Ester 3-(1H-imidazol-4-yl)propylique de l'acide 3-(4-iodophényl)propylique

La préparation est effectuée comme dans l'exemple 24 avec du chlorure de l'acide 3-(4-iodophényl)propylique. Le composé du titre est cristallisé sous forme d'hydrogénomaléate dans éthanol/diéthyléther.
C₁₅H₁₇N₂O₂I.C₄H₄O (500,29) F : 147°C rendement : 80 %

| analyse élémentaire : | | | |
|---|---|---|---|
| calculé | C 45,6 | H 4,23 | N 5,60 |
| trouvé | C 45,9 | H 4,29 | N 5,68 |

### Exemple 30

### Ester 3-(1H-imidazol-4-yl)propylique de l'acide 4-amino-3,5-diiodobenzoïque

La préparation est effectuée comme dans l'exemple 24 avec du chlorure de l'acide 4-amino-3,5-diiodobenzoïque. Le composé du titre est cristallisé sous forme d'hydrogénomaléate dans éthanol/diéthyléther.
C₁₃H₁₃N₃O₂I₂.C₄H₄O₄ (613,2) F : 155°C rendement : 75 %

| analyse élémentaire : | | | |
|---|---|---|---|
| calculé | C 33,3 | H 2,79 | N 6,85 |
| trouvé | C 33,1 | H 2,60 | N 6,83 |

### Exemple 31

### Ester 3-(1H-imidazol-4-yl)propylique de l'acide 4-(4-iodophényl)butanoïque

La préparation est effectuée comme dans l'exemple 24 avec du chlorure de l'acide 4-(4-iodophényl)butanoïque. Le composé du titre est cristallisé sous forme d'hydrogénomaléate dans éthanol/diéthyléther.
C₁₆H₁₉N₂O₂I.₄H₄O₄ (514,3) F : 126°C rendement : 80 %

| analyse élémentaire : | | | |
|---|---|---|---|
| calculé | C 46,7 | H 4,51 | N 5,45 |
| trouvé | C 46,7 | H 4,65 | N 5,31 |

### Exemple 32

### Ester 3-(1H-imidazol-4-yl)propylique de l'acide 2-(4-iodophényl)éthanoïque

La préparation est effectuée comme dans l'exemple 24 avec du chlorure de l'acide 2-(4-iodophényl)éthanoïque. Le composé du titre est cristallisé sous forme d'hydrogénomaléate dans éthanol/diéthyléther.
C₁₄H₁₅N₂O₂I.C₄H₄O₄ (486,3) F : 88°C rendement : 60 %

| analyse élémentaire : | | | |
|---|---|---|---|
| calculé | C 44,5 | H 3,93 | N 5,76 |
| trouvé | C 44,7 | H 3,99 | N 5,55 |

### Exemple 33

### Ester 3-(1H-imidazol-4-yl)propylique de l'acide 4-phénylbutanoïque

La préparation est effectuée comme dans l'exemple 24 avec du chlorure de l'acide 4-phénylbutanoïque. Le compose du titre est cristallisé sous forme d'hydrogénomaléate dans éthanol/diéthyléther.
Ç₁₆H₂₀N₂O₂C₄H₄O₄.0,5H₂O (397,4) F : 88°C rendement : 60 %

| analyse élémentaire : | | | |
|---|---|---|---|
| calculé | C 60,4 | H 6,34 | N 7,05 |
| trouvé | C 60,6 | H 6,19 | N 7,20 |

### Exemple 34

### N-benzyl-3-(1H-imidazol-4-yl)propyloxycarbamide

La préparation est effectuée comme dans l'exemple 23 avec du chlorhydrate de 3-(1H-imidazol-4-yl)propanol et 5 mmol de triéthylamine. L'huile restante est reprise dans un peu d'eau. Le composé du titre est cristallisé sous forme d'hydrogénomaléate dans éthanol/diéthyléther.
C₁₄H₁₇N₃O₂.C₄H₄O₄ (375,4) F : 123°C rendement : 90 %

| analyse élémentaire : | | | |
|---|---|---|---|
| calculé | C 57,6 | H 5,64 | N 11,2 |
| trouvé | C 57,2 | H 5,63 | N 11,2 |

### Exemple 35

### N-cyclohexylméthyl-3-(1H-imidazol-4-yl) propyloxycarbamide

La préparation s'effectue comme dans l'exemple 34 avec du 3-(1H-imidazol-4-yl)propanolate de sodium. L'huile restante est reprise dans un peu d'eau. Le composé du titre est filtré sous vide, séché et recristallisé sous forme d'hydrogénomaléate dans éthanol/diéthyléther.
C₁₄H₂₃N₃O₂.C₄H₄O₄ (381,4) F : 1O6°C rendement : 4O %

| Analyse élémentaire : | | | |
|---|---|---|---|
| calculé | C 56,7 | H 7,13 | N 11,O |
| trouvé | C 56,3 | H 7,33 | N 11,O |

### Exemple 36

### (3-cyclohexylpropyl)-3-(1H-imidazol-4-yl)propyléther

On mélange 5mmol de 3-(1-triphénylméthyl-imidazol-4-yl)propanolate de sodium dans 1O ml de toluène contenant O,5 mmol de 15-Crown-5 et 5 mmol de (3-chloropropyl)cyclohexane et on agite 24 h à 75°C. On concentre la suspension sous vide, on dissout le produit obtenu dans du diéthyléther, on filtre et on lave avec de l'éther de pétrole (4O-6O°C). Le filtrat obtenu est concentré et porté à l'ébullition 1 h dans une solution hydro-alcoolique (éthanol-eau) d'acide chlorhydrique 2N. L'éthanol est éliminé sous un vide faible et le précipité est filtré sous vide. La solution est rendue alcaline, extraite par du dichlorométhane et la phase organique est concentrée. Le composé du titre obtenu est recristallisé sous forme d'hydrogénomaléate dans éthanol/diéthyléther.
C₁₅H₂₆N₂O.C₄H₄O₄.O,25 H₂O (37O,9) F : 114-116°C
rendement : 35 %

| Analyse élémentaire : | | | |
|---|---|---|---|
| calculé | C 61,52 | H 8,29 | N 7,55 |
| trouvé | C 61,6O | H 8,25 | N 7,5O |

### Exemple 37

### 3-(3,4-difluorophényl)propyl-3-(1H-imidazol-4-yl)propyléther

La préparation est effectuée comme dans l'exemple 36 avec du chlorure de 3-(3,4-difluorophényl)propane. Le composé du titre est cristallisé sous forme d'hydrogénomaléate dans éthanol/diéthyléther
C₁₅H₁₈N₂OF₂.C₄H₄0₄(396,4)
F : 101°C
rendement : 25 %

| Analyse élémentaire : | | | |
|---|---|---|---|
| calculé | C 57,6 | H 5,59 | N 7,07 |
| trouvé | C 57,4 | H 5,52 | N 7,14 |

### Exemple 38

### 3-(4-bromophényl)propyl-3-(1H-imidazol-4-yl)propyléther

La préparation est effectuée comme dans l'exemple 36 avec du chlorure de 3-(4-bromophényl)propane. Le composé du titre est cristallisé sous forme d'hydrogénomaléate dans éthanol/diéthyléther
C₁₅H₁₉N₂OBr.C₄H₄0₄(439,3) F : 130-131 °C rendement : 35 %

| Analyse élémentaire : | | | |
|---|---|---|---|
| calculé | C 52,0 | H 5,28 | N 6,38 |
| trouvé | C 52,4 | H 5,35 | N 6,57 |

### Exemple 39

### 3-(3-trifluorométhylphényl)propyl-3-(1H-imidazol-4-yl)propyléther

La préparation est effectuée comme dans l'exemple 36 avec du chlorure de 3-(3-trifluorométhylphényl)propane. Le composé du titre est cristallisé sous forme d'hydrogénomaléate dans éthanol/diéthyléther
C₁₆H₁₉N₂OF₃.C₄H₄0₄(428,4) F : 85 °C rendement : 35 %

| analyse élémentaire : | | | |
|---|---|---|---|
| calculé | C 56,1 | H 5,41 | N 6,54 |
| trouvé | C 55,9 | H 5,44 | N 6,48 |

### Exemple 40

### 1-naphthylméthyl-3-(1H-imidazol-4-yl)propyléther

La préparation est effectuée comme dans l'exemple 36 avec le chlorure de 1-naphtylméthane. Le composé du titre est cristallisé sous forme d'hydrogénomaléate dans éthanol/diéthyléther.
C₁₇H₁₈N₂O.C₄H₄0₄.0,25H₂O(386,9) F : 75 °C rendement : 40%

| Analyse élémentaire : | | | |
|---|---|---|---|
| calculé | C 65,2 | H 5,86 | N 7,24 |
| trouvé | C 65,3 | H 5,71 | N 6,93 |

### Exemple 41

### (4-iodophényl)méthyl-3-(1H-imidazol-4-yl)propyléther

La préparation est effectuée comme dans l'exemple 36 avec le chlorure de (4-iodophényl)méthane. Le composé du titre est cristallisé sous forme d'hydrogénomaléate dans éthanol/diéthyléther.
C₁₃H₁₅N₂OI.C₄H₄0₄.0,5H₂O (467,3) F: 123-124 °C rendement : 25 %

| Analyse élémentaire : | | | |
|---|---|---|---|
| calculé | C 43,7 | H 4,31 | N 5,99 |
| trouvé | C 43,6 | H 3,94 | N 5,99 |

### Exemple 42

### 4-phénylbutyl-3-(1-imidazol-4-yl)propyléther

La préparation est effectuée comme dans l'exemple 36 avec du chlorure de 4-phénylbutane. Le composé du titre est cristallisé sous forme d'hydrogénomaléate dans éthanol/diéthyléther.
C₁₆H₂₂N₂O.C₄H₄0₄(374,4) F : 96 °C rendement : 40 %

| Analyse élémentaire : | | | |
|---|---|---|---|
| calculé | C 64,2 | H 7,00 | N 7,48 |
| trouvé | C 63,9 | H 7,09 | N 7,69 |

### Exemple 45

### (3-phénylpropyl)-3-(1H-imidazol-4-yl) propyléther

La préparation est effectuée comme dans l'exemple 36 avec le bromure de 3-phénylpropane. Le composé du titre obtenu est recristallisé sous forme d'hydrogénomaléate dans éthanol/éther.
C₁₅H_{2O}N₂O.C₄H₄O₄.O,25 H₂O (364,9) F : 1O3°C rendement : 4O %

| Analyse élémentaire : | | | |
|---|---|---|---|
| calculé | C 62,5 | H 6,77 | N 6,88 |
| trouvé | C 62,4 | N 6,78 | N 6,83 |

### Exemple 46

### (3-phénylpropyl)-3-(1H-imidazol-4-yl)propylthioéther

On introduit 5 mmol de chlorure de 3-(1H-imidazol-4-yl)propane dans une solution de 6 mmol de 3-phénylmercaptane et de 1O mmol de sodium dans 3O ml d'éthanol et on porte au reflux 3 h. On évapore la suspension sous vide jusqu'à siccité, on ajoute du carbonate de potassium et on agite dans méthanol/eau. L'huile semi-solide restante est portée à l'ébullition dans l'éthanol avec du charbon actif, filtrée et recristallisée sous forme d'hydrogénomaléate dans éthanol/diéthyléther.
C₁₅H_{2O}N₂S.C₄H₄O₄.O,5 H₂O (385,5) F : 1O6°C rendement : 35 %

| Analyse élémentaire : | | | |
|---|---|---|---|
| calculé | C 59,2 | H 6,54 | N 7,27 |
| trouvé | C 59,3 | H 6,38 | N 7,3O |

### Exemple 47

### 4-[(4-nitrobenzylthio)méthyl]-1H-imidazol

Un mélange de 1,53 g (10 mmol) de chlorhydrate de 4-chlorométhylimidazol et de 0,76 g (10 mmol) de thiourée est porté au reflux dans 10 ml d'éthanol pendant 15 min. On ajoute 5 ml d'éthanol, 20 ml d'eau et 200 mg (1,16 mmol) de chlorure de 4-nitrobenzyle et on refroidit le mélange à 0-10°C. On ajoute goutte à goutte 1,44 g (36 mmol) d'une solution d'hydroxyde de sodium dans 14 ml d'eau sous azote à 0-10°C, puis on agite pendant 1 heure à la même température et encore pendant 3 heures à la température ambiante. Le précité est recueilli et, lavé avec de l'eau pour fournir le composé du titre, F : 88°C.
C₁₁H₁₁N₃O₂S

| Analyse élémentaire : | | | |
|---|---|---|---|
| calculé | C 53,0 | H 4,45 | N 16,9 |
| trouvé | C 53,0 | H 4,22 | N 16,6 |

### Exemple 48

### (3-phénylpropyl)-3-(1H-imidazol-4-yl)propylsulfoxyde

On ajoute à 2 mmol du composé obtenu dans l'exemple 46 O,5 g de carbonate de potassium dans 4O ml de dichlorométhane et on agite 3O mn. On additionne lentement à cette suspension 2,5 mmol d'acide chloroperoxybenzoïque dans 2O ml de dichlorométhane et on agite 2 h à la température ambiante. On filtre la suspension et on purifie par chromatographie. Le composé du titre est recristallisé sous forme d'hydrogénomaléate dans éthanol/diéthyléther.
C₁₅H_{2O}N₂OS.C₄H₄O₄.O,5 H₂O (4O1,5) F : 128-13O°C rendement : 6O %

| Analyse élémentaire : | | | |
|---|---|---|---|
| calculé | C 56,8 | H 6,28 | N 6,98 |
| trouvé | C 56,8 | H 6,1O | N 6,88 |

### Exemple 49

### 1-(1H-imidazol-4-yl)-7-phénylheptan-4-one

On introduit 5 mmol de 4-(1-triphénylméthyl-imidazol-4-yl) butannitrile dans une solution de bromure de 3-phénylpropylmagnésium dans 3OO ml de diéthyléther et 1OO ml de tétrahydrofuranne et on porte au reflux 6 h. On hydrolyse avec une solution de chlorure d'ammonium, on sépare la phase organique et on agite avec du dichlorométhane. Les phases organiques réunies et concentrées sont portées à l'ébullition pendant 2 h dans une solution hydro-alcoolique (éthanol-eau) d'acide chlorhydrique 2N. On élimine l'éthanol sous vide, on filtre et on rend alcalin. Le composé du titre est agité dans le dichlorométhane et recristallisé sous forme d'hydrogénomaléate dans éthanol/diéthyléther.
C₁₆H_{2O}N₂O.C₄H₄O₄.O,5 H₂O (381,4) F : 129° C rendement : 25 %.

| Analyse élémentaire : | | | |
|---|---|---|---|
| calculé | C 63,O | H 6,61 | N 7,34 |
| trouvé | C 62,6 | H 6,53 | N 7,86 |

### Exemple 50

### 1-(1H-imidazol-4-yl)-7-phénylheptan-4-ol

On introduit 1 mmol du composé obtenu dans l'exemple 49 dans une suspension de 1O mmol d'hydrure de lithium et d'aluminium dans 3O ml de diéthyléther et 1O ml de dioxane et on agite pendant la nuit. On hydrolyse avec une solution d'hydroxyde de sodium 2N et on lave le précipité avec du dichlorométhane. Les phases organiques sont réunies et concentrées. Le composé du titre est recristallisé sous forme d'hydrogénomaléate dans éthanol/diéthyléther.
C₁₆H₂₂N₂O.C₄H₄O₄.H₂O (392,5) F : 87°C rendement : 4O %.

| Analyse élémentaire : | | | |
|---|---|---|---|
| calculé | C 61,2 | H 7,19 | N 7,14 |
| trouvé | C 61,6 | H 6,83 | N 7,36 |

### Exemple 55

### N-benzoyl-3-(1H-imidazol-4-yl)propyloxycarbamide

On porte au reflux pendant 2 h 5 mmol du chlorhydrate de 3-(1H-imidazol-4-yl-)propanol dans 1O ml d'acétonitrile avec 5 mmol de benzoylisocyanate. Après évaporation sous vide, le composé du titre est agité dans l'eau et filtré.
C₁₄H₁₅N₃O₃.O,25H₂O (277,8) F : 150°C rendement : 8O %

| Analyse élémentaire : | | | |
|---|---|---|---|
| calculé | C 6O,5 | H 5,62 | N 15,1 |
| trouvé | C 6O,4 | H 5,44 | N 15,2 |

### Exemple 56

### N-cyclobutylméthyl-3-(1H-imidazol-4-yl)propyloxycarbamide

La préparation est effectuée comme dans l'exemple 23 avec le cyclobutylméthylisocyanate. Le composé du titre est extrait de l'eau avec du dichlorométhane et cristallisé sous forme d'hydrogénomaléate dans éthanol/diéthyléther.
C₁₂H₁₉N₃O₂.C₄H₄O₄.O,25 H₂O (357,9) F : 94°C rendement : 8O %

| Analyse élémentaire : | | | |
|---|---|---|---|
| calculé | C 53,7 | H 6,62 | N 11,7 |
| trouvé | C 53,9 | H 67,2 | N 11,8 |

### Exemple 57

### N-cyclopropylméthyl-3-(1H-imidazol-4-yl)propyloxycarbamide

La préparation est effectuée comme dans l'exemple 23 avec le cyclopropylméthylisocyanate. Le composé du titre est extrait de l'eau par du dichlorométhane et cristallisé sous forme d'hydrogénomaléate dans éthanol/diéthyléther.
C₁₁H₁₇N₃O₂.C₄H₄O₄.O,5 H₂O (384,4) F : 93° C rendement : 5O %

| Analyse élémentaire : | | | |
|---|---|---|---|
| calculé | C 51,7 | H 6,36 | N 12,1 |
| trouvé | C 51,8 | H 6,25 | N 11,9 |

### Exemple 58

### (R)-(+)-N-1-phényléthyl-3-(1H-imidazol-4-yl)propyloxycarbamide

La préparation est effectuée comme dans l'exemple 23 avec le (R)-(+)-1-phényléthylisocyanate. Le composé du titre est extrait de l'eau avec du dichlorométhane, purifié par chromatographie et cristallisé sous forme d'hydrogénomaléate dans éthanol/diéthyléther.
C₁₅H₁₉N₃O₂.C₄H₄O₄O,25 H₂O (393,9) F : 1O5°C rendement : 5O %

| Analyse élémentaire : | | | |
|---|---|---|---|
| calculé | C 57,9 | H 6,O1 | N 1O,7 |
| trouvé | C 57,9 | H 5,88 | N 1O,6 |

### Exemple 59

### (S)-(-)-N-1-phényléthyl-3-(1H-imidazol-4-yl)propyloxycarbamide

La préparation est effectuée comme dans l'exemple 23 avec le (S)-(-)-1-phényléthylisocyanate. Le composé du titre est extrait de l'eau avec du dichlorométhane, purifié par chromatographie et cristallisé sous forme d'hydrogénomaléate dans éthanol/diéthyléther.
C₁₅H₁₉N₃O₂.C₄H₄O₄.O,25 H₂O (393,9) F : 1O5°C rendement : 5O %

| Analyse élémentaire : | | | |
|---|---|---|---|
| calculé | C 57,9 | H 6,O1 | N 1O,7 |
| trouvé | C 58,1 | H 6,O1 | N 1O,7 |

### Exemple 60

### N-cyclohexyl-3-(1H-imidazol-4-yl) propyloxycarbamide

La préparation est effectuée comme dans l'exemple 23 avec le cyclohexylisocyanate et 5 mmol de triéthylamine en laissant 4 h à la température ambiante. Le composé du titre est extrait de l'eau avec du dichlorométhane et cristallisé sous forme d'hydrogénomaléate dans éthanol/diéthyléther.
C₁₃H₂₁N₃O₂.C₄H₄O₄.O,25 H₂O (371,9) F : 76°C rendement : 6O %

| Analyse élémentaire : | | | |
|---|---|---|---|
| calculé | C 54,9 | H 6,91 | N 11,3 |
| trouvé | C 54,9 | H 6,91 | N 11,4 |

### Exemple 61

### N-phényl-3-(1H-imidazol-4-yl)propyloxycarbamide

La préparation est effectuée comme dans l'exemple 57 avec le phénylisocyanate. Le composé du titre est extrait de l'eau avec du dichlorométhane et cristallisé sous forme d'hydrogénomaléate dans éthanol/diéthyléther;
C₁₃H₁₅N₃O₂.C₄H₄O₄.O,25 H₂O (365,9) F : 115°C rendement : 7O %

| Analyse élémentaire : | | | |
|---|---|---|---|
| calculé | C 55,8 | H 5,37 | N 11,5 |
| trouvé | C 55,7 | H 5,28 | N 11,5 |

### Exemple 62

### N-(4-méthylphényl)-3-(1H-imidazol-4-yl)propyloxycarbamide

La préparation est effectuée comme dans l'exemple 23 avec le 4-méthylphénylisocyanate. Le composé du titre est agité avec de l'eau, filtré et cristallisé sous forme d'hydrogénomaléate dans éthanol/diéthyléther.
C₁₄H₁₇N₃O₂.C₄H₄O₄.O,25 H₂O (379,9) F : 138-14O°C rendement: 85 %

| Analyse élémentaire : | | | |
|---|---|---|---|
| calculé | C 56,9 | H 5,7O | N 11,1 |
| trouvé | C 57,2 | H 5,67 | N 11,1 |

### Exemple 63

### N-(4-trifluorométhylphényl)-3-(1H-imidazol-4-yl)propyloxycarbamide

La préparation est effectuée comme dans l'exemple 23 avec le 4-trifluorométhylphénylisocyanate. Le composé du titre est agité avec de l'eau, filtré et cristallisé sous forme d'hydrogénomaléate dans éthanol/diéthyléther.
C₁₄H₁₄N₃O₂F₃ . C₄H₄O₄ . O,25 H₂O (433,9) F : 129°C
rendement : 85 %

| Analyse élémentaire : | | | |
|---|---|---|---|
| calculé | C 49,8 | H 4,3O | N 9,69 |
| trouvé | C 5O,O | H 4,18 | N 9,74 |

### Exemple 64

### N-(3-trifluorométhylphényl)-3-(1H-imidazol-4-yl)propyloxycarbamide

La préparation est effectuée comme dans l'exemple 23 avec le 3-trifluorométhylphénylisocyanate. Le composé du titre est agité avec de l'eau, filtré et cristallisé sous forme d'hydrogénomaléate dans éthanol/diéthyléther.
C₁₄H₁₄N₃O₂F₃.C₄H₄O₄.O,25 H₂O (433,9) F : 128°C rendement : 85 %

| Analyse élémentaire : | | | |
|---|---|---|---|
| calculé | C 49,8 | H 4,3O | N 9,69 |
| trouvé | C 49,8 | H 4,17 | N 9,53 |

### Exemple 65

### N-(2-trifluorométhylphényl)-3-(1H-imidazol-4-yl) propyloxycarbamide

La préparation est effectuée comme dans l'exemple 23 avec le 2-trifluorométhylphénylisocyanate. Le composé du titre est agité avec de l'eau, filtré et cristallisé sous forme d'hydrogénomaléate dans éthanol/diéthyléther.
C₁₄H₁₄N₃O₂F₃.C₄H₄O₄.O,25 H₂O (433,9O) F : 83°C rendement : 85 %.

| Analyse élémentaire : | | | |
|---|---|---|---|
| calculé | C 49,8 | H 4,3O | N 9,69 |
| trouvé | C 5O,2 | H 4,29 | N 9,55 |

### Exemple 66

### N-(2-phenyléthyl)-2-(1H-imidazol-4-yl) éthoxycarbamide

La préparation est effectuée comme dans l'exemple 23 avec le phényléthylisocyanate et le 2-(1H-imidazol-4-yl)éthanol. Le composé du titre est extrait de l'eau avec du dichlorométhane, purifié par chromatographie et cristallisé sous forme d'hydrogénomaléate dans éthanol/diéthyléther.
C₁₄H₁₇N₃O₂.C₄H₄O₄.O,25 H₂O (379,9) F : 145°C rendement : 65 %

| Analyse élémentaire : | | | |
|---|---|---|---|
| calculé | C 56,9 | H 5,7O | N 11,1 |
| trouvé | C 56,9 | H 5,63 | N 1O,9 |

### Exemple 67

### N-(4-nitrobenzyl)-3-(1H-imidazol-4-yl)propyloxycarbamide

La préparation est effectuée comme dans l'exemple 23 avec le 4-nitrobenzylisocyanate. Le composé du titre est extrait de l'eau avec du dichlorométhane, purifié par chromatographie et cristallisé sous forme d'hydrogénomaléate dans éthanol/diéthyléther.
C₁₄H₁₆N₄O₄.C₄H₄O₄.O,25 H₂O (424,9) F :128-129°C rendement : 3O %

| Analyse élémentaire : | | | |
|---|---|---|---|
| calculé | C 5O,9 | H 4,86 | N 13,2 |
| trouvé | C 5O,9 | H 4,76 | N 13,2 |

### Exemple 68

### N-(4-aminobenzyl)-3-(1H-imidazol-4-yl)propyloxycarbamide

2 mmol du composé obtenu dans l'exemple 65 sont hydrogénés dans 1O ml de méthanol avec 4O mg de palladium sur charbon (1O %) avec de l'hydrogène. Après absorption de la quantité d'hydrogène calculée, la solution est filtrée, concentrée et purifiée par chromatographie. Le composé du titre obtenu est cristallisé sous forme d'hydrogénomaléate dans éthanol/diéthyléther.
C₁₄H₁₈N₄O₂.C₄H₄O₄.O,25 H₂O (394,9) F : 118°C rendement : 6O %

| Analyse élémentaire : | | | |
|---|---|---|---|
| calculé | C 54,8 | H 5,74 | N 14,2 |
| trouvé | C 54,8 | H 5,68 | N 14,1 |

### Exemple 69

### N-(3-nitrophényl)-3-(1H-imidazol-4-yl)propyloxycarbamide

La préparation est effectuée comme dans l'exemple 23 avec le 2-nitrophénylisocyanate. Le composé du titre est extrait de l'eau avec du dichlorométhane, purifié par chromatographie et cristallisé sous forme d'hydrogénomaléate dans éthanol/diéthyléther
C₁₃H₁₄N₄O₄.C₄H₄O₄.O,25 H₂O (41O,9) F : 162°C rendement : 55 %

| | | | | |
|---|---|---|---|---|
| Analyse élémentaire : | calculé | C 49,7 | H 4,54 | N 13,6 |
| | trouvé | C 49,5 | H 4,37 | N 13,4 |

### Exemple 71

### ester 3-cyclohexylpropylique de l'acide 3-(1H-imidazol-4-yl)propanoïque

5 mmol de l'ester méthylique de l'acide 3-(1H-imidazol-4-yl) propanoïque sont portés au reflux 1 h dans 2O ml de 3-cyclohexylpropanol en introduisant du HCl gaz. La solution est reprise dans l'acétate d'éthyle, lavée avec une solution aqueuse de carbonate de potassium et concentrée. Le composé du titre est cristallisé sous forme d'hydrogénomaléate dans acétonitrile/diéthyléther.
C₁₅H₂₄N₂O₂.C₄H₄O₄.O,5 H₂O (389,4) F : 126°C rendement : 8O %

| Analyse élémentaire : | | | |
|---|---|---|---|
| calculé | C 58,6 | H 7,51 | N 7,19 |
| trouvé | C 58,6 | H 7,34 | N 7,46 |

### Exemple 72

### N-(2-nitrophényl)-3-(1H-imidazol-4-yl)propyloxycarbamide

La préparation est effectuée comme dans l'exemple 23 avec le 2-nitrophénylisocyanate. Le composé du titre est séparé par filtration et cristallisé sous forme d'hydrogénomaléate dans éthanol/diéthyléther.
C₁₃H₁₄N₄O₄.HCl.O,25 H₂O (331,2) F : 16O°C rendement : 9O %

| Analyse élémentaire : | | | |
|---|---|---|---|
| calculé | C 47,1 | H 4,72 | N 16,9 |
| trouvé | C 47,O | H 4,73 | N 16,7 |

### Exemple 73

### N-(4-fluorophényl)-3-(1H-imidazol-4-yl)propyloxycarbamide

La préparation est effectuée comme dans l'exemple 23 avec le 4-fluorophénylisocyanate. Le composé du titre est extrait de l'eau avec du dichlorométhane, purifié par chromatographie et cristallisé sous forme d' hydrogénomaléate dans éthanol/diéthyléther.
C₁₃H₁₄N₃O₂F.C₄H₄O₄.0,25H₂O (383,8) F : 137°C rendement : 70%

| Analyse élémentaire : | | | |
|---|---|---|---|
| calculé | C 53,2 | H 4,86 | N 11,0 |
| trouvé | C 53,5 | H 4,76 | N 11,0 |

### Exemple 74

### N-2-(phényléthyl)-3-(1H-imidazol-4-yl)propyloxycarbamide

La préparation est effectuée comme dans l'exemple 23 avec le 2-phényléthylisocyanate. Le composé du titre est extrait de l'eau avec du dichlorométhane, purifié par chromatographie et cristallisé sous forme d'hydrogénomaléate dans éthanol/diéthyléther.
C₁₅H₁₉N₃O₂.C₄H₄O₄₀0,25 H₂O(393,9) F : 107-109 °C
rendement: 65 %

| Analyse élémentaire : | | | |
|---|---|---|---|
| calculé | C 57,9 | H 6,11 | N 10,7 |
| trouvé | C 57,8 | H 6,01 | N 10,7 |

### Exemple 75

### N-(4-fluorobenzyl)-3-(1H-imidazol-4-yl)propyloxycarbamide

La préparation est effectuée comme dans l'exemple 23 avec le 4-fluorobenzylisocyanate. Le composé du titre est extrait de l'eau avec du dichlorométhane, purifié par chromatographie et cristallisé sous forme d'hydrogénomaléate dans éthanol/diéthyléther.
C₁₄H₁₅N₃O₂F.C₄H₄O₄.0,25H₂O(397,9) F : 137-138 °C rendement: 60 %

| Analyse élémentaire : | | | |
|---|---|---|---|
| calculé | C 54,3 | H 5,19 | N 10,6 |
| trouvé | C 54,3 | H 5,19 | N 10,5 |

### Exemple 76

### N-(4-chlorophényl)-3-(1H-imidazol-4-yl)propyloxycarbamide

La préparation est effectuée comme dans l'exemple 23 avec le 4-chlorophénylisocyanate. Le composé du titre est extrait de l'eau avec du dichlorométhane, purifié par chromatographie et cristallisé sous forme d'hydrogénomaléate dans éthanol/diéthyléther.
C₁₃H₁₄N₃O₂Cl.C₄H₄O₄,0,25H₂O(400,3) F : 132 °C rendement : 50 %

| Analyse élémentaire : | | | |
|---|---|---|---|
| calculé | C 51,0 | H 4,66 | N 10,5 |
| trouvé | C 51,0 | H 4,51 | N 10,4 |

### Exemple 77

### N-(4-chlorobenzyl)-3-(1H-imidazol-4-yl)propyloxycarbamide

La préparation est effectuée comme dans l'exemple 23 avec le 4-chlorobenzylisocyanate. Le composé du titre est extrait de l'eau avec du dichlorométhane, purifié par chromatographie et cristallisé sous forme d'hydrogénomaléate dans éthanol/diéthyléther.
C₁₄H₁₆N₃O₂Cl.C₄H₄O₄.0,25H₂O(414,3) F : 133-134 °C
rendement : 60 %

| Analyse élémentaire : | | | |
|---|---|---|---|
| calculé | C 52,2 | H 4,99 | N 10,1 |
| trouvé | C 52,4 | H 4,90 | N 10,1 |

### Exemple 78

### N-(3-iodophényl)-3-(1H-imidazol-4-yl)propyloxycarbamide

La préparation est effectuée comme dans l'exemple 23 avec le 3-iodophénylisocyanate. Le composé du titre est extrait de l'eau avec du dichlorométhane, purifié par chromatographie et cristallisé sous forme d'hydrogénomaléate dans éthanol/diéthyléther.
c₁₃H₁₄N₃O₂I.C₄H₄O₄.0,25H₂O(491,8) F : 132 °C rendement : 60 %

| Analyse élémentaire : | | | |
|---|---|---|---|
| calculé | C 41,5 | H 3,79 | N 8,54 |
| trouvé | C 41,9 | H 3,88 | N 8,67 |

### Exemple 79

### N-(2-iodophényl)-3-(1H-imidazol-4-yl)propyloxycarbamide

La préparation est effectuée comme dans l'exemple 23 avec le 2-iodophénylisocyanate. Le composé du titre est extrait de l'eau avec du dichlorométhane, purifié par chromatographie et cristallisé sous forme d'hydrogénomaléate dans éthanol/diéthyléther.
C₁₃H₁₄N₃O₂I.C₄H₄O₄.0,25H₂O(491,8) F : 114 °C rendement : 50 %

| analyse élémentaire : | | | |
|---|---|---|---|
| calculé | C 41,5 | H 3,79 | N 8,54 |
| trouvé | C 41,3 | H 3,62 | N 8,43 |

### Exemple 80

### N-(4-iodophényl)-3-(1H-imidazol-4-yl)propyloxycarbamide

La préparation est effectuée comme dans l'exemple 23 avec le 4-iodophénylisocyanate. Le composé du titre est extrait de l'eau avec du dichlorométhane, purifié par chromatographie et cristallisé sous forme d'hydrogénomaléate dans éthanol/diéthyléther.
C₁₃H₁₄N₃O₂I.C₄H₄O₄.0,25H₂O(491,8) F : 139 °C rendement : 55 %

| Analyse élémentaire : | | | |
|---|---|---|---|
| calculé | C 41,5 | H 3,79 | N 8,54 |
| trouvé | C 41,7 | H 3,75 | N 8,44 |

### Exemple 81

### N-3-(phénylpropyl)-3-(1H-imidazol-4-yl)propyloxycarbamide

La préparation est effectuée comme dans l'exemple 23 avec le 3-phénylpropylisocyanate. Le composé du titre est extrait de l'eau avec du dichlorométhane, purifié par chromatographie et cristallisé sous forme d'hydrogénomaléate dans éthanol/diétyléther
Ç₁₆H₂₁N₃O₂C₄H₄O₄.0,25H₂O(407,9) F : 90 °C rendement : 60 %

| Analyse élémentaire : | | | |
|---|---|---|---|
| calculé | C 58,9 | H 6,30 | N 10,3 |
| trouvé | C 59,1 | H 6,38 | N 10,5 |

### Exemple 82

### N-(4-trifluorométhylbenzyl)-3-(1H-imidazol-4-yl)propyloxycarbamide

La préparation est effectuée comme dans l'exemple 23 avec le 4-trifluorobenzylisocyanate. Le composé du titre est agité avec de l'eau, séparé par filtration et cristallisé sous forme d'hydrogénomaléate dans éthanol/diéthyléther
C₁₅H₁₆N₃O₂F₃.C₄H₄O₄.O,25 H₂O (447,9) F : 97°C rendement : 4O %

| Analyse élémentaire : | | | |
|---|---|---|---|
| calculé | C 51,O | H 4,61 | N 9,38 |
| trouvé | C 51,1 | H 4,45 | N 9,O4. |

### Exemple 83

### N-benzyl-N-méthyl-3-(1H-imidazol-4-yl)propyloxycarbamide

La préparation est effectuée comme dans l'exemple 23 avec le chlorure de N-benzyl-N-méthylcarbamoyle. Le composé du titre est agité avec de l'eau, séparé par filtration et cristallisé sous forme d'hydrogénomaléate dans éthanol/diéthyléther.
C₁₅H₁₉N₃O₂.C₄H₄O₄.O,25 H₂O (393,9) F : 7O°C rendement : 15 %

| Analyse élémentaire : | | | |
|---|---|---|---|
| calculé | C 57,9 | H 6,O1 | N 1O,7 |
| trouvé | C 58,2 | H 6,O7 | N 1O,6 |

### Exemple 84

### N-benzyl-N-isopropyl-3-(1H-imidazol-4-yl)propyloxycarbamide

La préparation est effectuée comme dans l'exemple 23 avec le chlororure de N-benzyl-N-isoproylcarbamoyle. Le composé du titre est agité avec l'eau, séparé par filtration et cristallisé sous forme d'hydrogénomaléate dans éthanol/diéthyléther.
C₁₇H₂₃N₃O₂.C₄H₄O₄.0,5H₂O(426,5) F : 74-76 °C rendement : 30 %

| Analyse élémentaire : | | | |
|---|---|---|---|
| calculé | C 59,1 | H 6,62 | N 9,85 |
| trouvé | C 59,4 | H 6,54 | N 9,56 |

### Exemple 85

### 3-(4-chlorophényl)propyl-3-(1H-imidazol-4-yl)propyléther

La préparation est effectuée comme dans l'exemple 36 avec du chlorure de 3-(4-chlorophényl)propane. Le composé du titre est cristallisé sous forme d'hydrogénomaléate dans éthanol/diéthyléther.
C₁₅H₁₉N₂OCl.C₄H₄O₄ (394,9) F : 13O°C rendement : 5O %

| Analyse élémentaire : | | | |
|---|---|---|---|
| calculé | C 57,8 | H 5,87 | N 7,O9 |
| trouvé | C 58,2 | H 6,O2 | N 7,3O |

### Exemple 86

### (4-chlorophényl)méthyl-3-(1H-imidazol-4-yl)propyléther La préparation est effectuée comme dans l'exemple 36 avec le chlorure de (4-chlorophényl)méthane. Le composé du titre est cristallisé sous tonne d'hydrogénomaléate dans éthanol/diéthyléther.

C₁₃H₁₅N₂OCl.C₄H₄O₄ (366,8) F : 1O9°C rendement : 5O %

| Analyse élémentaire : | | | |
|---|---|---|---|
| calculé | C 55,7 | H 5,22 | N 7,64 |
| trouvé | C 55,6 | H 5,44 | N 7,73 |

### Exemple 87

### cyclohexylmethyl-(1H-imidazol-4-yl)méthyléther

La préparation est effectuée comme dans l'exemple 36 avec le chlorure de cyclohexylméthane et (1-triphénylméthyl-imidazol-4-yl)méthanolate de sodium. Le composé du titre est cristallisé sous forme d'hydrogénomaléate dans éthanol/diéthyléther.
C₁₁H₁₈N₂O.C₄H₄O₄.0.25H₂O (314,5) F : 102°C rendement 50%

| Analyse élémentaire : | | | |
|---|---|---|---|
| calculé | C 56,4 | H 8,77 | N 7,26 |
| trouvé | C 56,3 | H 8,96 | N 7,19 |

### Exemple 88

### 3-(4-fluorophényl)propyl-3-(1H-imidazol-4-yl)propyléther

La préparation est effectuée comme dans l'exemple 36 avec le chlorure de 3-(4-fluorophényl)propane. Le composé du titre est cristallisé sous forme d'hydrogénomaléate dans éthanol/diéthyléter.
C₁₅H₁₉N₂OF.C₄H₄O₄ . 0,25H₂O (382,9) F: 118°C rendement 45%

| Analyse élémentaire : | | | |
|---|---|---|---|
| calculé | C 59,6 | H 6,19 | N 7,32 |
| trouvé | C 59,3 | H 6,04 | N 7,24 |

### Exemple 89

### p-nitrophényl-3-(1H-imidazol-4-yl)-2-trans-propénoate

On ajoute 2 ml (27,5 mmol) de chlorure de thionyle fraîchement distillé à un mélange finement broyé de 1,51 g (1O,8 mmol) de p-nitrophénol et de 1,5 g (1O,8 mmol) d'acide trans-urocanique dans un ballon muni d'un tube sécheur contenant CaCl₂. Le mélange est progressivement porté à 14O°C. Après 4 h, le mélange est lentement refroidi à la température ambiante. On extrait une poudre brune avec CHCl₃/MeOH (150 ml, 2 : 1) en chauffant. On sépare par filtration le matériau non dissous et on traite le filtrat avec du charbon actif. On concentre sous vide, on ajoute du diéthyléther de façon à induire la précipitation d'un solide (1,96 g ; rendement global brut : 61 %). Celui-ci est recristallisé dans MeOH et le produit est lavé avec du diéthyléther pour éliminer toute trace de p-nitrophénol, ce qui donne un solide blanc cristallin, F 22O-222°C.
C₁₂H₉N₃O₄.HCl

| Analyse élémentaire : | | | | |
|---|---|---|---|---|
| calculé | C 48,7 | H 3,41 | N 14,2 | Cl 12,0 |
| trouvé | C 48,4 | H 3,48 | N 14,2 | Cl 11,9 |

### Exemple 106

### 2-[1H-imidazol-4-yl]méthylthio-5-nitropyridine

Un mélange de O,6 g (3,9 mmol) de 4-chlorométhylimidazol et d'un équivalent équimolaire de thiourée (O,3 g) dans 1O ml d'éthanol est porté au reflux pendant 3O mn. On ajoute à ce mélange 5 ml d'éthanol, 2O ml d'eau et 1,2 équivalent molaire de 2-chloro-5-nitropyridine (O,74 g) dans 5 ml d'éthanol chaud. On refroidit la solution claire à O-1O°C dans un bain de glace et on ajoute goutte à goutte une solution de O,49 g d'hydroxyde de sodium dans 1O ml d'eau sous azote à O-1O°C, puis on agite pendant encore 3 h à 21°C. Le précipité est recueilli, lavé avec de l'eau et chromatographié sur un gel de silice avec un mélange de chloroforme et de méthanol (1-2O %) pour donner le composé du titre, F : 155-156°C.
C₉H₈N₄O₂S contenant 2 % de matériau minéral

| Analyse élémentaire | | | |
|---|---|---|---|
| calculé | C 44,8 | H 3,34 | N 23,2 |
| trouvé | C 45,1 | H 3,33 | N 23,3. |

### Exemple 107

### 2-{2-[1H-imidazol-4-yl]éthylthio}-5-nitropyridine

On ajoute à une solution de O,2 g (1,1 mmol) de dibromhydrate de 5-{2-[1H-imidazol-4-yl]éthyl} isothiourée dans 2 ml d'eau, une solution chaude de O,2 g (1,3 mmol) de 2-chloro-5-nitropyridine dans 5 ml d'éthanol. La suspension résultante est agitée sous azote. Le mélange réactionnel est refroidi à O-5°C et une solution de 4 équivalents molaires d'hydroxyde de sodium (O,16 g) dans 2 ml d'eau est ajoutée goutte à goutte sous N₂. Le mélange réactionnel est agité pendant 1 h à la même température et pendant 3 h à 21°C. Le mélange réactionnel est évaporé à siccité et le résidu est soumis à une chromatographie sur colonne (gel de silice) éluée avec un mélange gradient de chloroforme et de méthanol (1-1O %) pour fournir le composé du titre qui est ensuite cristallisé dans du 2-propanol, F : 147-148°C.
C_{1O}H_{1O}N₄O₂S, O,25 H₂O

| Analyse élémentaire : | | | | |
|---|---|---|---|---|
| calculé | C 47,1 | H 4,15 | N 22,O | S 12,6 |
| trouvé | C 47,4 | H 3,86 | N 21,5 | S 12,O |

### Exemple 108

### 2-{2-[1H-imidazol-4-yl]éthyl}thiopyridine

O,5 g (4,4 mmol) de 4-(2-hydroxyéthyl)-1H-imidazol et O,49 g (44,4 mmol) de 2-mercaptopyridine sont portés au reflux dans 5 ml de HBr aqueux à 47 % pendant 24 h. Le solvant est éliminé par voie azéotropique avec de l'isopropanol sous pression réduite pour fournir le compose du titre sous la forme d'un sel de dibromhydrate monohydrate qui, après cristallisation dans l'isopropanol, fond à 189-19O°C.
C_{1O}H₁₁N₃S, 2HBr, H₂O

| Analyse élémentaire : | | | | |
|---|---|---|---|---|
| calculé | C 31,2 | H 3,92 | N 1O,9 | S 8,3 |
| trouvé | C 31,6 | H 3,87 | N 11,O | S 8,6 |

### Exemple 109

### 2-{2-[1H-imidazol-4-yl]éthyl}thio-1H-imidazol

183 mg (1,1 mmol) de chlorhydrate de 4-(2-chloroéthyl)-1H-imidazol, 11O mg (1 mmol) de 2-mercapto-1H-imidazol et 2OO mg (3,26 mmol) d'hydroxyde de potassium solide sont portés au reflux dans 1O ml de 2-propanol pendant 4 h. Le mélange est ensuite évaporé et le résidu semi-solide est chromatographié sur un gel de silice en utilisant un mélange chloroforme : méthanol (5 : 1). Le produit résultant est traité par l'acide oxalique dans l'éthanol pour fournir le composé du titre sous la forme d'un sel d'oxalate, F : 224 - 226°C
C₈H_{1O}N₄S, 1,61 C₂H₂O₄

| Analyse élémentaire : | | | | |
|---|---|---|---|---|
| calculé | C 39,7 | H 3,92 | H 16,5 | S 9,45 |
| trouvé | C 39,5 | H 3,83 | N 16,5 | S 9,5 |

### Exemple 110

### 4-[2-(4-nitrophénoxy)éthyl]-1H-imidazol

2,O9 mg (1,5 mmol) de 4-nitrophénol, 251 mg (1,4 mmol) de chlorhydrate de 4-[2-chloroéthyl]-1H-imidazol, 475 mg (3 mmol) de carbonate de potassium et du iodure de sodium (catalyseur) sont agités à 8O°C pendant 5 jours dans 5 ml de diméthylformamide. Le mélange réactionnel est refroidi et 1OO ml de diéthyléther sont ajoutés. Le précipité est séparé par filtration. Le filtrat est évaporé sous pression réduite pour donner un résidu que l'on soumet à une chromatographie sur colonne (en utilisant comme premier éluant du chloroforme et comme second éluant un mélange chloroforme : méthanol 9 :1O) pour donner le composé du titre qui, après cristallisation dans le néthanol, fond à : 197-2OO°C.
C₁₁H₁₁N₃O₃

| Analyse élémentaire | | | |
|---|---|---|---|
| calculé | C 56,7 | H 4,75 | N 18,O |
| trouvé | C 56,9 | H 4,7O | N 17,9 |

### Exemple 111

### 4-[2-(4-carbométhoxyphénoxy)éthyl]-1H-imidazol

228 mg (1,5 mol) de 4-hydroxybenzoate de méthyle, 251 mg (1,5 mmol)de chlorhydrate de 4-[2-chloroéthyl]-1H-imidazol, 5OO mg (3,6 mmol) de carbonate de potassium et du iodure de sodium (catalyseur) sont agités à 8O°C pendant 3 jours dans 5 ml de diméthylformamide. Le mélange réactionnel est refroidi et filtré, et le filtrat est évaporé sous pression réduite pour donner un résidu huileux que l'on soumet à une chromatographie sur colonne (en utilisant comme premier éluant du chloroforme et comme second éluant un mélange chloroforme : méthanol 95 : 5). Le produit est purifié par chromatographie liquide préparative à haute performance et cristallisé dans l'eau ; F : 116 - 118°C.
C₁₃H₁₄N₂O₃, CF₃CO₂H, O,3 H₂O

| Analyse élémentaire | | | |
|---|---|---|---|
| calculé | C 49,3 | H 4,3O | N 7,7 |
| trouvé | C 49,3 | H 3,92 | N 7,6 |

### Exemple 112

### 4-[2-(4-cyanophénoxy)éthyl]-1H-imidazol

36O mg (3 mmol) de 4-cyanophénol, 251 mg (1,5 mmol) de chlorhydrate de 4-[2-chloroéthyl]imidazol, 5OO mg (3,6 mmol) de carbonate de potassium et du iodure de sodium (catalyseur)sont agités à 8O°C pendant 5 jours dans 5 ml de diméthylformamide. Le mélange est refroidi et filtré. Le filtrat est évaporé sous pression réduite pour donner un résidu huileux. Le 4-cyanophénol qui n'a pas réagi, est précipité avec un mélange méthanol : diéthyléther (1 : 1O). Après filtration, le filtrat est évaporé sous pression réduite pour donner le composé du titre que l'on cristallise dans un mélange méthanol : eau (1O : 3), F : 181-183°C
C₁₂H₁₁N₃O

| Analyse élémentaire | | | |
|---|---|---|---|
| calculé | C 67,6 | H 5,2O | N 19,7 |
| trouvé | C 67,2 | H 5,18 | N 19,7 |

### Exemple 113

### 4-[2-(4-acétylphénoxy)éthyl]-1H-imidazol

816 mg (6 mmol) de 5-hydroxyacétophénone, 251 mg (1,5 mmol) de chlorhydrate de 4-[2-chloroéthyl]-1H-imidazol, 5OO mg (3,6 mmol) de carbonate de potassium et du iodure de sodium (catalyseur) son agités à 8O°C pendant 5 jours dans 5 ml de diméthylformamide. Le mélange réactionnel est refroidi, filtré et le filtrat est évaporé sous pression réduite pour donner un résidu huileux, duquel on extrait la 4-hydroxyacétophénone par l'éther à pH = 1. Une extraction ultérieure par l'acétate d'éthyle à pH = 9 donne le composé du titre. Celui-ci a été traité par l'acide oxalique dans du 2-propanol pour donner l'oxalate du composé du titre que l'on cristallise dans un mélange méthanol : éther (1O : 1), F 178 - 182°C.
C₁₃H₁₄N₂O₂, C₂H₂O₄

| Analyse élémentaire | | | |
|---|---|---|---|
| calculé | C 56,2 | H 5,O4 | N 8,8 |
| trouvé | C 56,4 | H 5,O6 | N 8,9 |

### Exemple 114

### 4-[2-(4-éthoxycarbonyl)phénoxy)éthyl]-1H-imidazol

1,49 g (9 mmol) de 4-hydroxybenzoate d'éthyle, 251 mg (1,5 mmol) de chlorhydrate de 4-(2-chloroéthyle)-1H-imidazol, 500 g (3,6 mmol) de carbonate de potassium et du iodure de sodium (catalyseur) sont agités à 80°C pendant 4 jours dans 5 ml de diméthylformamide. Le mélange réactionnel est refroidi, puis filtré. Le filtrat est évaporé sous pression réduite pour donner un résidu huileux. L'excès de 4-hydroxybenzoate d'éthyle est extrait avec de l'éther à pH=1. La solution aqueuse est évaporée sous pression réduite pour donner le composé du titre. Celui-ci est purifié par chromatographie préparative à haute performance et transformé en oxalate qui, après cristallisation dans un mélange isopropanol : éther, fond à 160-164°C.
C₁₄H₁₆N₂O₃, 0,8 C₂H₂O₄

| Analyse élémentaire : | | | |
|---|---|---|---|
| calculé | C 56,4 | H 5,34 | N 8,4 |
| trouvé | C 56,4 | H 5,29 | N 8,3 |

### Exemple 115

### 4-[2-(3-nitrophénoxy)éthyl]-1H-imidazol

A une solution de 1,67 g (12 mmol) de 3-nitrophénol dans 10 ml de diméthylformamide, on ajoute lentement 240 mg (60 % dans l'huile, 6 mmol) d'hydrure de sodium et on agite le mélange à la température ambiante pendant 1 heure. On ajoute 200 mg (1,2 mmol) de chlorhydrate de 4-(2-chloroéthyl)-1H-imidazol et du iodure de tétrabutylammonium (catalyseur) et on agite à 80°C pendant 3 jours. On refroidit le mélange réactionnel et on ajoute 150 ml de diéthyléther. Le précipité est séparé par filtration et le filtrat est évaporé sous pression reduite pour donner un résidu qui est purifié par chromatographie préparative à haute performance. Le composé du titre est transformé en oxalate et cristallisé dans l'éthanol, F : 174-178°C.
C₁₁H₁₁N₃O₃, 0,75 C₂H₂O₄

| Analyse élémentaire : | | | |
|---|---|---|---|
| calculé | C 49,9 | H 4,19 | N 14,0 |
| trouvé | C 50,1 | H 4,06 | N 13,8 |

### Exemple 116

### 4-[2-(4-méthoxyphénoxy)éthyl]-1H-imidazol

A une solution de 1,48 g (12 mmol) de 4-méthoxyphénol dans 7 ml de diméthylformamide, on ajoute lentement 240 mg (60 % dans l'huile, 6 mmol) d'hydrure de sodium et on agite le mélange à la température ambiante pendant 1 heure. On ajoute 200 mg (1,2 mmol) de chlorhydrate de 4-(2-chloroéthyl)-1H-imidazol et du iodure de tétrabutylammonium (catalyseur) et on agite à 80°C pendant 2 jours. Le mélange réactionnel est refroidi, et 150 ml de diéthyléther sont ajoutés. Le précipité est séparé par filtration et le filtrat est évaporé sous pression réduite pour donner un résidu qui est purifié par chromatographie préparative à haute performance. Le composé du titre est transformé en oxalate et recristallisé dans l'éthanol, F : 178-181°C.

| Analyse élémentaire : | | | |
|---|---|---|---|
| calculé | C 55,8 | H 5,37 | N 9,5 |
| trouvé | C 55,9 | H 5,40 | N 9,6 |

### Exemple 117

### 4-[2-(4-propylphénoxy)éthyl]-1H-imidazol

A une solution de 1,63 g (12 mmol) de 4-propylphénol dans 10 ml de diméthylformamide, on ajoute lentement 240 mg (60 % dans l'huile ; 6 mmol) d'hydrure de sodium et on agite le mélange à la température ambiante pendant 1 heure. On ajoute 200 mg (1,2 mmol) de chlorhydrate, de 4-(2-chloroéthyl)-1H-imidazol et du iodue de tétrabutylammonium et on agite à 80°C pendant 3 jours. Le mélange réactionnel est refroidi et 150 ml de diéthyléther sont ajoutés. Le précipité est séparé par filtration et le filtrat est évaporé sous pression réduite pour donner un résidu qui est purifié par chromatographie préparative à haute performance. Le composé du titre est transformé en oxalate (dans un mélange éthanol : diéthyléther), F : 168-171°C
C₁₄H₁₈N₂O, 1,1 C₂H₂O₄

| Analyse élémentaire : | | | |
|---|---|---|---|
| calculé | C 59,1 | H 6,18 | N 8,5 |
| trouvé | C 58,9 | H 6,04 | N 8,5 |

### Exemple 118

### 4-[2-(4-bromophénoxy)éthyl]-1H-imidazol

A une solution de 2,07 g (12 mmol) de 4-bromophénol dans 10 ml de formamide, on ajoute lentement 240 mg (60 % dans l'huile ; 6 mmol) d'hydrure de sodium et on agite à la température ambiante pendant 1 heure. On ajoute 200 mg (1,2 mmol) de chlorhydrate de 4-(2-chloroéthyl)-1H-imidazol et du iodure de tétrabutylammonium (catalyseur) et on agite à 80°C pendant 3 jours. Le mélange réactionnel est refroidi et 150 ml de diéthyléther sont ajoutés. Le précipité est séparé par filtration et le filtrat est évaporé sous pression réduite pour donner un résidu qui est soumis à une chromatographie sur colonne sur un gel de silice (premier éluant acétate d'éthyle et second éluant acétate d'éthyleméthanol 95:5). Le composé du titre est purifié par une chromatographie préparative à haute performance, puis transformé en oxalate qui, après cristallisation dans un mélange éthanol : diéthyléther, fond à 162-165°C.
C₁₁H₁₁BrN₂O, 1,1 C₂H₂O₄

| Analyse élémentaire : | | | |
|---|---|---|---|
| calculé | C 43,3 | H 3,63 | N 7,7 |
| trouvé | C 43,2 | H 3,52 | N 7,7 |

### Exemple 119

### 4-[2-(3,5-dichlorophénoxy)éthyl]-1H-imidazol

A une solution de 2,34 g (14,4 mmol) de 3,5-dichlorophénol dans 4 ml de diméthylformamide, on ajoute 288 mg (60 % dans l'huile, 7,2 mmol) d'hydrure de sodium, en refroidissant dans un bain de glace. On agite le mélange à la température ambiante pendant 1 heure sous azote. On ajoute ensuite 200 mg (1,20 mmol) de chlorhydrate de 4-(2-chloroéthyl)-1H-imidazol et du iodure de tétrabutylammonium (quantité catalytique). On chauffe le mélange à 80° C pendant 3 jours, puis on évapore le solvant sous pression réduite.

On reprend le résidu huileux restant dans du dichlorométhane et on sèche sur du sulfate de magnésium anhydre. On évapore le solvant et on chromatographie le résidu sur une colonne de gel de silice (dichlorométhane/méthanol 10,1) pour éliminer l'excès de 3,5-dichlorophénol. Le mélange des composés obtenu après chromatographie sur colonne, est séparé par HPLC préparative (eau contenant 0,1 % de TFA : méthanol contenant 0,1 % de TFA 30 : 70). On obtient le produit sous forme de trifluoroacétate, F : 115-116 °C.
C₁₁H₁₀Cl₂N₂O CF₃COOH

| Analyse élémentaire : | | | |
|---|---|---|---|
| calculé | C 42,1 | H 2,99 | N 7,6 |
| trouvé | C 42,0 | H 2,76 | N 7,3 |

### Exemple 120

### 4-[2-(2,3,4,5,6-pentafluorophénoxy)éthyl]-1H-imidazol

A une solution de 2,2 g (12 mmol) de 2,3,4,5,6-pentafluorophénol dans 4 ml de diméthylformamide, on ajoute 240 mg (60 % dans l'huile, 6 mmol) d'hydrure de sodium, en refroidissant dans un bain de glace. On agite le mélange à la température ambiante pendant 1 heure sous azote. On ajoute ensuite 200 mg (1,20 mmol) de chlorhydrate de 4-(2-chloroéthyl)-1H-imidazol et du iodure de tétrabutylammonium (quantité catalytique). On chauffe le mélange à 80° C pendant 3 jours, puis on évapore le solvant sous pression réduite.

On reprend le résidu huileux restant dans le dichlorométhane et on extrait l'excès de 2,3,4,5,6-pentafluorophénol avec une solution aqueuse de bicarbonate de sodium. On évapore le dichlorométhane et on met sous forme d'oxalate le composé du titre obtenu par traitement dans l'isopropanol. On évapore l'isopropanol, on dissout le composé dans l'éthanol et on le traite avec du charbon actif décolorant. Le produit est obtenu après filtration suivie d'une précipitation par le diéthyléther, F : 163-164 °C
C₁₁H₇F₃N₂O 1,2 C₂H₂O₄ 0,2H₂O

| Analyse élémentaire : | | | |
|---|---|---|---|
| calculé | C 41,3 | H 2,53 | N 7,2 |
| trouvé | C 40,9 | H 2,09 | N 7,7 |

### Exemple 121

### 4-[2-(4-trifluorométhylphénoxy)éthyl]-1H-imidazol

A 708 g (2 mmol) de 1-triphénylméthyl-4-(2-hydroxyéthyl)imidazol, on ajoute sous azote 15 ml de tétrahydrofuranne fraîchement distillé, 340 mg (2,1 mmol) de 4-trifluorométhylphénol et 550 mg (2,1 mmol) de triphénylphosphine. On refroidit le mélange résultant et on agite 5 mn. On ajoute alors lentement 66 mg (2,1 mmol) de diéthylazodicarboxylate dans 10 ml de tétrahydrofuranne. On poursuit l'agitation à la température ambiante pendant 2 heures sous azote. On évapore ensuite le solvant sous pression réduite et on chromatographie le résidu sur une colonne de gel de silice, en utilisant du diéthyléher comme éluant pour donner le 1-triphénylméthyl-4-[2-(4-trifluorométhyl-phénoxy)éthyl]imidazol.

On chauffe une solution de 640 mg (1,28 mmol) de 1-(triphénylméthyl)-4-[2-(4-trifluorométhylphénoxy)-éthyl]-1H-imidazol dans 2 ml de tétrahydrofuranne et 5 ml d'acide chlorhydrique 2 N à 70°C pendant 2 heures. On évapore ensuite le tétrahydrofuranne sous pression réduite et on extrait le résidu avec du diéthyléther. On rend basique la solution aqueuse avec du carbonate de potassium, on filtre le solide blanc et on le lave 3 fois à l'eau pour donner le composé du titre,
F : 105-109 °C
C₁₂H₁₁N₂OF₃

| Analyse élémentaire : | | | |
|---|---|---|---|
| calculé | C 56,25 | H 4,33 | N 10,93 |
| trouvé | C 56,30 | H 4,33 | N 10,67 |

### Exemple 122

### 4-[2-(4-éthylphénoxy)éthyl]-1H-imidazol

A une solution de 1,1 g (9 mmol) de 4-éthylphénol dans 7 ml de diméthylformamide, on ajoute lentement 180 mg (60 % dans l'huile ; 4,5 mmol) d'hydrure de sodium et on agite à la température ambiante pendant 2 heures. On ajoute 150 mg (0,9 mmol) de chlorhydrate de 4-(2-chloroéthyl)-1H-imidazol et du iodure de tétrabutylammonium (catalyseur) et on agite à 80°C pendant 3 jours. Le solvant est concentré, et 50 ml de diéthyléther sont ajoutés. Le précipité est séparé par filtration et le filtrat est évaporé sous pression réduite pour donner un résidu qui est soumis à une chromatographie sur colonne sur un gel de silice (premier éluant acétate d'éthyle, second éluant acétate d'éthyle : méthanol 95:5). Le composé du titre est purifié ensuite par chromatographie préparative, puis transformé en oxalate qui, après cristallisation dans un mélange éthanol : diéthyléther, fond à 178°C.
C₁₃H₁₆N₂O, 0,85 C₂H₂O₄

| Analyse élémentaire : | | | |
|---|---|---|---|
| calculé | C 60,3 | H 6,09 | N 9,6 |
| trouvé | C 60,3 | H 6,00 | N 9,6 |

### Exemple 123

### 4-[2-(3-cyanophénoxy)éthyl]-1H-imidazol

A une solution de 1,43 g (12 mmol) de 3-hydroxybenzonitrile dans 10 ml de diméthylformamide, on ajoute 240 mg (60 % dans l'huile ; 6 mmol) d'hydrure de sodium et on agite le mélange à la température ambiante pendant 1 heure sous azote. On ajoute 200 mg (1,2 mmol) de chlorhydrate de 4-(2-chloroéthyl)-1H-imidazol et du iodure de tétrabutylammonium (quantité catalytique) et on chauffe le mélange pendant 3 heures à 80°C. On évapore le solvant sous pression réduite, on agite le résidu huileux dans l'éthanol et on filtre. Le filtrat est évaporé et le résidu est purifié par chromatographie sur une colonne de gel de silice (premier éluant chloroforme, second éluant chloroforme : méthanol 95:5). Le composé du titre est transformé en oxalate et cristallisé dans le mélange éthanol/diéthyléther 2:1, F : 189-190 °C
C₁₂H₁₁N₃O, C₂H₂O₄

| Analyse élémentaire : | | | |
|---|---|---|---|
| calculé | C 55,5 | H 4,32 | N 13,9 |
| trouvé | C 55, 4 | H 4,18 | N 13,8 |

### Exemple 124

### 4-[2-(2-cyanophénoxy)éthyl]-1H-imidazol

A une solution de 1,43 g (12 mmol) dans 10 ml de diméthylformamide, on ajoute 240 mg (60 % dans l'huile ; 6 mmol) d'hydrure de sodium. On agite le mélange à la température ambiante pendant 1 heure sous azote. On ajoute 200 mg (1,2 mmol) de chlorhydrate de 4-(2-chloroéthyl)-1H-imidazol et du iodure de tétrabutylammonium (quantité catalytique) et on chauffe le mélange à 80°C pendant 3 jours. On évapore le solvant sous pression réduite, on agite le résidu huileux dans l'éthanol et on filtre. Le filtrat est évaporé et le résidu est soumis à une chromatographie sur une colonne de gel de silice (premier éluant chloroforme, second éluant chloroforme : méthanol 95:5). Le composé du titre est transformé en oxalate et cristallisé dans un mélange éthanol/diéthyléther (2:1), F : 170-171 °C
C₁₂H₁₁N₃O, 0,95 (C₂H₂O₄)

| Analyse élémentaire : | | | |
|---|---|---|---|
| calculé | C 55,9 | H 4,35 | N 14,1 |
| trouvé | C 56,1 | H 4,25 | N 14,1 |

### Exemple 125

### 4-[2-(2-naphtyloxy)éthyl]-1H-imidazol

A une solution de 1,73 g (12 mmol) de 2-naphtol dans 10 ml de diméthylformamide, on ajoute 240 mg (60 % dans l'huile, 6 mmol) d'hydrure de sodium. on agite le mélange à la température ambiante pendant 3 heures sous azote. On ajoute 200 mg (1,2 mmol) de chlorhydrate de 4-(2-chloroéthyl)-1H-imidazol et du iodure de tétrabutylammonium (quantité catalytique) et on chauffe le mélange à 100 °C pendant 3 jours, puis on évapore le solvant sous pression réduite. On extait l'excès de 2-naphtol avc du diéthyléther dans des conditions acides (acide chlorhydrique dilué). On rend basique la solution aqueuse avec du carbonate de potassium et on extrait le produit avec du diéthyléther. Les extraits éthérés sont concentrés et le résidu obtenu est cristallisé dans un mélange méthanol-eau (1:2), F : 157-158 °C
C₁₅H₁₄N₂O 0,1H₂O

| Analyse élémentaire : | | | |
|---|---|---|---|
| calculé | C 75,0 | H 5,96 | N 11,7 |
| trouvé | C 75,0 | H 5,79 | N 11,7 |

### Exemple 126

### 4-[2-(1-naphtyloxy)éthyl]-1H-imidazol

A une solution de 1,73 g (12 mmol) de 2-naphtol dans 10 ml de diméthylformamide, on ajoute 240 mg (60 % dans l'huile, 6 mmol) d'hydrure de sodium. On agite le mélange à la température ambiante pendant 1 heure sous azote. On ajoute 200 mg (1,2 mmol) de chlorhydrate de 4-(2-chloroéthyl)-1H-imidazol et du iodure de tétrabutylammonium (quantité catalytique). On chauffe le mélange à 100 °C pendant 3 jours, puis on évapore le solvant sous pression réduite. On reprend le résidu huileux restant dans du dichlorométhane et une solution aqueuse de carbonate de sodium et on traite avec du charbon actif. On sépare la solution de dichlorométhane, on évapore et on chromatographie le résidu restant sur une colonne de gel de silice (dichlorométhane/méthanol 10:1) pour donner le composé du titre sous forme de base. On prépare l'oxalate dans l'isopropanol et on le cristallise dans l'éthanol, F : 187-189 °C
C₁₅H₁₄N₂O 1,75 C₂H₂O₄

| Analyse élémentaire : | | | |
|---|---|---|---|
| calculé | C 64,8 | H 5,11 | N 9,2 |
| trouvé | C 64,8 | H 4,76 | N 8,9 |

### Exemple 127

### 4-[2-(4-benzoylphénoxy)éthyl]-1H-imidazol

A une solution de 2,34 g (12 mmol) de 4-hydroxybenzophénone dans 10 ml de diméthylformamide, on ajoute 240 mg (60 % dans l'huile, 6 mmol) d'hydrure de sodium. On agite le mélange à la température ambiante pendant 1 heure sous azote. On ajoute ensuite 200 mg (1,2 mmol) de chlorhydrate de 4-(2-chloroéthyl)-1H-imidazol et du iodure de tétrabutylammonium (quantité catalytique). On chauffe le mélange à 100 °C pendant 2 jours, puis on évapore le solvant sous pression réduite. On ajoute au résidu huileux de l'acide chlorhydrique dilué et on extrait l'excès d'hydroxybenzophénone avec du diéthyléther. On rend basique la solution aqueuse avec du carbonate de potassium et on extrait le produit avec du diéthyléther. Le composé du titre est transformé en dioxalate. Une cristallisation dans un mélange éthanol/diéthyléther (2:1) donne un produit pur, F 193-194 °C.
C₁₈H₁₆N₂O₂ 1,3 C₂H₂O₄

| Analyse élémentaire : | | | |
|---|---|---|---|
| calculé | C 60,4 | H 4,58 | N 6,8 |
| trouvé | C 60,2 | H 4,63 | N 7,1 |

### Exemple 128

### 4-[2-(4-nitrophényl)thioéthyl]-1H-imidazol

A une solution de 1,86 g (12 mmol) de 4-nitrothiophénol dans 10 ml de diméthylformamide, on ajoute 200 mg (60 % dans l'huile, 5 mmol) d'hydrure de sodium. On agite le mélange à la température ambiante pendant 1 heure sous azote. On ajoute ensuite 200 mg (1,2 mmol) de chlorhydrate de 4-(2-chloroéthyl)-1H-imidazol et du iodure de tétrabutylammonium (quantité catalytique). On chauffe le mélange pendant 1 jour à 80 °C, puis on évapore le solvant sous pression réduite. On agite le résidu dans du diéthyléther et on filtre. On ajoute de l'acide chlorhydrique dilué au filtrat et on élimine l'excès de 4-nitrothiophénol par extraction avec du diéthyléther. La solution aqueuse est rendue basique avec du carbonate de potassium, puis le composé du titre est extrait avec du chloroforme et cristallisé dans l'éthanol, F : 167-168 °C.
C₁₁H₁₁N₂O₂S

| Analyse élémentaire : | | | |
|---|---|---|---|
| calculé | C 53,0 | H 4,45 | N 16,9 |
| trouvé | C 53,1 | H 4,41 | N 16,8 |

### Exemple 129

### 4-[3-(4-fluorophenoxy)propyl]-1H-imidazol

A 368 mg (1 mmol) de 1-triphénylméthyl-4-(3-hydroxypropyl) imidazol, on ajoute sous azote 5 ml de tétrahydrofuranne fraîchement distillé, 120 mg (1 mmol) de 4-fluorophénol et 270 mg (1 mmol) de 4-triphénylphosphine. On refroidit le mélange résultant et on agite 5 mn. On ajoute alors lentement 180 mg (1 mmol) de diéthylazodicarboxylate dans 5 ml de tétrahydrofuranne. On poursuit l'agitation à la température ambiante pendant 3 jours sous azote. On évapore ensuite le solvant sous pression réduité et on soumet le résidu à une chromatographie sur une colonne de gel de silice, en utilisant le diéthyléther comme éluant pour donner le 1-triphénylméthyl-4-[3-(4-fluorophénoxy)propyl]-1H-imidazol.

On chauffe à 70 °C pendant 2 heures une solution de 147 mg (0,4 mmol) de 1-(triphénylméthyl)-4-[3-(4-fluorophénoxy)propyl]-1H-imidazol dans 1 ml de tétrahydrofuranne et 2 ml d'acide chlorhydrique 2N. On évapore ensuite le tétrahydrofuranne sous pression réduite. La solution aqueuse est filtrée et rendue basique avec du bicarbonate de potassium. Le produit est extrait par du chloroforme et séché sur du sulfate de magnésium. Le solvant est évaporé sous pression résuite pour donner le composé du titre sous forme d'un solide blanc, F : 135-137 °C
C₁₂H₁₃N₂OF 0,07 CHCl₃

| Analyse élémentaire : | | | |
|---|---|---|---|
| calculé | C 63,4 | H 5,76 | N 12,25 |
| trouvé | C 63,3 | H 5,66 | N 12,33 |

### Exemple 130

### 4-[3-(4-cyanophénoxy)propyl]-1H-imidazol

(a) 1-(triphénylméthyl)-4-(3-hydroxypropyl)imidazol
On traite une solution de 2,0 g (15,85 mmol) de 4-(3-hydroxypropyl)-1H-imidazol, de 5,5 ml (54,3 mmol) d'éthylamine sèche dns 15,6 ml de diméthylformamide avec 4,86 g (17,4 mmol) de chlorure de triphénylméthyle dans 5 ml de diméthylformamide sous azote. On agite le mélange obtenu à la température ambiante pendant 2 heures, puis on le verse sur 350 g de glace pilée. On recueille le solide résultant par filtration, on le lave trois fois à l'eau et on le purifie sur une colonne chromatographique en utilisant comme éluant du chloroforme, puis un mélange chloroforme/méthanol (1:1) pour donner le 1-triphénylméthyl-4-(3-hydroxypropyl)imidazol, F : 132-133 °C.
(b) A 184 mg (0,5 mmol) de 1-triphénylméthyl-4-(3-hydroxypropyl)imidazol, on ajoute sous azote 5 ml de tétrahydrofuranne fraîchement distillé, 71 mg (0,6 mmol) de triphénylphosphine. On refroidit le mélange résultant et on l'agite 5 mn. On ajoute ensuite lentement 104 mg (0,6 mmol) de diéthylazodicarboxylate dans 3 ml de tétrahydrofuranne et on poursuit l'agitation à la température ambiante pendant 3 heures sous azote. On évapore ensuite le solvant sous pression réduite et on chromatographie le résidu sur une colonne de gel de silice (premier éluant éther de pétrole/diéthyléther (1:1) ; second éluant diéthyléher) pour donner le 1-triphénylméthyi-4-[3-(4-cyanophénoxy)propyl] imidazol, F : 187-188 °C
C₁₂H₂₇N₃O 0,3 H₂O

| Analyse élémentaire : | | | |
|---|---|---|---|
| calculé | C 81,5 | H 5,82 | N 8,9 |
| trouvé | C 81,2 | H 5,41 | N 8,8 |

(c) On chauffe une solution de 96 mg (0,2 mmol) de 1-(triphénylméthyl)-4-[3-(4-cyanophénoxy)propyl]imidazol dans 2 ml de tétrahydrofuranne et 5 ml d'acide chlorhydrique 2N à 70 °C pendant 6 heures. On évapore ensuite le tétrahydrofuranne sous pression réduite et on extrait le résidu avec du diéthyléther. On rend basique la solution aqueuse avec du carbonate de potassium, on extrait le produit avec du chloroforme et on le sèche sur du sulfate de magnésium. On évapore ensuite le solvant sous pression réduite pour donner le composé du titre sous forme d'un solide blanc que l'on cristallise dans un mélange éthanol/éther (1:2), F 194-195 °C.
C₁₃H₁₃N₃O 0,1 H₂O

| Analyse élémentaire : | | | |
|---|---|---|---|
| calculé | C 68,2 | H 5,81 | N 18,3 |
| trouvé | C 68,3 | H 5,51 | N 18,1 |

### Exemple 131

### 4-[3-(4-trifluorométhylphénoxy)propyl]-1H-imidazol

(a) A 280 g (0,76 mmol) de 1-(triphénylméthyl)-4-(3-hydroxypropyl)imidazol, on ajoute sous azote 5 ml de tétrahydrofuranne, 129 mg (0,8 mmol) de 4-trifluorométhylphénol et 209 mg (0,8 mmol) de triphénylphosphine. On agite le mélange résultant pendant 5 mn. On ajoute ensuite lentement 139 mg (0,8 mmol) de diéthylazodicarboxylate dans 5 ml de tétrahydrofuranne. On poursuit l'agitation à la température ambiante pendant 3 heures sous azote. On évapore ensuite le solvant, puis on chromatographie sur une colonne de gel de silice (premier éluant éther de pétrole contenant des qualités croissantes de diéthyléther jusqu'à 100 %) pour donner le monohydrate de 1-triphénylméthyl-4-[3-(4-trifluorométhylphénoxy)-propyl]-imidazol que l'on cristallise dans l'éthanol, F : 150-151°C
C₃₂H₂₇FN₂O 1,1 H₂O

| Analyse élémentaire : | | | |
|---|---|---|---|
| calculé | C 72,2 | H 5,53 | N 5,3 |
| trouvé | C 72,1 | H 5,44 | N 5,2 |

(b) On chauffe une solution de 260 mg (0,5 mmol) de 1-triphénylméthyl-4-[3-(4-trifluorométhylphénoxy) propyl]imidazol dans 2 ml de tétrahydrofuranne et 5 ml d'acide chlorhydrique 2N à 70 °C pendant 6 heures. On évapore ensuite le solvant organique sous pression réduite et on lave le résidu résultant avec du diéthyléther. On rend basique la solution aqueuse avec du carbonate de potassium et on extrait le composé du titre avec du diéthyléther. On sèche l'extrait sur du sulfate de magnésium et on évapore le solvant sous pression réduite pour donner le composé du titre sous forme d'un solide blanc. On transforme celui-ci en oxalate que l'on cristallise dans un mélange éthanol/diéthyléther, F 201-204 °C.
C₁₃H₁₃F₃N₂O 1,1 C₂H₂O₄

| Analyse élémentaire : | | | |
|---|---|---|---|
| calculé | C 49,4 | H 4,15 | N 7,6 |
| trouvé | C 49,7 | H 3,79 | N 7,5 |

Les composés des exemples précédents sont rassemblés dans le tableau I suivant.

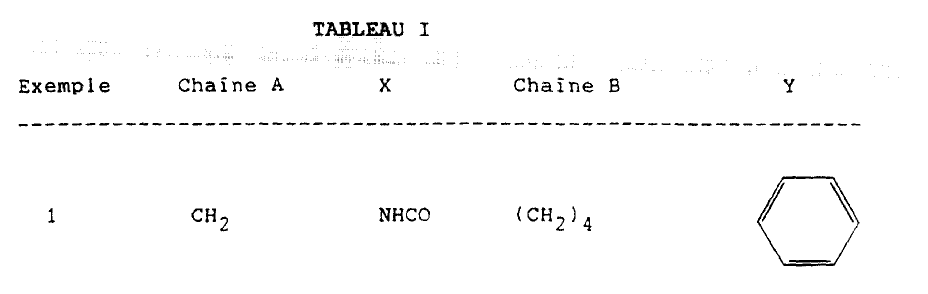

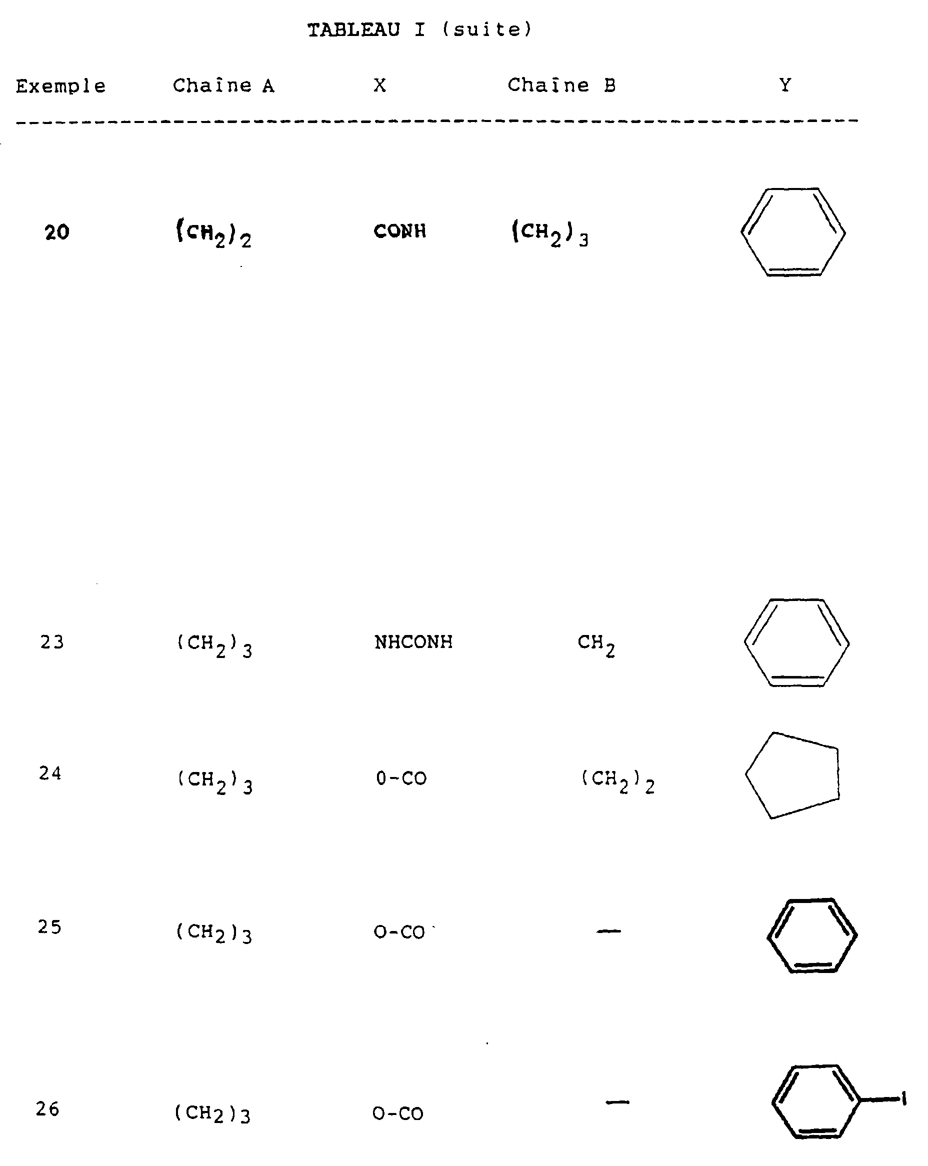

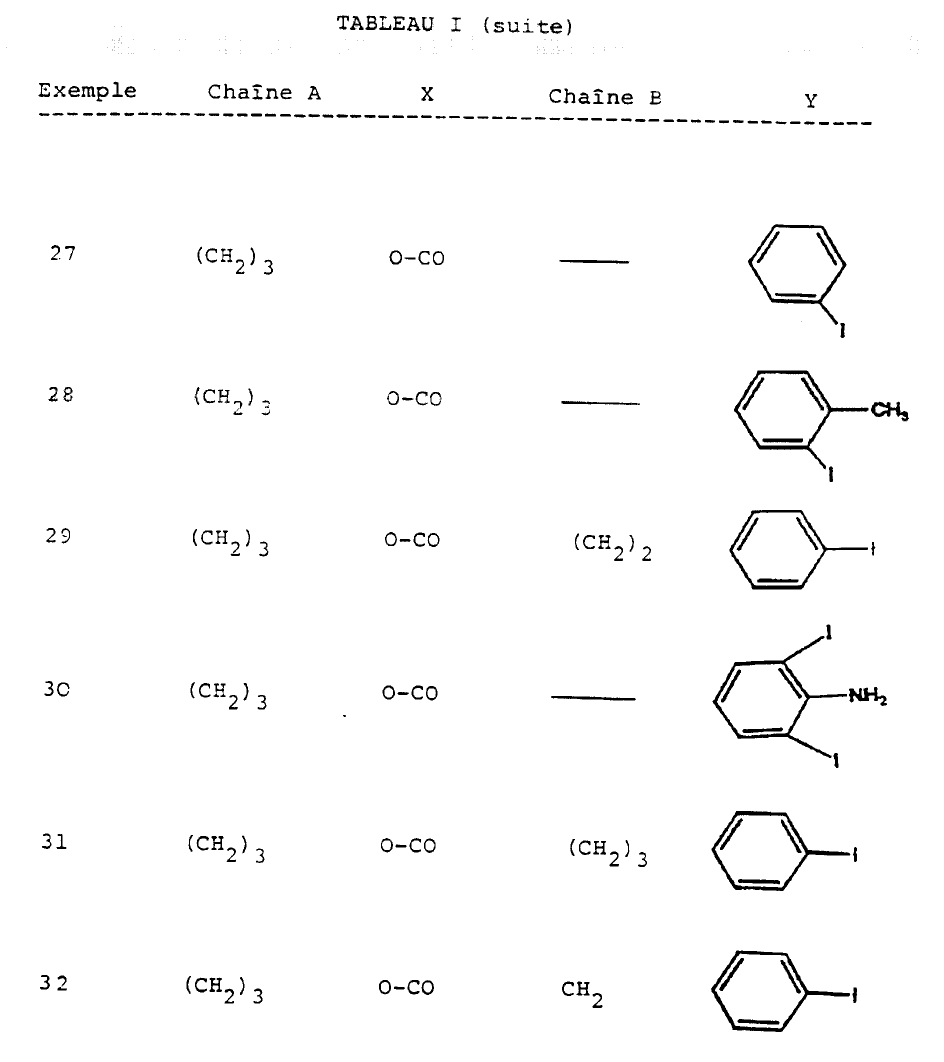

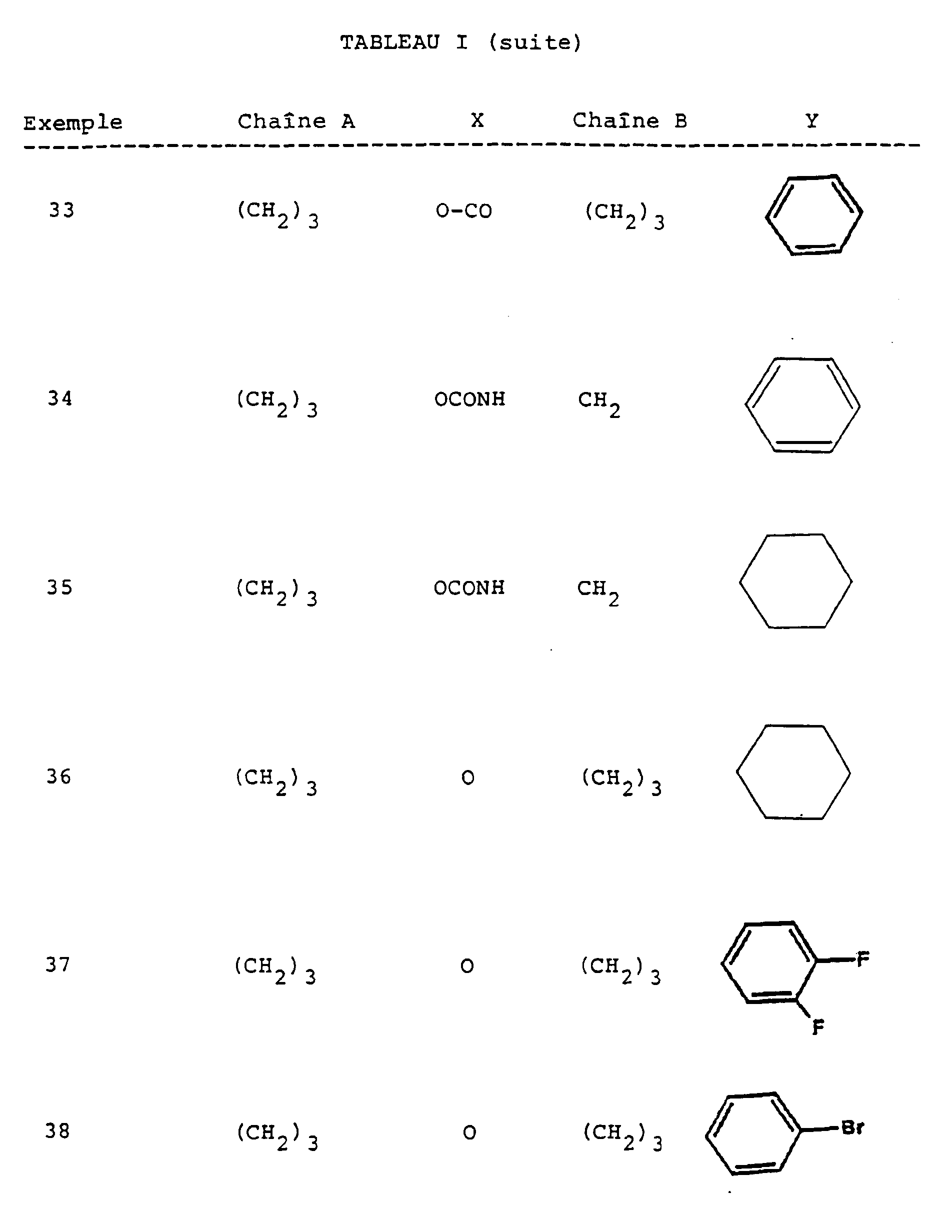

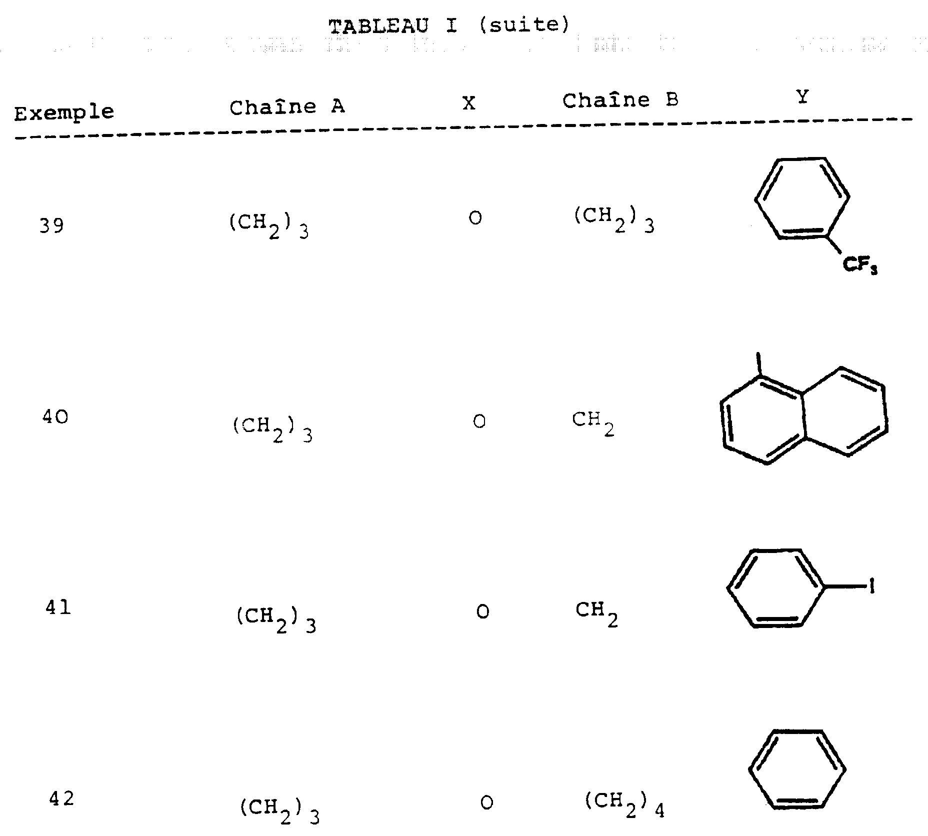

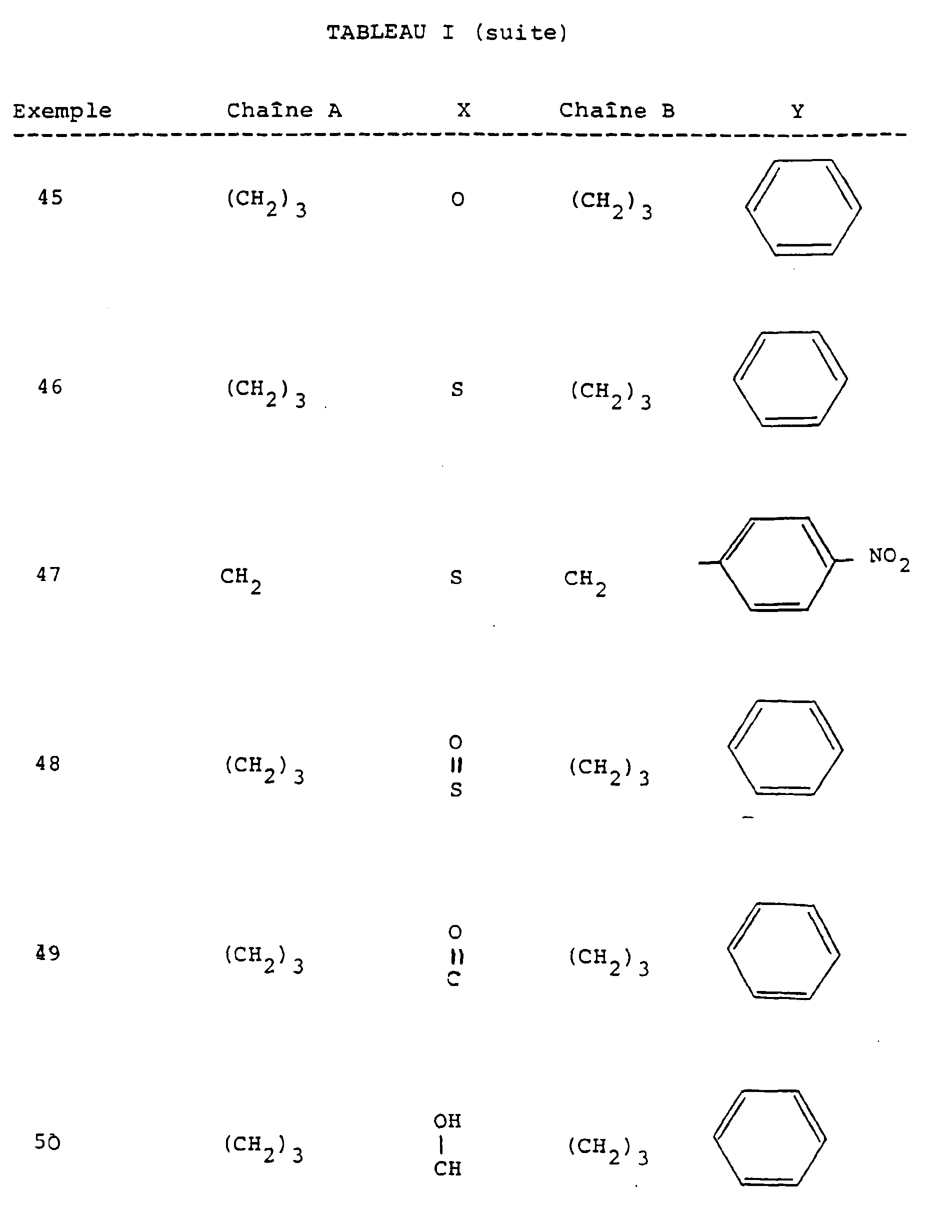

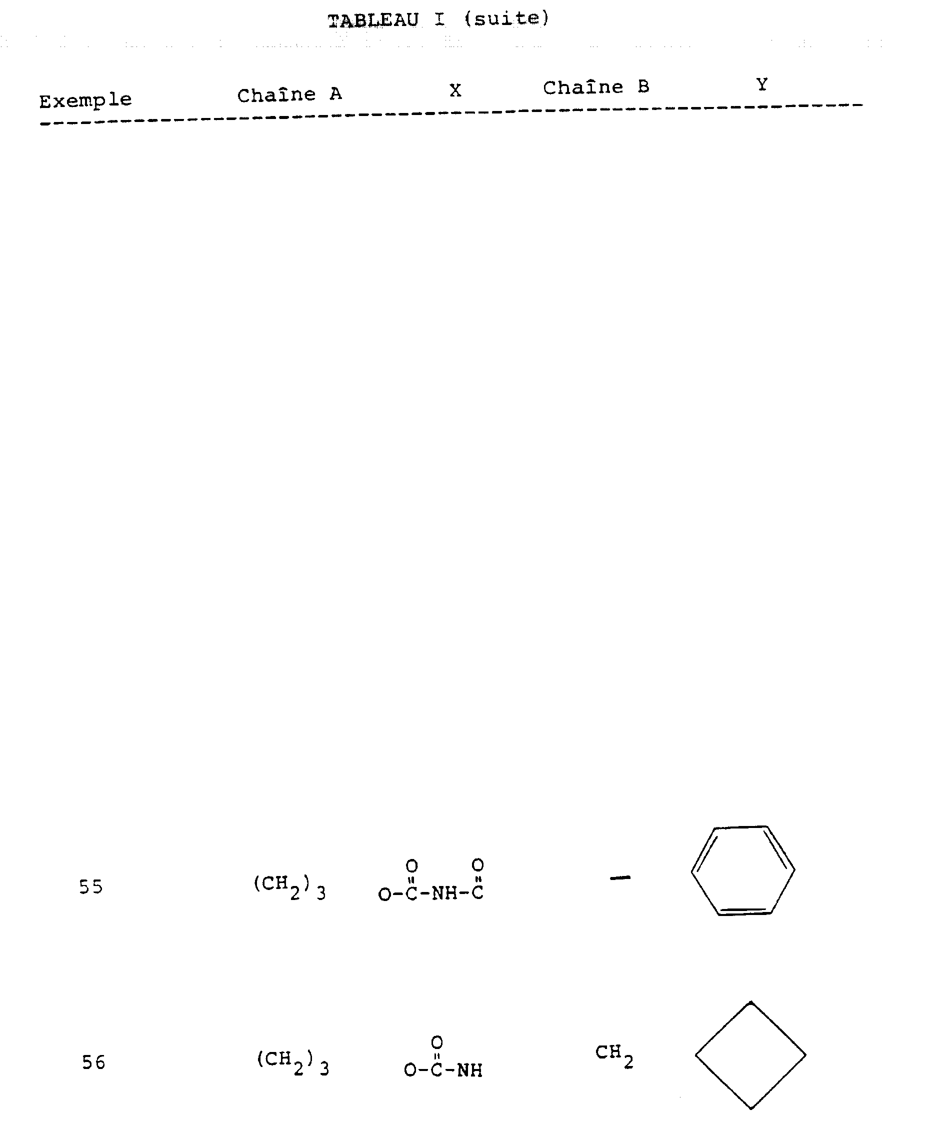

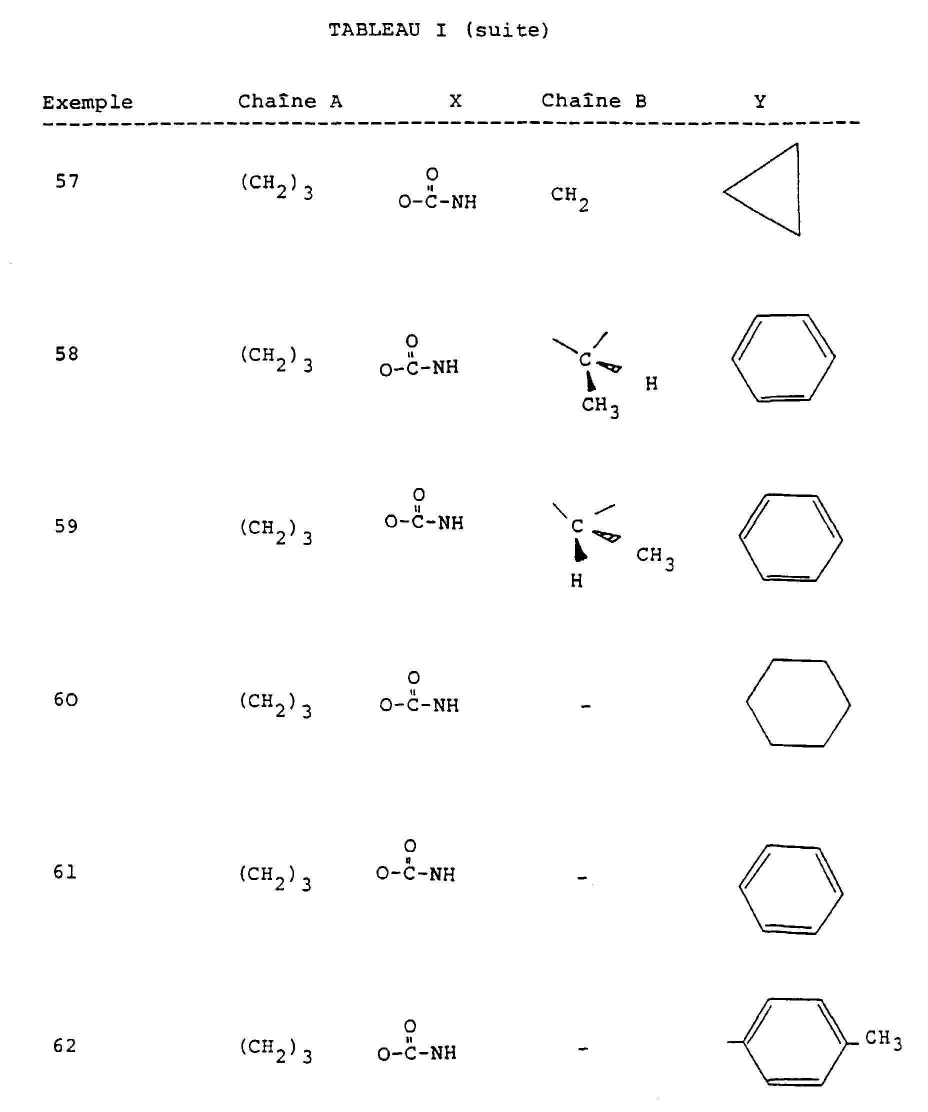

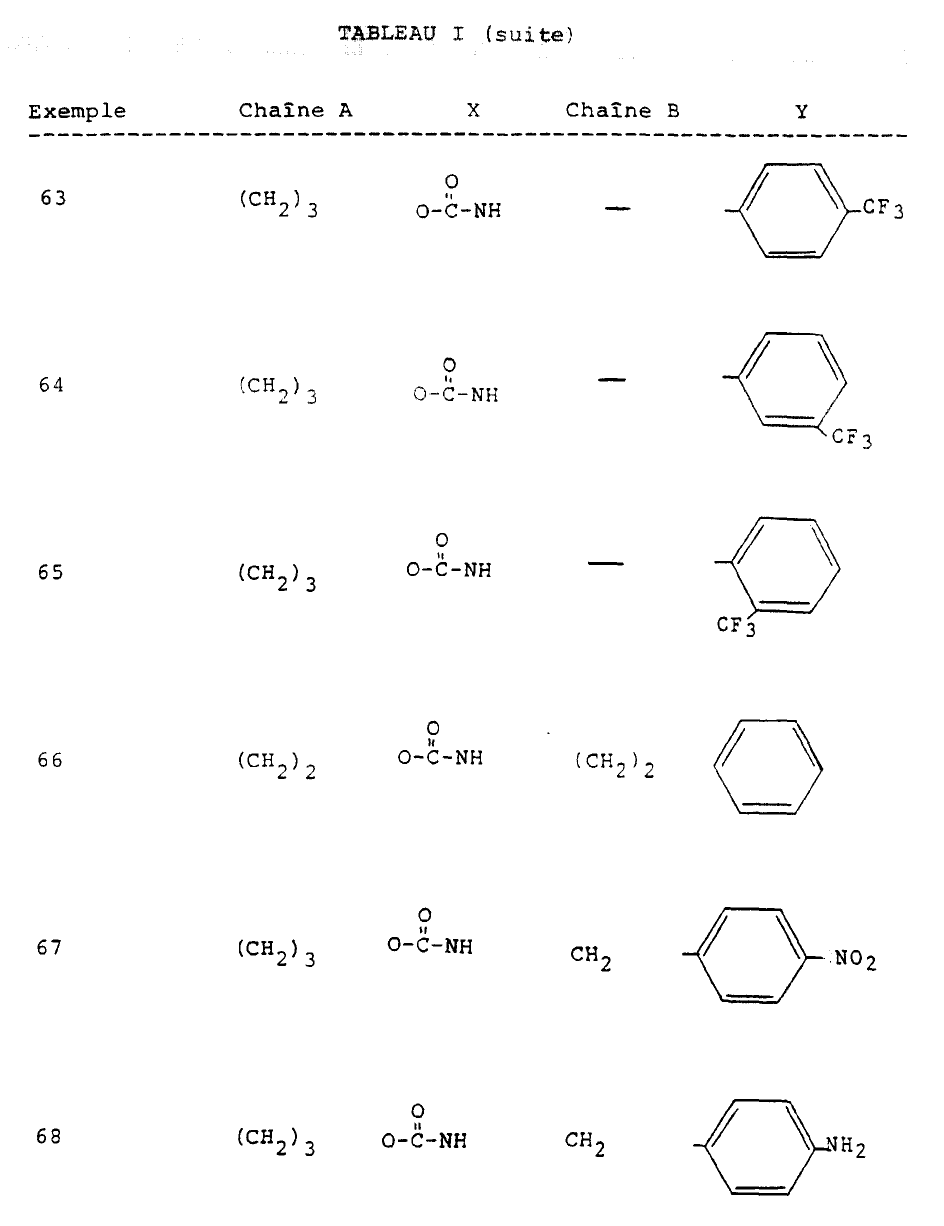

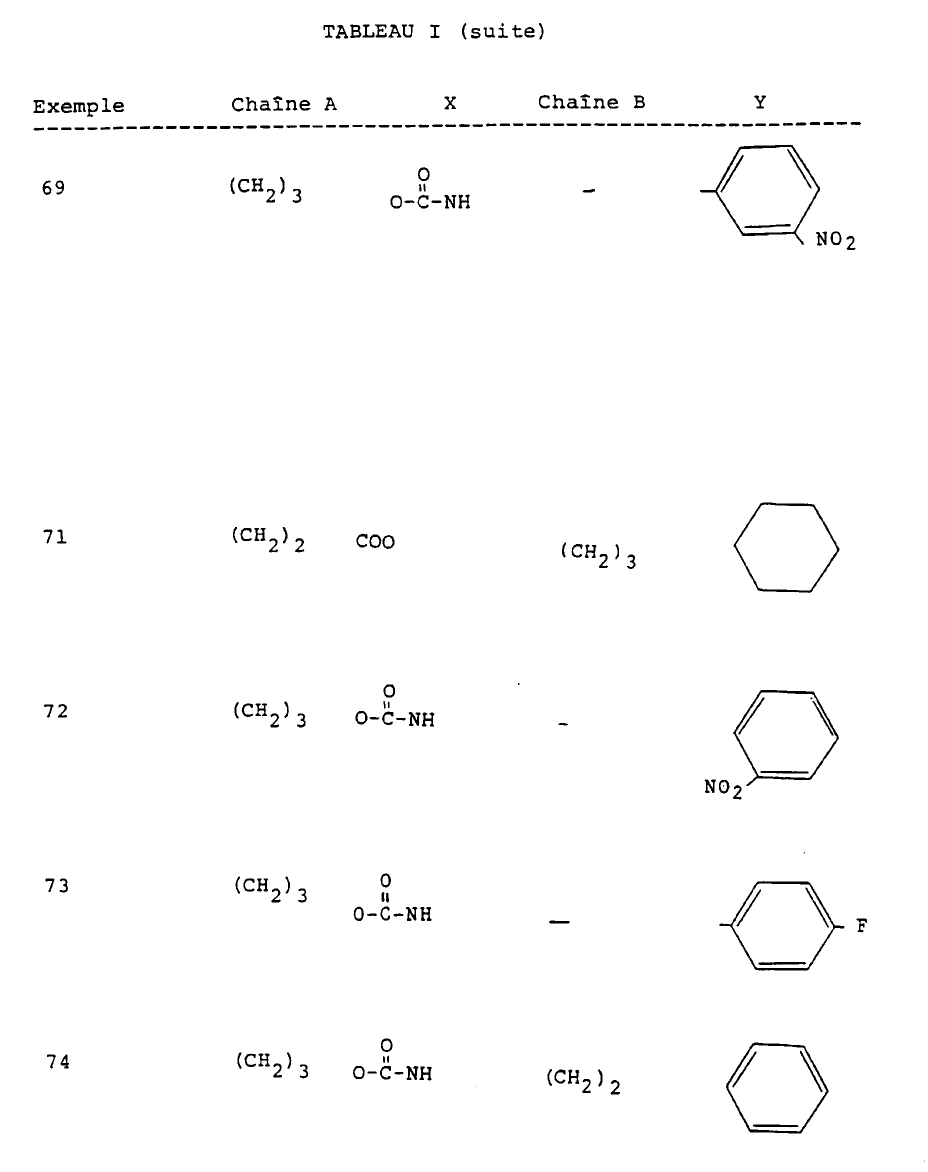

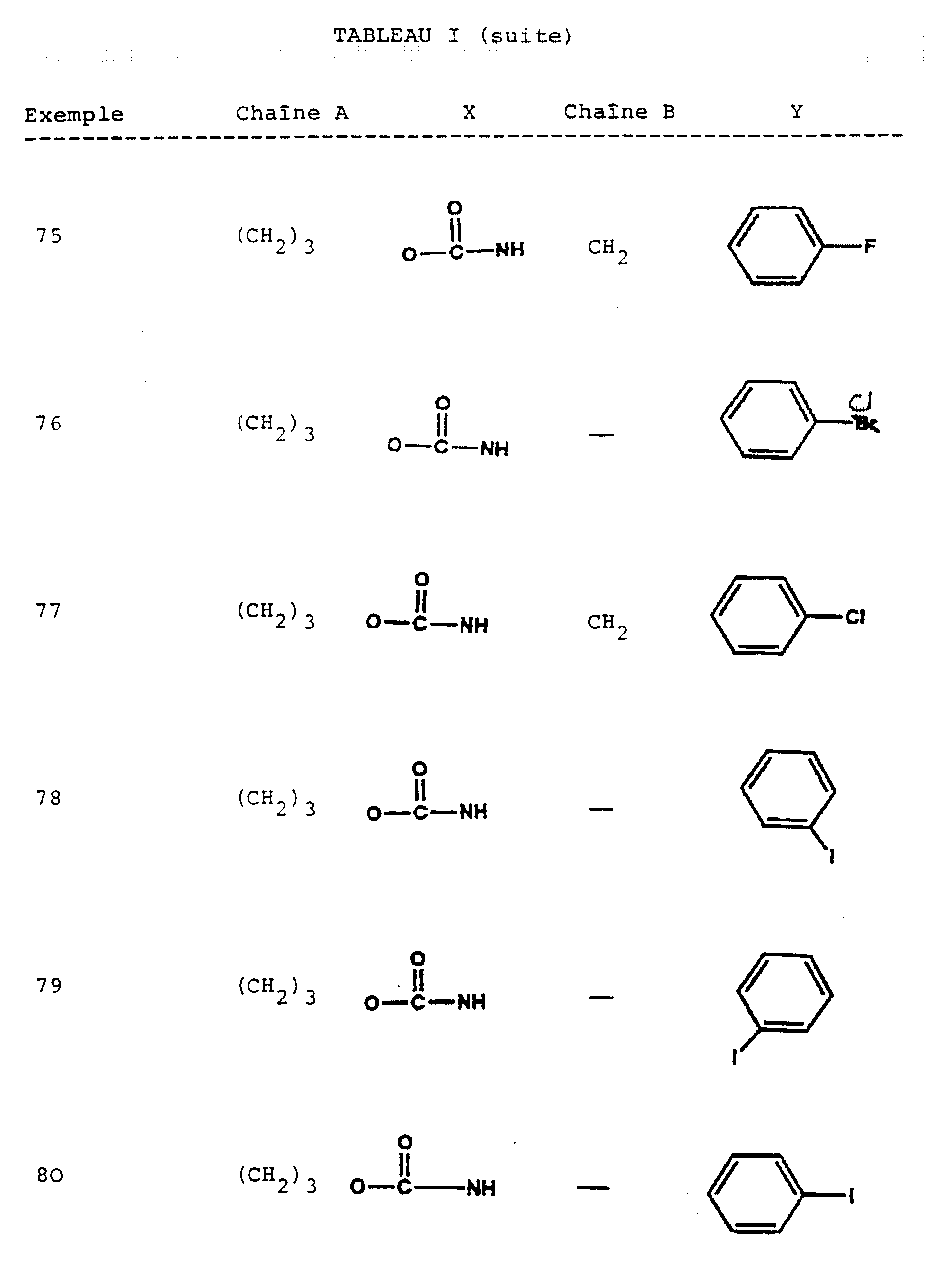

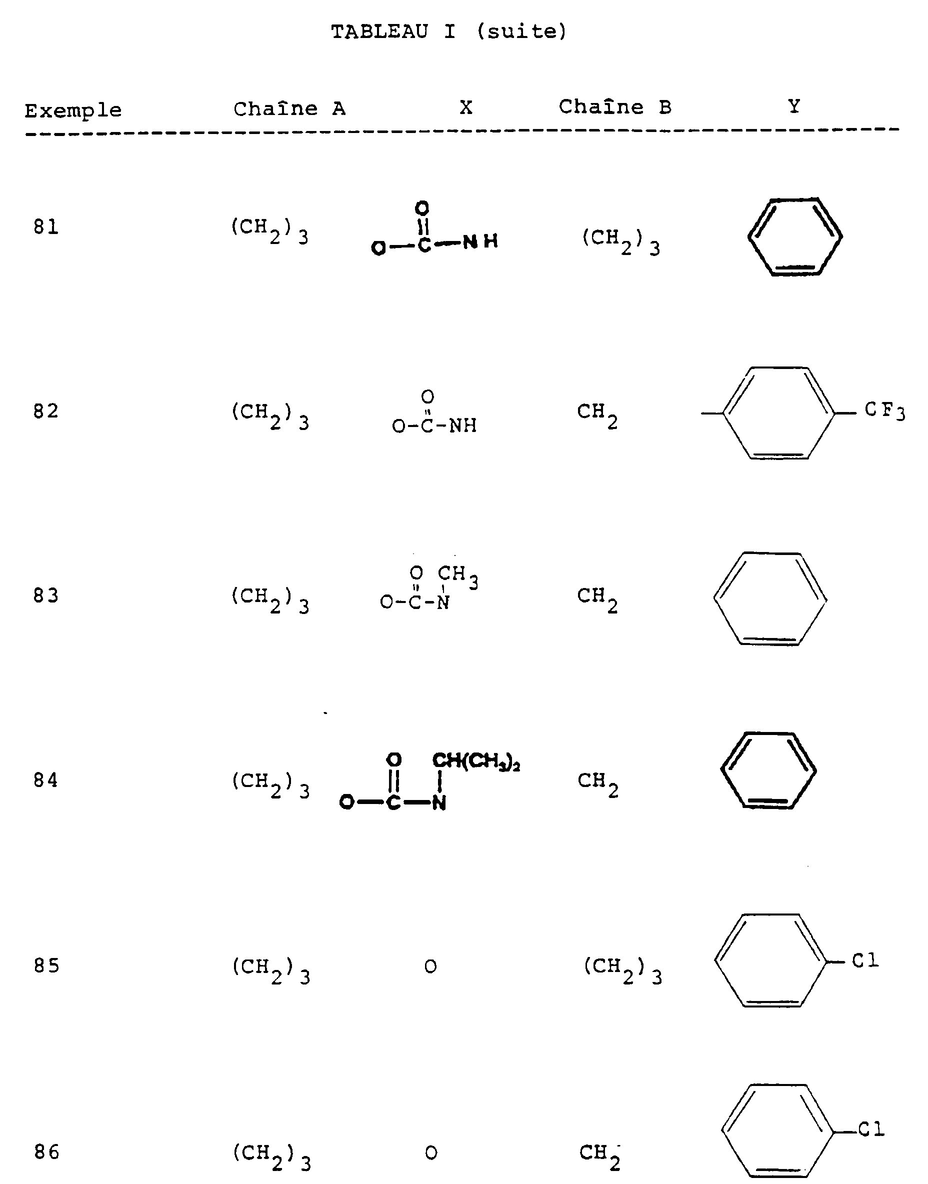

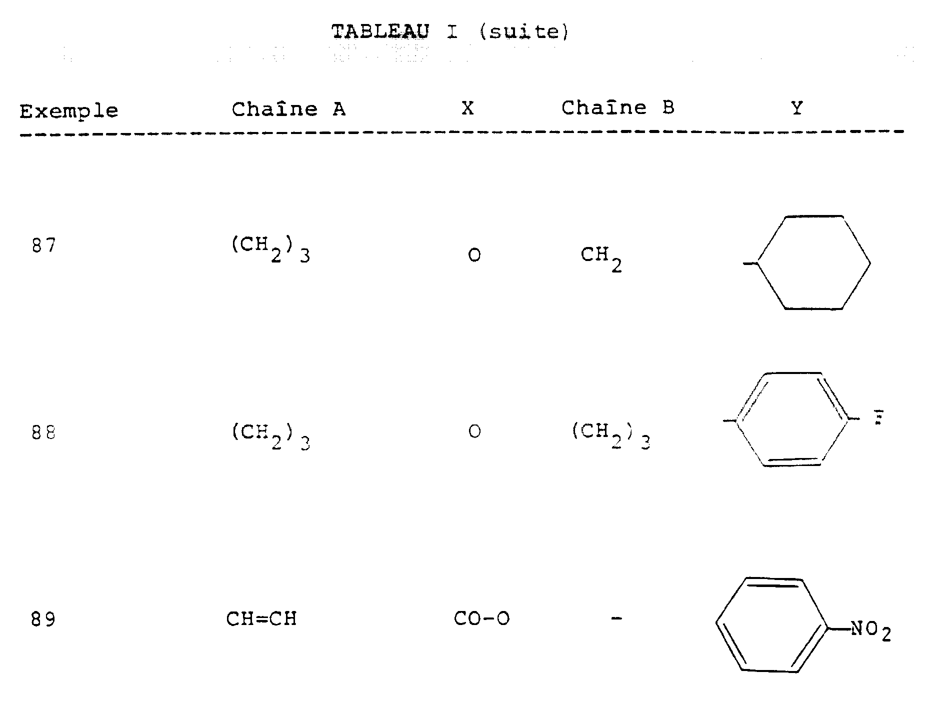

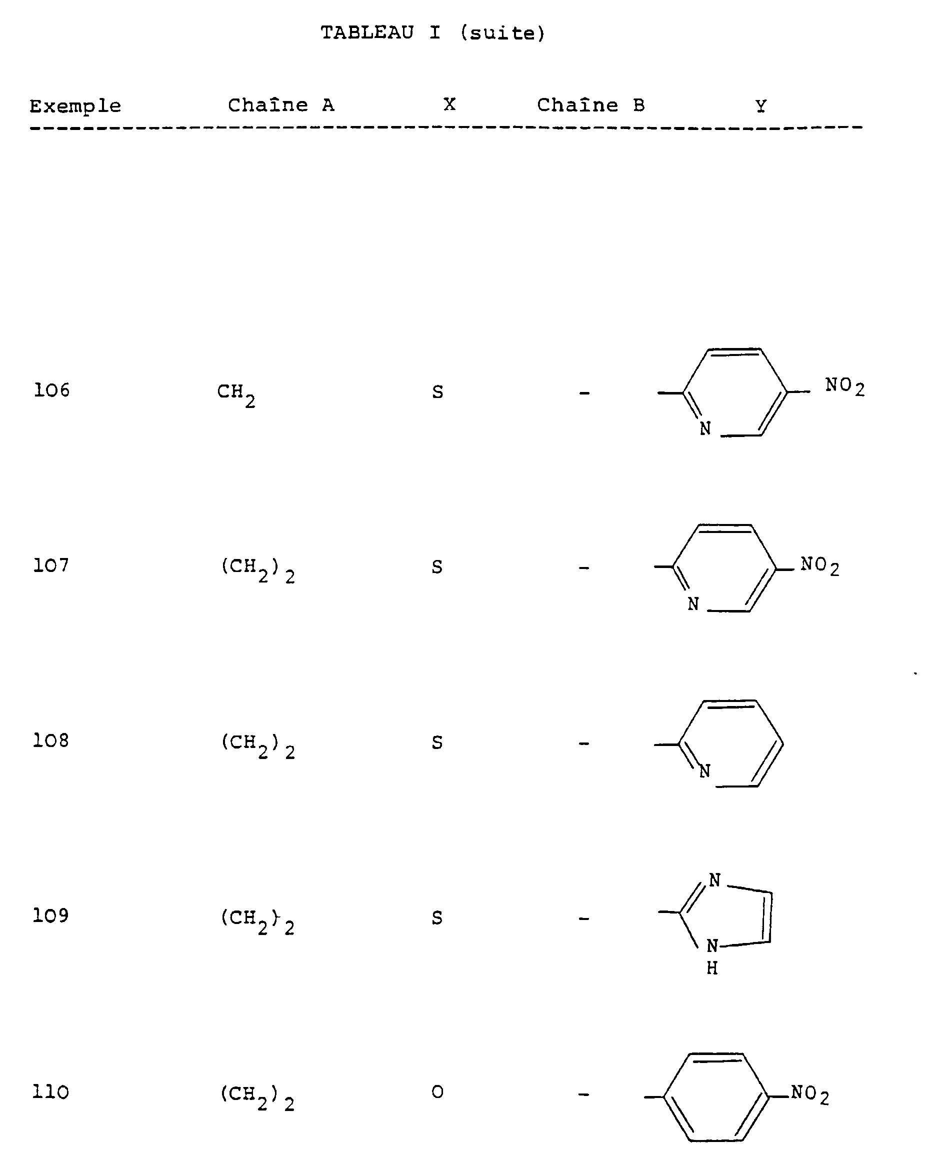

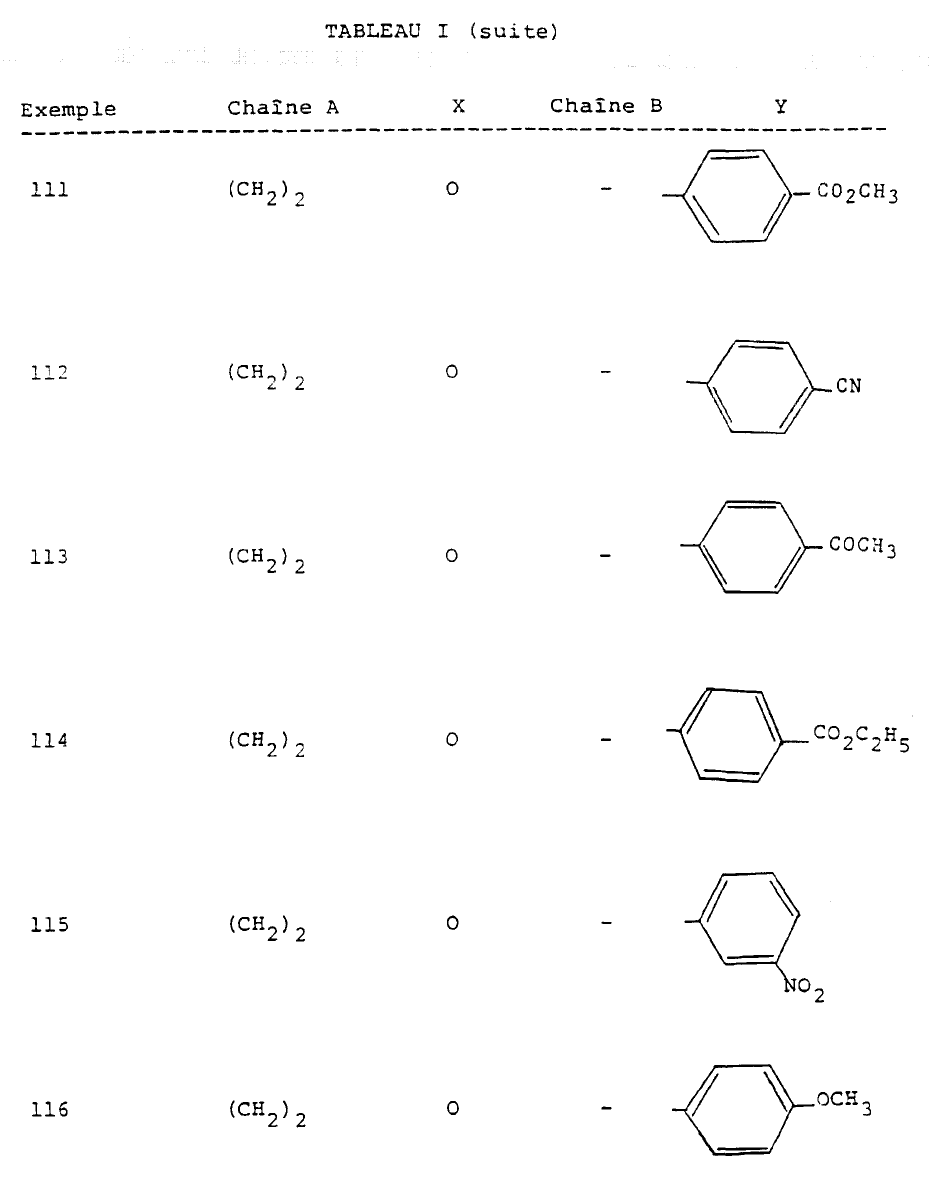

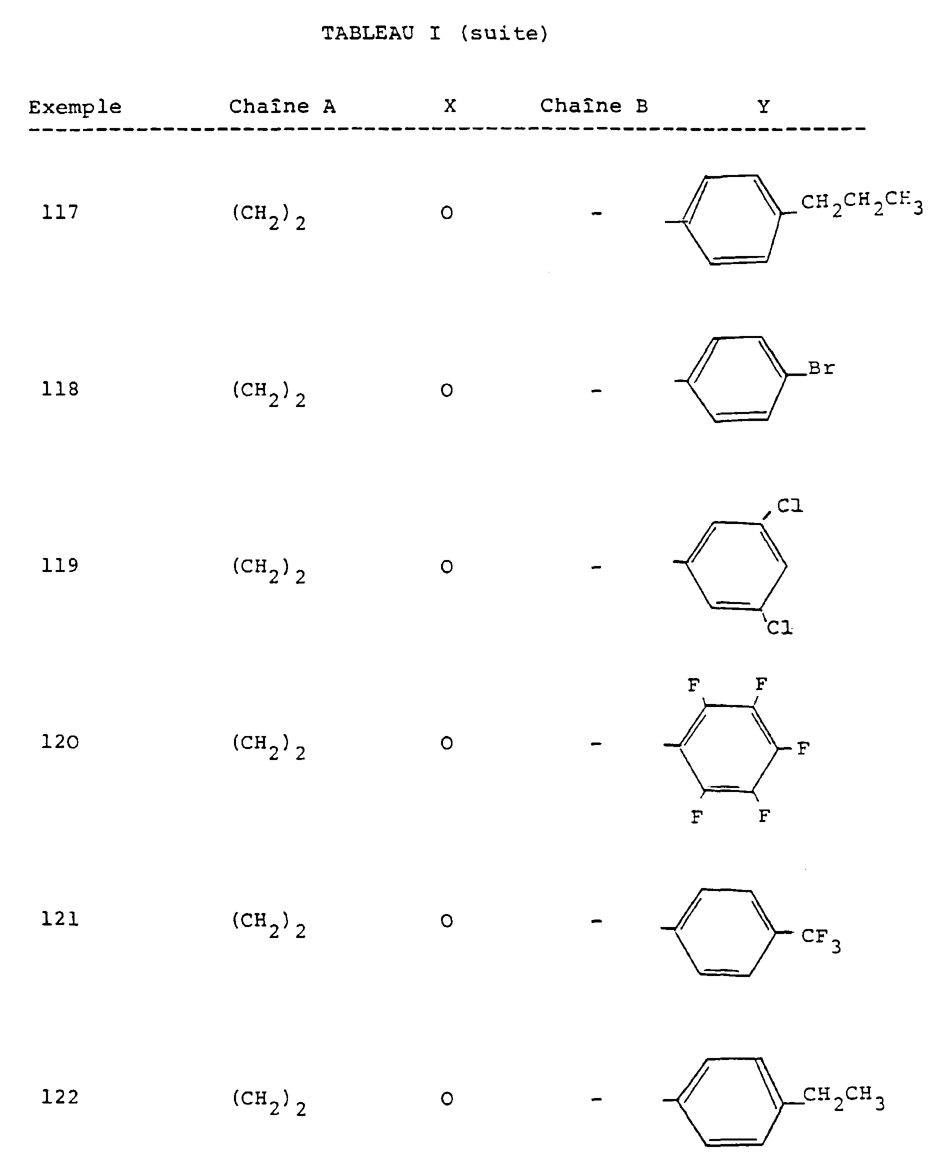

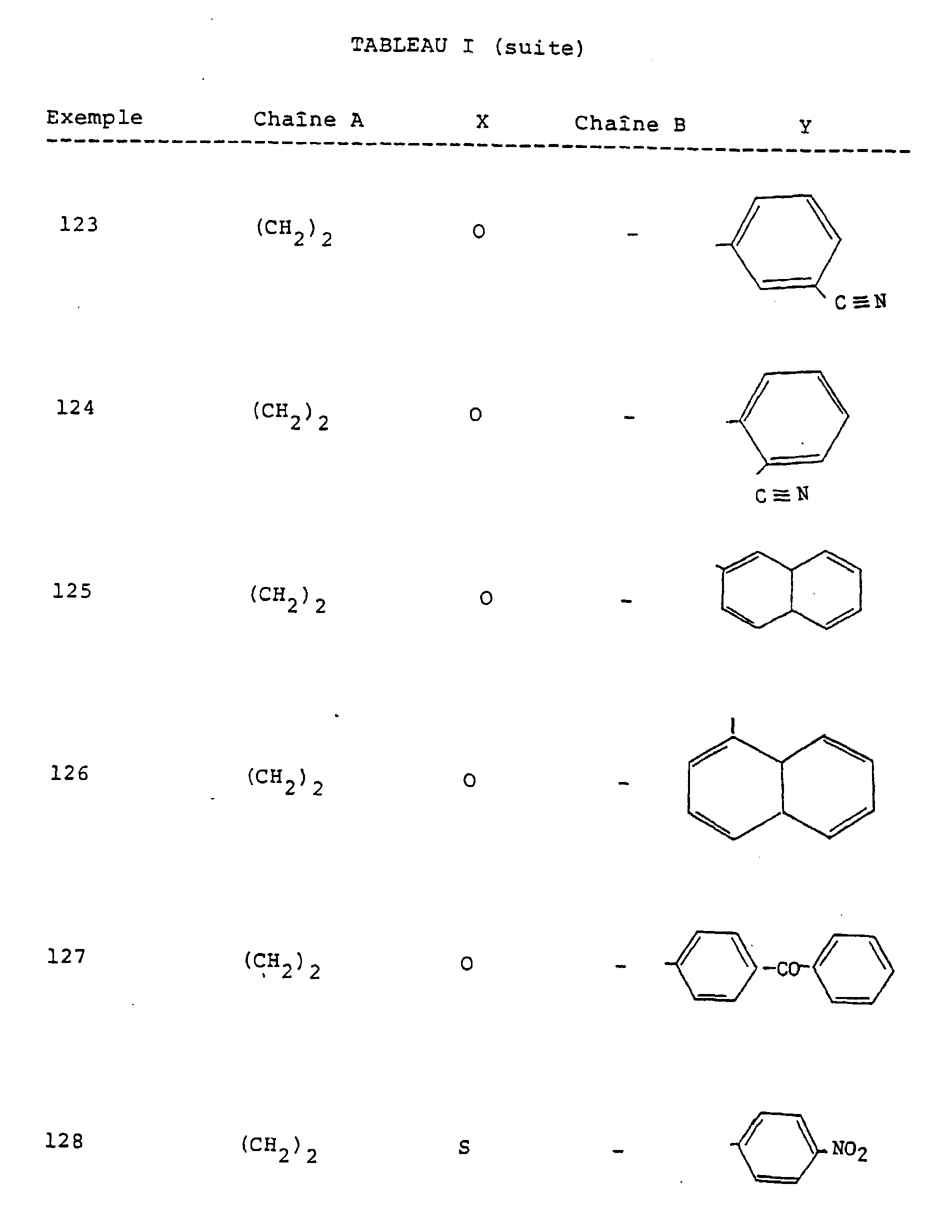

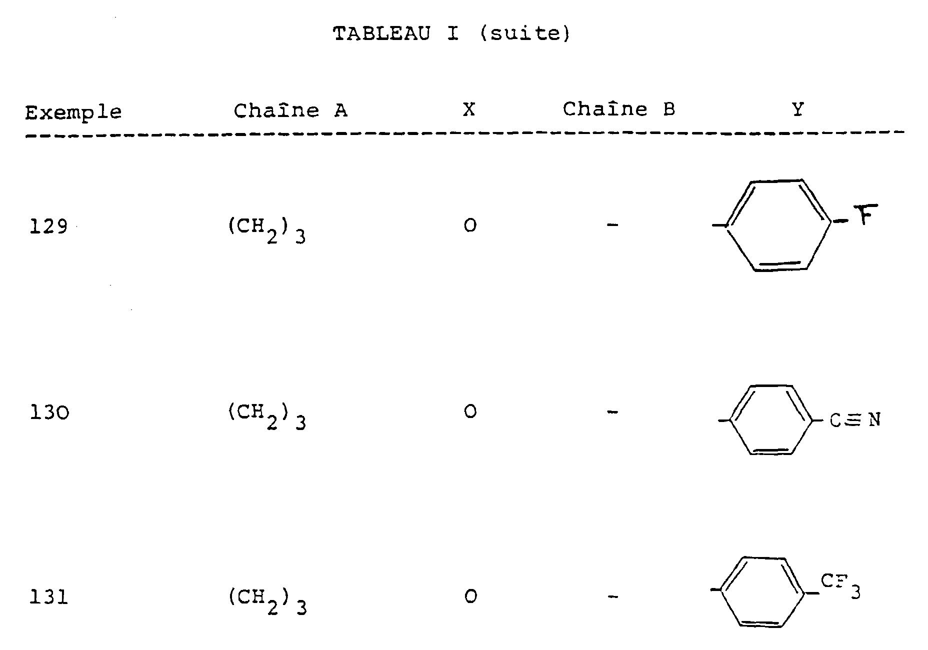

### ETUDE PHARMACOLOGIQUE

Les composés de formule IA ou IB conformes à l'invention provoquent, in vitro, un blocage des récepteurs histaminergiques H₃ contrôlant la libération et la formation d'histamine cérébrale, et in vivo, une augmentation de la vitesse de renouvellement de l'histamine cérébrale, effets établissant notamment une action psychotrope.

L'antagonisme de la stimulation par l'histamine des récepteurs H₃ centraux a été mis en évidence grâce au procédé décrit par Arrang et Coll. (Nature, 1983, 3O2 ; 832-837). Ce procédé fait appel à des coupes de cortex cérébral de rats et a permis la caractérisation pharmacologique des récepteurs H₃ (NATURE, 1987, 327, 117-123).

L'histamine exogène (à la concentration de 1µM) produit une inhibition de libération d'environ 5O%. Cet effet est progressivement renversé en présence d'antagonistes H₃ tels que les composés de l'invention, ajoutés en concentrations croissantes. La concentration de ces derniers pour laquelle l'effet de l'histamine exogène est réduit de moitié (CI_{5O}) est déterminée et la constante apparente d'inhibition (Ki) est alors calculée suivant Cheng et Prusoff (Biochim. Pharmacol. 1973, 22, 3O99-31O8), en tenant compte de la concentration efficace 5O % de l'histamine (CE = O,1 µM). Les résultats sont rassemblés dans le tableau II suivant.

**TABLEAU II**

| CONSTANTES APPARENTES DE DISSOCIATION (Ki) de DIVERS DERIVES DE L'INVENTION COMME ANTAGONISTES DE L'HISTAMINE SUR LES RECEPTEURS H₃ DU CERVEAU DE RAT. | |
|---|---|
| Exemple N° | Ki(nM) |
| 24 | 3 |
| 36 | 20±8 |
| 38 | 15±4 |
| 45 | 17±3 |
| 49 | 20 |
| 61 | 14±8 |
| 73 | 24±4 |
| 85 | 13±4 |
| 88 | 7±2 |
| 110 | 35±6 |
| 112 | 9±5 |
| 117 | 19±9 |
| 125 | 90±27 |
| 130 | 12±3 |
| 131 | 14±6 |

Les composés de l'invention provoquent in vivo, après administration intrapéritonéale ou orale chez le rat, une augmentation de la vitesse de renouvellement de l'histamine cerébrale. Cette dernière est estimée, soit par l'étude de la décroissance du taux de l'histamine cérébrale après blocage de sa synthèse (Garbarg et Coll., Europ. Jr. Pharmacol. 164, 1-11, 1989), soit par l'étude de l'augmentation du taux catabolite de l'histamine, la téléméthylhistamine (Garbarg et Coll., J. Neurochem. 53, 1724-173O, 1989).

Cette propriété d'antagonistes H₃ actifs par voie générale fait des composés de l'invention des dérivés utiles en médecine humaine et vétérinaire. Leurs applications thérapeutiques concernent notamment le système nerveux central (y compris comme psychostimulants). Les composés de formule IA ou IB sont avantageusement utilisés comme principe actif de médicaments agissant en tant qu'antagoniste des récepteurs H₃ de l'histamine, de médicaments à effets sédatifs, régulateur du sommeil, anticonvulsivant, psychostimulant, modulateur de la circulation cérébrale, antidépresseur, antiulcéreux.

La présente invention concerne donc également les compositions pharmaceutiques qui contiennent, à titre de principe actif, une quantité thérapeutiquement efficace d'un des composés de formule IA ou IB.

La composition pharmaceutique conforme à l'invention est administrable à l'homme par voie orale, perlinguale, nasale, rectale et parentérale, le principe actif étant associé à un excipient ou véhicule thérapeutiquement convenable.

Chaque dose unitaire contient avantageusement de 0,1 à 100 mg de principe actif, les doses administrables journellement pouvant varier de 0,3 mg à 300 mg de principe actif.

L'invention a aussi pour objet l'utilisation des dérivés conformes à l'invention pour la préparation de médicaments antagonistes H₃ selon les modalités précitées.

Des composés analogues de formules IA et IB dans lesquelles
a) X représente -NH-, la chaîne A le groupe -(CH₂)₂-, la chaîne B le groupement -(CH₂)₂-O- ou -(CH₂)ₙ-S- et Y le groupe phényle ou p-chlorophényle,
b) X représente le groupe -NHCO-, la chaîne A le groupe -(CH₂)₂ et Y le groupe méthyle (formule IB) ou la chaîne B et Y (formule IA) représentent une chaîne alkylène droite -(CH₂)ₙ-, n étant compris entre 1 et 4, les groupes -CH₂-O-, -CH₂-S-CH₂- et un groupe phényle, ou encore les groupes -CH₂-CH₂-, -CH₂-S-CH₂- et le groupe diphényle, ou encore les groupes -(CH₂)₃-, -CH₂-S-CH₂- et le groupe pyridyle, ou encore les groupes -CH₂-CH₂-, -CH₂-S-et le groupe diphényle, ou encore le groupe -(CH₂)₃- et le groupe imidazolyle ou cyclohexyle,
c) X représente -NHCO-, la chaîne A le groupe -CH₂-CH(CH₃)-, la chaîne B le groupe -(CH₂)₃- et Y le groupe phényle,
d) X représente -NHCSNH- ou -NHCONH-, la chaîne A le groupe -(CH₂)₂-, la chaîne B le groupe -(CH₂)₂- et Y le groupe phényle,
et leurs propriétés antagonistes des récepteurs H₃ de l'histamine ont été divulgués lors d'un Symposium qui s'est tenu à Budapest on Août 1988 ("10th International Symposium on Medicinal Chemistry") et plus récemment à Noordwijkerhout (Juillet 1990).

L'invention a également pour objet l'utilisation des dérivés conformes à l'invention pour la préparation de médicaments à effet sédatif, régulateur du sommeil, anti-convulsivant, psychotrope, psychostimulant, modulateur de la circulation cérébrale, antidépresseur ou antiulcéreux.

L'invention concerne encore une méthode de traitement des affections précipitées selon laquelle on administre un médicament contenant une dose thérapeutiquement efficace d'un composé de formule générale IA ou IB éventuellement associé à un véhicule ou excipient thérapeutiquement acceptable.

## Revendications

1. Composés répondant à la formule générale IA ou IB 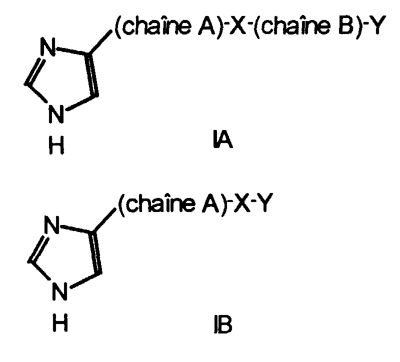 dans laquelle
la chaîne A représente une chaîne hydrocarbonée, droite ou ramifiée, saturée ou insaturée, comportant 1 à 6 atomes de carbone, la chaîne hydrocarbonée saturée pouvant être interrompue par un atome de soufre,
X représente un atome d'oxygène ou de soufre, -OCO-, -COO-, -OCONH-, -OCON(alkyle en C₁-C₃), -OCONHCO-, -CONH-, -SO-, -CO- ou -CHOH-.
la chaîne B représente une chaîne alkylène droite -(CH₂)ₙ-, n étant un nombre entier pouvant varier entre 0 et 5 ou une chaîne alkylène ramifiée renfermant de 2 à 8 atomes de carbone, la chaîne alkylène pouvant être interrompue par un ou plusieurs atomes d'oxygène ou de soufre, ou une chaîne -(CH₂)ₘ-O- ou -(CH₂)ₘ-S- où m est un nombre entier égal à 1 ou 2,
Y représente un groupe alkyle droit ou ramifié comportant 1 à 8 atomes de carbone ; un cycloalkyle comportant 3 à 6 atomes de carbone ; un groupe bicycloalkyle ; un groupe cycloalkényle ; un groupe naphtyle ; un groupe phényle non substitué ou mono- ou polysubstitué par un atome d'halogène, un groupe alkyle comportant au plus 6 atomes de carbone, un groupe CF₃, CN, COCH₃, COOR₁ ou OR₁, R₁ représentant un groupe alkyle comportant au plus 6 atomes de carbone, un groupe NO₂ ou NR₂R₃, R₂ et R₃ représentant un atome d'hydrogène et/ou un radical alkyle comportant au plus 6 atomes de carbone ; un radical hétérocyclique à 5 ou 6 chaînons comportant un ou deux hétéroatomes choisis parmi les atomes d'azote et de soufre, ledit radical hétérocyclique étant non substitué ou mono- ou polysubstitué par un groupe NO₂, CF₃, CH₃, NH₂, un atome d'halogéne ou le groupe COOCH₃; ou encore un radical bicyclique résultant de l'accolement d'un cycle benzénique à un hétérocycle tel que défini précédemment,
sous réserve que, lorsque X représente un atome d'oxygène, la chaîne A un groupe -CH₂-, Y dans la formule IB ne peut représenter un groupe phényle substitué ;
sous réserve que, lorsque X représente -CONH-, la chaîne A le groupe -(CH₂)₂-, Y dans la formule IB ne peut représenter un groupe phényle non substitué ou substitué ;
sous réserve que, lorsque X représente le groupe -CO-, la chaîne A le groupe -(CH₂)₂-, Y dans la formule IB ne peut représenter un radical naphtyle ou un radical phényle non substitué ou substitué, un radical hétérocyclique à 5 chaînons comportant le soufre comme hétéroatome, non substitué ou substitué, un radical bicyclique résultant de l'accolement d'un cycle benzénique à un hétérocycle à 5 ou 6 chaînons comportant comme hétéroatomes les atomes d'azote et/ou de soufre ;
sous réserve que, lorsque X représente le groupe -CO- et A représente un groupe méthylène, B et Y ne peuvent représenter simultanément -C(CH₃)₂-(CH₂)ₚ- (p = 0 ou 1) et un groupe naphtyle ou un groupe phényle substitué ou non substitué
ainsi que leurs sels pharmaceutiquement acceptables, leurs hydrates, leurs sels hydratés, les structures cristallines polymorphiques et les formes tautomères de ces composés.

2. Composés selon la revendication 1, caractérisés en ce que la chaîne A est une chaîne alkylène droite -(CH₂)ₙ-, n étant un nombre entier compris entre 1 et 6.

3. Composés selon l'une des revendications 1 et 2, caractérisés en ce que la chaîne A est une chaîne alkylène substituée par un ou plusieurs radicaux méthyle ou éthyle.

4. Composés selon l'une quelconque des revendications 1 à 3, caractérisés en ce que Y représente un groupement cycloalkyle choisi parmi le cyclopentyle, le cyclohexyle ou un groupe bicycloalkyle.

5. Composés selon l'une des revendications 1 à 3, caractérisés en ce que Y représente un groupe phényle mono- ou poly substitué.

6. Composés selon l'une quelconque des revendications 1 à 3, caractérisés en ce que Y représente un radical hétérocyclique choisi parmi le radical pyridyle, le radical pyridyle N-oxyde ou le radical pyrazinyle, non substitué ou mono- ou polysubstitué par NO₂, CF₃, CH₃, NH₂, un halogène, COOCH₃ ou encore le radical imidazolyle ou le radical thiazyle.

7. Composés selon l'une quelconque des revendications 1 à 3, caractérisés en ce que Y représente un radical bicyclique résultant de la condensation d'un cycle benzénique et d'un hétérocycle tel que défini dans la revendication 1.

8. Composés selon l'une des revendications 1 à 7, caractérisés en ce qu'ils sont choisis parmi :
- le N-(3-phénylpropyl)-3-(1H-imidazol-4-yl)propanamide,
- l'ester 3-(1H-imidazol-4-yl)propylique de l'acide 3-cyclopentylpropanoïque,
- l'ester 3-(1H-imidazol-4-yl)propylique de l'acide benzoïque,
- l'ester 3-(1H-imidazol-4-yl)propylique de l'acide 4-iodobenzoïque,
- l'ester 3-(1H-imidazol-4-yl)propylique de l'acide 3-iodobenzoïque,
- l'ester 3-(1H-imidazol-4-yl)propylique de l'acide 3-iodo-4-méthylbenzoïque,
- l'ester 3-(1H-imidazol-4-yl)propylique de l'acide 3-(4-iodophényl)propylique,
- l'ester 3-(1H-imidazol-4-yl)propylique de l'acide 4-amino-3,5-diiodobenzoïque,
- l'ester 3-(1H-imidazol-4-yl)propylique de l'acide 4-(4-iodophényl)butanoïque,
- l'ester 3-(1H-imidazol-4-yl)propylique de l'acide 2-(4-iodophényl)éthanoïque,
- l'ester 3-(1H-imidazol-4-yl)propylique de l'acide 4-phénylbutanoïque,
- le N-benzyl-3-(1H-imidazol-4-yl)propyloxycarbamide,
- le N-cyclohexylméthyl-3-(1H-imidazol-4-yl) propyloxycarbamide,
- le (3-cyclohexylpropyl)-3-(1H-imidazol-4-yl)propyléther
- le 3-(3,4-difluorophényl)propyl-3-(1H-imidazol-4-yl) propyléther,
- le 3-(4-bromophényl)propyl-3-(1H-imidazol-4-yl) propyléther,
- le 3-(3-trifluorométhylphényl)propyl-3-(1H-imidazol-4yl) propyléther,
- le 1-naphtylméthyl-3-(1H-imidazol-4-yl)propyléther,
- le (4-iodophényl)méthyl-3-(1H-imidazol-4-yl)propyléther,
- le 4-phénylbutyl-3-(1H-imidazol-4-yl)propyléther,
- le (3-phénylpropyl)-3-(1H-imidazol-4-yl)propyléther,
- le (3-phénylpropyl)-3-(1H-imidazol-4-yl)propylthioéther,
- le 4-[(4-nitrobenzythio)méthyl]-1H-imidazol,
- le (3-phénylpropyl)-3-(1H-imidazol-4-yl)propylsulfoxyde,
- la 1-(1H-imidazol-4-yl)-7-phénylheptan-4-one,
- le 1-(1H-imidazol-4-yl)-7-phénylheptan-4-ol,
- le N-benzoyl-3-(1H-imidazol-4-yl)propyloxycarbamide,
- le N-cyclobutylméthyl-3-(1H-imidazol-4-yl)propyloxycarbamide,
- le N-cyclopropylmethyl-3-(1H-imidazol-4-yl)propyloxycarbamide,
- le (R)-(+)-N-1-phényléthyl-3-(1H-imidazol-4-yl)propyloxycarbamide,
- le (S)-(-)-1-phényléthyl-3-(1H-imidazol-4-yl)propyloxyloxycarbamide,
- le N-cyclohexyl-3-(1H-imidazol-4-yl)propyloxycarbamide,
- le N-phényl-3-(1H-imidazol-4-yl)propyloxycarbamide,
- le N-(4-méthylphényl)-3-(1H-imidazol-4-yl) propyloxycarbamide,
- le N-(4-trifluorométhylphényl)-3-(1H-imidazol-4-yl) propyloxycarbamide,
- le N-(3-trifluorométhylphényl)-3-(1H-imidazol-4-yl) propyloxycarbamide,
- le N-(2-trifluorométhylphényl)-3-(1H-imidazol-4-yl) propyloxycarbamide,
- le N-(2-phényléthyl )-2-(1H-imidazol-4-yl)éthoxycarbamide,
- le N-(4-nitrobenzyl)-3-(1H-imidazol-4-yl) propyloxycarbamide,
- le N-(4-aminobenzyl)-3-(1H-imidazol-4-yl) propyloxycarbamide,
- le N-(3-nitrophényl)-3-(1H-imidazol-4-yl) propyloxycarbamide,
- l'ester 3-cyclohexylpropylique de l'acide 3-(1H-imidazol-4-yl)propanoïque,
- le N-(2-nitrophényl)-3-(1H-imidazol-4-yl) propyloxycarbamide,
- le N-(4-fluorophényl)-3-(1H-imidazol-4-yl) propyloxycarbamide,
- le N-2-(phényléthyl)-3-(1H-imidazol-4-yl) propyloxycarbamide,
- le N-(4-fluorobenzyl)-3-(1H-imidazol-4-yl) propyloxycarbamide,
- le N-(4-bromophényl)-3-(1H-imidazol-4-yl) propyloxycarbamide,
- le N-(4-chlorobenzyl-3-(1H-imidazol-4-yl) propyloxycarbamide,
- le N-(3-iodophényl)-3-(1H-imidazol-4-yl) propyloxycarbamide,
- le N-(2-iodophényl)-3-(1H-imidazol-4-yl) propyloxycarbamide,
- le N-(4-iodophényl)-3-(1H-imidazol-4-yl) propyloxycarbamide,
- le N-(3-phénylpropyl)-3-(1H-imidazol-4-yl) propyloxycarbamide,
- le N-(4-trifluorométhylbenzyl)-3-(1H-imidazol-4-yl) propyloxycarbamide,
- le N-benzyl-N-méthyl-3-(1H-imidazol-4-yl) propyloxycarbamide,
- le N-benzyl-N-isopropyl-3-(1H-imidazol-4-yl) propyloxycarbamide,
- le 3-(4-chlorophényl)propyl-3-(1H-imidazol-4-yl) propyléther,
- le (4-chlorophényl)méthyl-3-(1H-imidazol-4-yl)propyléther,
- le cyclohexylméthyl-(1H-imidazol-4-yl)méthyléther,
- le 3-(4-fluorophényl)propyl-3-(1H-imidazol-4-yl)propyléther,
- le p-nitrophényl-3-(1H-imidazol-4-yl)-2-transpropénoate,
- la 2-[1H-imidazol-4-yl]méthylthio-5-nitropyridine,
- la 2-(2-[1H-imidazol-4-yl]éthylthio)-5-nitropyridine,
- la 2-(2-[1H-imidazol-4-yl]éthyl)-5-thiopyridine,
- le 2-(2-[1H-imidazol-4-yl]éthyl)-thio-1H-imidazol,
- le 4-[2-(4-nitrophénoxy)éthyl]-1H-imidazol,
- le 4-[2-(4-carbométhoxyphénoxy)éthyl]-1H-imidazol,
- le 4-[2-(4-cyanophénoxy)éthyl]-1H-imidazol,
- le 4-[2-(4-acétylphénoxy)éthyl]-1H-imidazol,
- le 4-[2-(4-éthoxycarbonyl)phénoxy)éthyl]-1H-imidazol,
- le 4-[2-(3-nitrophénoxy)éthyl]-1H-imidazol,
- le 4-[2-(4-méthoxyphénoxy)éthyl]-1H-imidazol,
- le 4-[2-(4-propylphénoxy)éthyl]-1H-imidazol,
- le 4-[2-(4-bromophénoxy)éthyl]-1H-imidazol,
- le 4-[2-(3,5-dichlorophénoxy)éthyl]-1H-imidazol,
- le 4-[2-(2,3,4,5,6-pentafluorophénoxy)éthyl]-1H-imidazol,
- le 4-[2-(4-trifluorométhylphénoxy)éthyl]-1H-imidazol,
- le 4-[2-(4-éthylphénoxy)éthyl]-1H-imidazol,
- le 4-[2-(3-cyanophénoxy)éthyl]-1H-imidazol,
- le 4-[2-(2-cyanophénoxy)éthyl]-1H-imidazol,
- le 4-[2-(2-naphtyloxy)éthyl]-1H-imidazol,
- le 4-[2-(1-naphtyloxy)éthyl]-1H-imidazol,
- le 4-[2-(4-nitrophénylthio)éthyl]-1H-imidazol,
- le 4-[3-(4-fluorophénoxy)propyl-1H-imidazol,
- le 4-[3-(4-cyanophénoxy)propyl-1H-imidazol,
- le 4-[3-(4-trifluorométhylphénoxy)propyl-1H-imidazol.

9. Le 4-[2-(4-benzoylphénoxy)ethyl]-1H-imidazol.

10. Procédé de préparation des composés selon la revendication 1, de formule IA ou IB dans lesquelles X représente -OCO-, la chaîne A, la chaîne B et Y ayant les significations données dans la revendication 1, caractérisé en ce que l'on fait réagir un chlorure d'acide de formule
ClCO-(chaîne B)-Y
ou
ClCO-Y
sur un alcool, se présentant sous forme de chlorohydrate, de formule

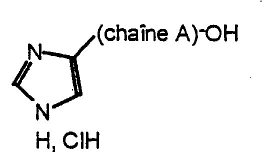

11. Procédé de préparation des composés selon la revendication 1, de formule IA ou IB dans lesquelles X représente -CO-O-, la chaîne A, la chaîne B et Y ayant les significations données dans la revendication 1, caractérisé en ce que l'on fait réagir un acide de formule 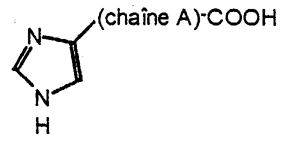 avec un alcool de formule
HO ― (Chaîne B) ― Y
ou
HO ― Y
en présence de chlorure de thionyle.

12. Procédé de préparation des composés selon la revendication 1, de formule IA ou IB dans lesquelles X représente -OCONH-, la chaîne A, la chaîne B et Y ayant les significations données dans la revendication 1, caractérisé en ce que l'on fait réagir un alcool, se présentant sous forme de chlorhydrate, de formule 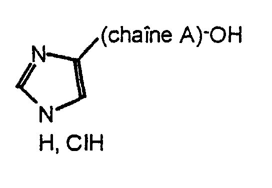 avec un isocyanate de formule
OCN-(chaîne B)-Y
ou
OCN ― Y
en présence d'un solvant apolaire.

13. Procédé de préparation des composés selon la revendication 1 de formule IA ou IB dans lesquelles X représente -O-, la chaîne A, la chaîne B et Y ayant les significations données dans la revendication 1, caractérisé en ce que l'on fait réagir un alcoolate de formule 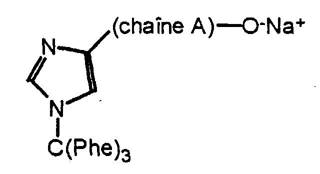 Phe désignant le radical phényle,
avec un composé halogéné de formule
Hal ― (chaîne B) ― Y
ou
Hal ― Y
Hal désignant un halogène,
en présence d'un solvant neutre
puis en clivant le groupement -C(Phe)₃ par une solution acide.

14. Procédé de préparation des composés selon la revendication 1 de formule IA ou IB dans lesquelles X représente -O-, la chaîne A, la chaîne B et Y ayant les significations données dans la revendication 1, caractérisé en ce que l'on fait réagir un composé halogéné, se présentant sous forme de chlorhydrate, de formule 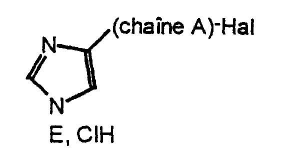 Hal désignant un halogène avec un alcool de formule
HO ― (chaîne B) ― Y
ou
HO ― Y

15. Procédé de préparation des composés selon la revendication 1 de formule IB dans laquelle X représente -O-, la chaîne A a la signification donnée dans la revendication 1 et Y représente un groupe phényle éventuellement substitué, caractérisé en ce que l'on fait réagir un alcool de formule 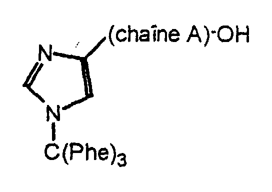 avec un composé phénolique de formule 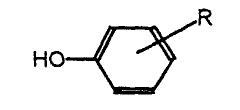 dans laquelle R représente le substituant du groupe phényle de Y, en présence de triphénylphosphine et de diéthylazodicarboxylate dans un solvant et en ce que l'on clive le groupe -C(Phe)₃ par traitement par une solution acide.

16. Procédé de préparation des composés selon la revendication 1, de formule IA ou IB dans lesquelles X représente -S-, la chaîne A, la chaîne B et Y ayant les significations données dans la revendication 1, caractérisé en ce que l'on fait réagir une isothiourée de formule 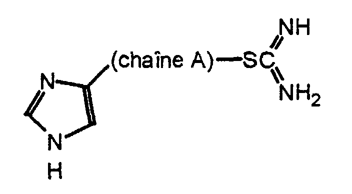 avec un composé halogéné de formule
Hal ― (chaîne B) ― Y
ou
Hal ― Y
Hal désignant un halogène, en présence d'un solvant.

17. Procédé de préparation des composés selon la revendication 1, de formule IA ou IB dans lesquelles X représente -S-, la chaîne A, la chaîne B et Y ayant les significations données dans la revendication 1, caractérisé en ce que l'on fait réagir un alcool de formule 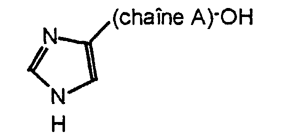 avec un composé de formule
SH ― (chaîne B) ― Y
ou
SH ― Y
en présence d'un hydracide halogéné ou
en faisant réagir un composé halogéné de formule 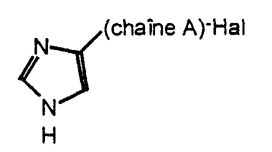 Hal désignant un halogène, avec un composé de formule
SH ― (chaîne B) ― Y
ou
SH ― Y
en présence d'une base.

18. Procédé de préparation des composés selon la revendication 1, de formule IA ou IB dans lesquelles X représente -SO-, la chaîne A, la chaîne B et Y ayant les significations données dans la revendication 1, caractérisé en ce que l'on traite le composé de formule 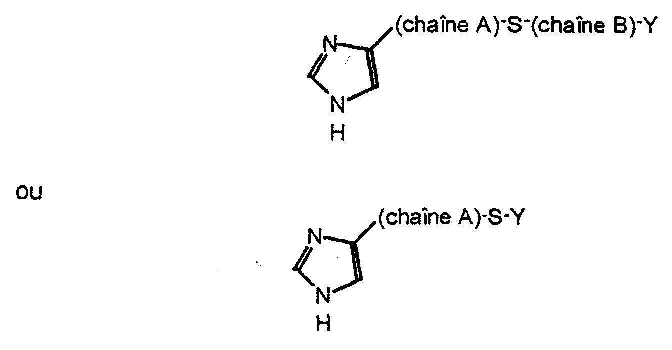 par une base, en présence d'un solvant puis par un agent d'oxydation.

19. Procédé de préparation des composés selon la revendication 1, de formule IA u IB dans lesquelles X représente -CO-, la chaîne A, la chaîne B et Y ayant les significations données dans la revendication 1, caractérisé en ce que l'on fait réagir un composé de formule 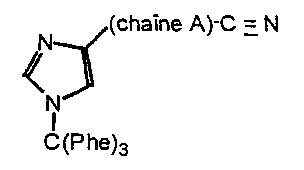 Phe désignant le radical phényle, avec
Hal - Mg - (chaîne B) - Y
ou
Hal ― Mg ― Y
Hal désignant un halogène, en présence d'un solvant, puis en ce que l'on hydrolyse le produit obtenu.

20. Utilisation d'un composé de formule IA ou IB 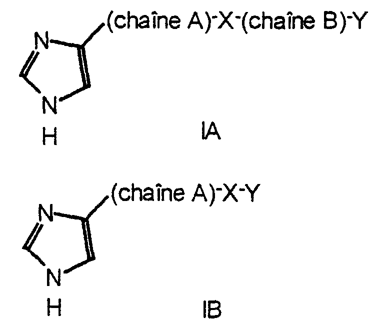 dans laquelle
la chaîne A représente une chaîne hydrocarbonée, droite ou ramifiée, saturée ou insaturée, comportant 1 à 6 atomes de carbone, la chaîne hydrocarbonée saturée pouvant être interrompue par un atome de soufre,
X représente un atome d'oxygène ou de soufre, -O-CO-, -CO-O-, -OCONH-, -OCON-(alkyle en C₁-C₃)-, -OCONH-CO, -CONH-, -SO-, -CO-, -CHOH-,
la chaîne B représente une chaîne alkylène droite -(CH₂)ₙ-, n étant un nombre entier pouvant varier entre 0 et 5 ou une chaîne alkylène ramifiée renfermant de 2 à 8 atomes de carbone, la chaîne alkylène pouvant être interrompue par un ou plusieurs atomes d'oxygène ou de soufre, ou une chaîne -(CH₂)ₘ-O- ou -(CH₂)ₘ-S- où m est un nombre entier égal à 1 ou 2,
Y représente un groupe alkyle droit ou ramifié comportant 1 à 8 atomes de carbone ; un cycloalkyle comportant 3 à 6 atomes de carbone ; un groupe bicycloalkyle ; un groupe cycloalkényle ; un groupe naphtyle ; un groupe phényle non substitué ou mono- ou polysubstitué par un atome d'halogène, un groupe alkyle comportant au plus 6 atomes de carbone, un groupe CF₃, CN, COCH₃, COOR₁ ou OR₁, R₁ représentant un groupe alkyle comportant au plus 6 atomes de carbone, un groupe NO₂ ou NR₂R₃, R₂ et R₃ représentant un atome d'hydrogène et/ou un radical alkyle comportant au plus 6 atomes de carbone ; un radical hétérocyclique à 5 ou 6 chaînons comportant un ou deux hétéroatomes choisis parmi les atomes d'azote et de soufre, ledit radical hétérocyclique étant non substitué ou mono- ou polysubstitué par un groupe NO₂, CF₃, CH₃, NH₂, un atome d'halogène ou le groupe COOCH₃; ou encore un radical bicyclique résultant de l'accolement d'un cycle benzénique à un hétérocycle tel que défini précédemment,
ainsi que de leurs sels pharmaceutiquement acceptables, de leurs hydrates, de leurs sels hydratés, des structures cristallines polymorphiques et des formes tautomères de ces composés
pour la fabrication d'un médicament agissant comme antagoniste des récepteurs H₃ de l'histamine.

21. Utilisation selon la revendication 20, caractérisé en ce que le composé IA ou IB est tel que défini selon l'une quelconque des revendications 2 à 8.

22. Utilisation d'un composé de formule générale IA ou IB selon l'une quelconque des revendications 20 et 21 pour la fabrication d'un médicament contrôlant la synthèse de l'histamine, notamment à effets sédatifs, régulateurs du sommeil, anticonvulsivant, psychostimulant, antidépresseur, antiallergique, anti-secrétoire ou anti-ulcéreux.

23. Médicament agissant comme antagoniste des récepteurs H₃ de l'histamine, contenant, à titre de principe actif, un composé de formule générale IA ou IB, selon l'une quelconque des revendications 1 à 8, éventuellement associé à un véhicule ou excipient thérapeutiquement acceptable.

24. Médicament selon la revendication 23, contenant, à titre de principe actif, un composé choisi dans le groupe défini dans la revendication 8.

25. Médicaments contrôlant la synthèse de l'histamine, notamment à effets sédatifs, régulateur du sommeil, anticonvulsivant, psychostimulant, anti-allergique, anti-secrétoire ou anti-ulcéreux, contenant, à titre de principe actif, un composé de formule générale IA ou IB selon l'une quelconque des revendications 1 à 8, éventuellement associé à un véhicule ou excipient thérapeutiquement acceptable.

26. Médicament selon la revendication 25, contenant à titre de principe actif, un composé choisi dans le groupe défini dans la revendication 8.

27. Utilisation du composé selon la revendication 9 pour la fabrication d'un médicament agissant comme antagoniste des recepteurs H₃ de l'histamine.

## Claims

1. Compounds according to the general formula 1A or 1B 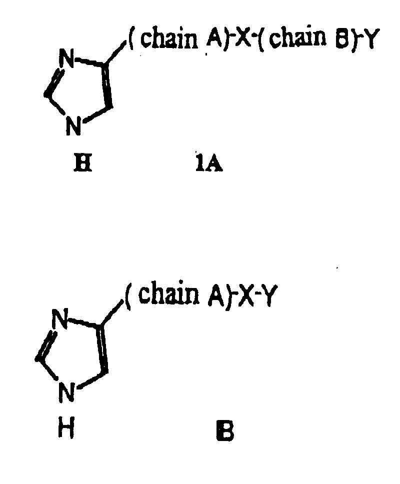 wherein
chain A represents a straight or branched, saturated or unsaturated hydrocarbon chain, containing 1 to 6 carbon atoms, wherein the saturated hydrocarbon chain may be interrupted by a sulphur atom,
X represents an oxygen or a sulphur atom, -OCO-, -COO-, -OCONH-, -OCON-(C₁-C₃ alkyl)-, -OCONHCO-, -CONH-, -SO-, -CO- or -CHOH-,
chain B represents a straight alkylene chain -(CH₂)ₙ-, n being a whole number which may vary between 0 and 5 or a branched alkylene chain containing 2 to 8 carbon atoms, wherein the alkylene chain may be interrupted by one or several oxygen or sulphur atoms, or a -(CH₂)ₘ-O- or - (CR₂)ₘ-S-chain where m is a whole number of 1 or 2,
Y represents a straight or branched alkyl group containing 1 to 8 carbon atoms; a cycloalkyl group containing 3 to 6 carbon atoms; a bicycloalkyl group; a cycloalkenyl group; a naphthyl group; a phenyl group either unsubstituted or mono- or polysubstituted by a halogen atom, an alkyl group containing not more than 6 carbon atoms, a CF₃, CN, COCH₃, COOR₁ or OR₁ group, where R₁ represents an alkyl group containing not more than 6 carbon atoms, an NO₂ or NR₂R₃ group, where R₂ and R₃ represent a hydrogen atom and/or an alkyl radical containing not more than 6 carbon atoms; a 5 or 6-member heterocyclic radical containing one or two heteroatoms selected from the group consisting of nitrogen and sulphur atoms, said heterocyclic radical being unsubstituted or mono- or polysubstituted by an NO_{2,} CF_{3,} CH_{3,} NH₂ group, a halogen atom or the group COOCH_{3,} or a bicyclic radical resulting from the fusion of a benzene ring with a heterocycle as defined previously,
excepting that, when X represents an oxygen atom and chain A represents a -CH₂- group, Y in the formula 1B cannot represent a substituted phenyl group;
excepting that, when X represents -CONH- and chain A represents the -(CH₂)₂- group, Y in the formula 1B cannot represent an unsubstituted or substituted phenyl group;
excepting that, when X represents the -CO- group and chain A represents the -(CH₂)₂-group, Y in the formula 1B cannot represent a naphthyl radical or an unsubstituted or substituted phenyl radical, a 5-member heterocyclic radical containing sulphur as an unsubstituted or a substituted heteroatom, a bicyclic radical resulting from the fusion oh benzene ring with a 5 or 6-member heterocycle containing nitrogen and/or sulphur atoms in the form of heteroatoms;
excepting that; when X represents the -CO- group and A represents a methylene group, B and Y cannot represent simultaneously -C(CR₃)₂-(CH₂)ₚ- (p = 0 or 1) and a napthyl group or a substituted or unsubstituted phenyl group,
as well as their pharmaceutically acceptable salts, their hydrates, their hydrated salts, the polymorphic crystalline structures and the tautomeric forms of these compounds.

2. Compounds according to claim 1, characterized in that chain A is a straight alkylene chain -(CH₂)ₙ-, n being a whole number between 1 and 6.

3. Compounds according to one of claims 1 and 2, characterized in that chain A is an alkylene chain substituted by one or several methyl or ethyl radicals.

4. Compounds according to any one of claims 1 to 3, characterized in that Y represents a cycloalkyl group selected from the group consisting of cyclopentyl, cyclohexyl or a bicycloalkyl group.

5. Compounds according to any one of claims 1 to 3, characterized in that Y represents a mono- or polysubstituted phenyl group.

6. Compounds according to any one of claims 1 to 3, characterized in that Y represents a heterocyclic radical selected from the group consisting of the pyridyl radical, the pyridyl N-oxide radical or the pyrazinyl radical, unsubstituted or mono- or polysubstituted by NO₂, CF₃, CH₃, NH₂, a halogen, COOCH₃ or the imidazolyl radical or the thiazyl radical.

7. Compounds according to any one of claims 1 to 3, characterized in that Y represents a bicyclic radical resulting from the condensation of a benzene ring and of a heterocycle as defined in claim 1.

8. Compounds according to one of claims 1 to 7, characterized in that they are selected from the group consisting of:
- N-(3-phenylpropyl)-3-(1H-imidazole-4-yl)propanamide,
- 3-cyclopentylpropanoic acid 3-(1H-imidazole-4-yl)propyl ester,
- benzoic acid 3-(1H-imidazole-4-yl)propyl ester,
- 4-iodobenzoic acid 3-(1H-imidazole-4-yl)propyl ester,
- 3-iodobenzoic acid 3-(1H-imidazole-4-yl)propyl ester,
- 3-iodo-4-methylbenzoic acid 3-(1H-imnidazole-4-yl)propyl ester,
- 3-(4-iodophenyl)propylic acid 3-(1H-imidazole-4-yl) propyl ester,
- 4-amino-3,5-diiodobenzoic acid 3-(1H-imidazole-4-yl)propyl ester,
- 4-(4-iodophenyl)butanoic acid 3-(1H-imidazole-4-yl)propyl ester,
- 2-(4-iodophenyl)ethanoic acid 3-(1H-imidazole-4-yl)propyl ester,
- 4-phenylbutanoic acid 3-(1H-imidazole-4-yl)propyl ester,
- N-benzyl-3-(1H-imidazole-4-yl)propyloxycarbamide,
- N-cyclohexymethyl-3-(1H-imidazole-4-yl)propyloxycarbamide,
- (3-cyclohexylpropyl)-3-(1H-imidazole-4-yl)propyl ether,
- 3-(3,4-difluorophenyl)propyl-3-(1H-imidazole-4-yl)propyl ether,
- 3-(4-bromophenyl)propyl-3-(1H-imidazole-4-yl)propyl ether,
- 3-(3-trifluoromethylphenyl)propyl-3-(1H-imidazole-4-yl)propyl ether,
- 1-naphthylmethyl-3-(1H-imidazole-4-yl)propyl ether,
- (4-iodophenyl)methyl-3-(1H-imidazole-4-yl)propyl ether,
- 4-phenylbutyl-3-(1H-imidazole-4-yl)propyl ether,
- (3-phenylpropyl)-3-(1H-imidazole-4-yl)propyl ether,
- (3-phenylpropyl)-3-(1H-imidazole-4-yl)propyl thioether,
- 4-[(4-nitrobenzythio)methyl]-1H-imidazole,
- (3-phenylpropyl)-3-(1H-imidazole-4-yl)propyl sulphoxide,
- 1-(1H-imidazole-4-yl)-7-phenylheptan-4-one,
- 1-(1H-imidazole-4-yl)-7-phenylheptan-4-ol,
- N-benzoyl-3-(1H-imidazole-4-yl)propyloxycarbamide,
- N-cyclobutylmethyl-3-(1H-imidazole-4-yl)propyloxycarbamide,
- N-cyclopropylmethyl-3-(1H-imidazole-4-yl)propyloxycarbamide,
- (R)-(+)-N-1-phenylethyl-3-(1H-imidazole-4-yl)propyloxycarbamide,
- (S)-(-)-1-phenylethyl-3-(1H-imidazole-4-yl)propyloxycarbamide,
- N-cyclohexyl-3-(1H-imidazole-4-yl)propyloxycarbamide,
- N-phenyl-3-(1H-imidazole-4-yl)propyloxycarbamide,
- N-(4-methylphenyl)-3-(1H-imidazole-4-yl)propyloxycarbamide,
- N-(4-trifluoromethylphenyl-3-(1H-imidazole-4-yl)propyloxycarbamide,
- N-(3-trifluoromethylphenyl)-3-(1H-imidazole-4-yl)propyloxycarbamide,
- N-(2-trifluoromethylphenyl)-3-(1H-imidazole-4-yl) propyloxycarbamide,
- N-(2-phenylethyl)-2-(1H-imidazole-4-yl)ethoxycarbamide,
- N-(4-nitrobenzyl)-3-(1H-imidazole-4-yl) propyloxycarbamide,
- N-(4-aminobenzyl)-3-(1H-imidazole-4-yl) propyloxycarbamide,
- N-(3-nitrophenyl)-3-(1H-imidazole-4-yl)propyloxycarbamide,
- 3-(1H-imidazole-4-yl)propanoic acid 3-cyclohexylpropyl ester,
- N-(2-nitrophenyl)-3-(1H-imidazoIe-4-yl)propyloxycarbamide,
- N-(4-fluorophenyl)-3-(1H-imidazole-4-yl)propyloxycarbamide,
- N-2-(phenylethyl)-3-(1H-imidazole-4-yl)propyloxycarbamide,
- N-(4-fluorobenzyl)-3-(1H-imidazole-4-yl)propyloxycarbamide,
- N-(4-bromophenyl)-3-(1H-imidazole-4-yl)propyloxicarbomide,
- N-(4-chlorobenzyl-3-(1H-imidazole-4-yl)propyloxycarbamide,
- N-(3-iodophenyl)-3-(1H-imidazole-4-yl)propyloxycarbamide,
- N-(2-iodophenyl)-3-(1H-imidazole-4-yl)propyloxycarbamide,
- N-(4-iodophenyl)-3-(1H-imidazole-4-yl)propyloxycarbamide,
- N-(3-phenylpropyl)-3-(1H-imidazole-4-yl)propyloxycarbamide,
- N-(4-trifluoromethylbenzyl)-3-(1H-imidazole-4-yl)propyloxycarbamide,
- N-benzyl-N-methyI-3-(1H-imidazole-4-yl)propyloxycarbamide,
- N-benzyl-N-isopropyl-3-(1H-imidazole-4-yl)propyloxycarbamide,
- 3-(4-chlorophenyl)propyl-3-(1H-imidazole-4-yl)propyl ether,
- (4-chlorophenyl)methyl-3-(1H-imidazole-4-yl)propyl ether,
- cyclohexylmethyl-(1H-imidazole-4-yl)methyl ether,
- 3-(4-fluorophenyl)propyl-3-(1H-imidazole-4-yl)propyl ether,
- p-nitrophenyl-3-(1H-imidazole-4-yl)-2-transpropenoate,
- 2-[1H-imidazole-4-yl]methylthio-5-nitropyridine,
- 2-(2-[1H-imidazole-4-yl]ethylthio)-5-nitropyridine,
- 2-(2-[1H-imidazole-4-yl]ethyl)-5-thiopyridine,
- 2-(2-[1H-imidazole-4-yl]ethyl)-thio-1H-imidazole,
- 4-[2-(4-nitrophenoxy)ethyl]-1H-imidazole,
- 4-[2-(4-carbomethoxyphenoxy)ethyl-1H-imidazole,
- 4-[2-(4-cyanophenoxy)ethyl]-1H-imidazole,
- 4-[2-(4-acetylphenoxy)ethyl]-1H-imidazole,
- 4-[2-(4-ethoxycarbonyl)phenoxy)ethyl]-1H-imidazole,
- 4-[2-(3-nitrophenoxy)ethyl]-1H-imidazole,
- 4-[2-(4-methoxyphenoxy)ethyl]-1H-imidazole,
- 4-[2-(4-propylphenoxy)ethyl]-1H-imidazole,
- 4-[2-(4-bromophenoxy)ethyl]-1H-imidazole,
- 4-[2-(3,5-dichlorophenoxy)ethyl]-1H-imidazole,
- 4-[2-(2,3,4,5,6-pentafluorophenoxy)ethyl]-1H-imidazole,
- 4-[2-(4-trifluoromethylphenoxy)ethyl]-1H-imidazole,
- 4-[2-(4-ethylphenoxy)ethyl]-1H-imidazole,
- 4-[2-(3-cyanophenoxy)ethyl]-1H-imidazole,
- 4-[2-(2-cyanophenoxy)ethyl-1H-imidazole,
- 4-[2-(2-naphthyloxy)ethyl]-1H-imidazole,
- 4-[2-(1-naphthyloxy)ethyl]-1H-imidazole,
- 4-[2-(4-nitrophenylthio)ethyl]-1H-imidazole,
- 4-[3-(4-fluorophenoxy)propyl-1H-imidazole,
- 4-[3-(4-cyanophenoxy)propyl-1H-imidazole,
- 4-[3-(4-trifluoromethylphenoxy)propyl-1H-imidazole.

9. 4-[2-(4-benzoylphenoxy)ethyl]-1H-imidazole.

10. A process for preparing compounds according to claim 1, having the formula 1A or 1B, wherein X represents -OCO- and wherein chain A, chain B and Y have the meanings given in claim 1, characterized in that an acid chloride having the formula
ClCO-(chain B)-Y
or
ClCO-Y
is reacted with an alcohol, occurring as a chlorohydrate, having the formula

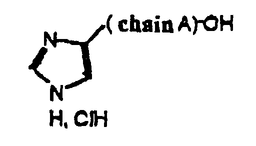

11. A process for preparing compounds according to claim 1, having the formula 1A or 1B, wherein X represents -CO-O- and wherein chain A, chain B and Y have the meanings given in claim 1, characterized in that an acid having the formula 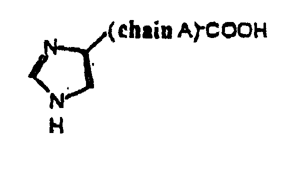 is reacted with an alcohol having the formula
HO ― (Chain B) ― Y
or
HO ― Y
in the presence of thionyl chloride.

12. A process for preparing compounds according to claim 1, having the formula 1A or 1B, wherein X represents -OCONH- and wherein chain A, chain B and Y have the meanings given in claim 1, characterized in that an alcohol, occurring as a chlorohydrate, having the formula 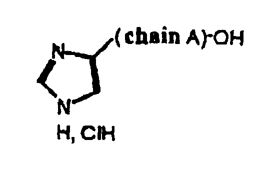 is reacted with an isocyanate having the formula
OCN-(chain B)-Y
or
OCN ― Y
in the presence of an apolar solvent.

13. A process for preparing compounds according to claim 1, having the formula 1A or 1B, wherein X represents -O- and wherein chain A, chain B and Y have the meanings given in claim 1, characterized in that an alcoholate having the formula 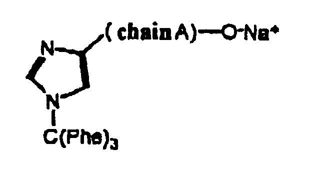 wherein Phe denotes the phenyl radical,
is reacted with a halogenated compound having the formula
Hal ― (chain B) ― Y
or
Hal ― Y
wherein Hal denotes a halogen,
in the presence of a neutral solvent
and the -C(Phe)₃ group is then split off by an acid solution.

14. A process for preparing compounds according to claim 1 having the formula 1A or 1B, wherein X represents -O- and wherein chain A, chain B and Y have the meanings given in claim 1, characterized in that a halogenated compound occurring as chlorohydrate, having the formula 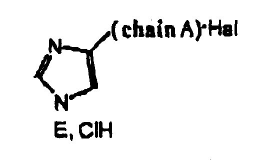 wherein Hal denotes a halogen, is reacted with an alcohol having the formula
HO ― (chain B) ― Y
or
HO ― Y

15. A process for preparing compounds according to claim 1, having the formula 1B, wherein X represents -O-, chain A has the meaning given in claim 1 and Y represents a possibly substituted phenyl group, characterized in that an alcohol having the formula 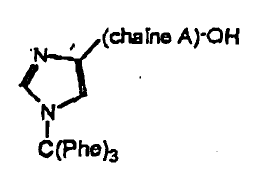 is reacted with a phenolic compound having the formula 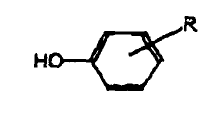 wherein R represents the substituent of the phenyl group Y, in the presence of triphenylphosphine and of a diethylazodicarboxylate in a solvent and characterized in that the -C(Phe)₃ group is split off by treatment with an acid solution.

16. A process for preparing compounds according to claim 1, having the formula 1A or 1B, wherein X represents -S- and wherein chain A, chain B and Y have the meanings given in claim 1, characterized in that an isothiourea having the formula 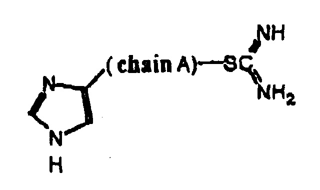 is reacted with a halogenated compound having the formula
Hal ― (chain B) ― Y
or
Hal ― Y
where Hal denotes a halogen, in the presence of a solvent.

17. A process for preparing compounds according to claim 1, having the formula 1A or 1B, wherein X represents -S- and wherein chain A, chain B and Y have the meanings given in claim 1, characterized in that an alcohol having the formula 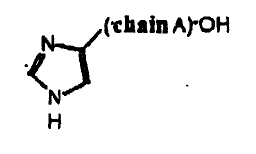 is reacted with a compound having the formula
SH ― (chain B) ― Y
or
SH ― Y
in the presence of a halogenated hydracid or
by reacting a halogenated compound having the formula 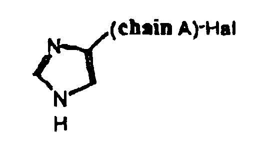 wherein Hal denotes a halogen, with a compound having the formula
SH ― (chain B) ― Y
or
SH ― Y
in the presence of a base.

18. A process for preparing compounds according to claim 1, having the formula 1A or 1B, wherein X represents -SO- and which chain A, and B and Y having the meanings given in claim 1, characterized in that the compound having the formula 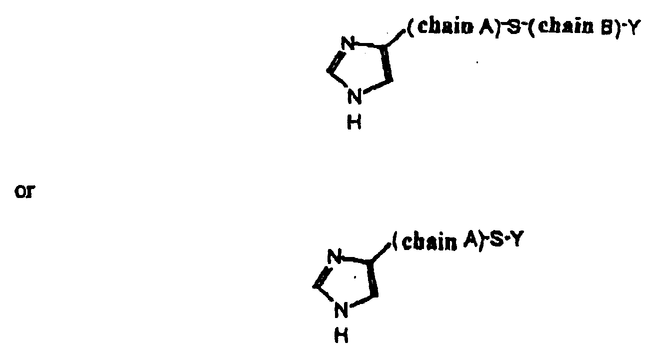 is treated with a base, in the presence of a solvent and then with an oxidizing agent.

19. A process for preparing compounds according to claim 1, having the formula 1A or 1B, wherein X represents -CO- and wherein chain A, chain B and Y have the meanings given in claim 1, characterized in that a compound having the formula 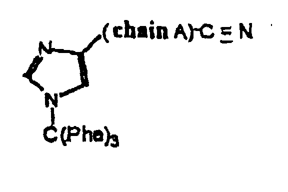 wherein Phe denotes the phenyl radical, is reacted with
Hal ― Mg ― (chain B) ― Y
or
Hal ― Mg ― Y
where Hal denotes a halogen, in the presence of a solvent, and characterized in that the product thus obtained is then hydrolysed.

20. Use of a compound having the formula 1A or 1B 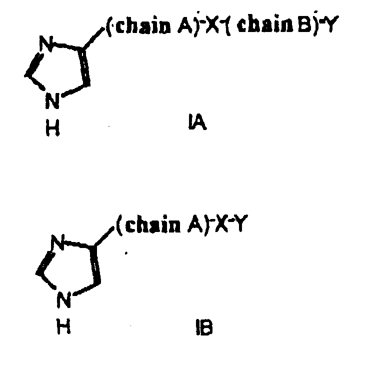 wherein
chain A represents a straight or branched, saturated or unsaturated hydrocarbon chain containing 1 to 6 carbon atoms, wherein said saturated hydrocarbon chain may be interrupted by a sulphur atom,
X represents an oxygen or a sulphur atom, -O-CO-, -CO-O-, -OCONH-, -OCON-(C₁-C₃ alkyl)-, -OCONH-CO, -CONH-, -SO-, -CO-, -CHOH-,
chain B represents a straight alkylene chain -(CH₂)ₙ-, n being a whole number which may vary between 0 and 5 or a branched alkylene chain containing from 2 to 8 carbon atoms, and wherein said alkylene chain may be interrupted by one or several oxygen or sulphur atoms, or a -(CH₂)ₘ-O- or -(CH₂)ₘ-S- chain where m is a whole number of 1 or 2,
Y represents a straight or branched alkyl group containing 1 to 8 carbon atoms; a cycloalkyl containing 3 to 6 carbon atoms; a bicycloalkyl group; a cycloalkenyl group; a naphthyl group; a phenyl group either unsubstituted or mono- or polysubstituted by a halogen atom, an alkyl group containing not more than 6 carbon atoms, a CF₃, CN, COCH₃, COOR₁ or OR₁ group, where R₁ represents an alkyl group containing not more than 6 carbon atoms, an NO₂ or NR₂R₃ group, where R₂ and R₃ represent a hydrogen atom and/or an alkyl radical containing not more than 6 carbon atoms; a 5 or 6-member heterocyclic radical containing one or two heteroatorns selected from the group consisting of nitrogen and sulphur atoms, said heterocyclic radical being unsubstituted or mono- or polysubstituted by an NO₂, CF₃, CH₃, NH₂ group, a halogen atom or the COOCH₃ group; or a bicyclic radical resulting from the fusion of a benzene ring with a heterocycle as defined previously,
as well as their pharmaceutically acceptable salts, their hydrates, their hydrated salts, the polymorphic crystalline structures and the tautomeric forms of these compounds
for the manufacture of a medicine acting as an antagonist of histamine H₃ receptors.

21. Use according to claim 20, characterized in that the compound 1A or 1B is as defined according to any one of claims 2 to 8.

22. Use of a compound having the general formula 1A or 1B according to either of claims 20 and 21 for the manufacture of a medicine controlling the synthesis of histamine, notably with sedative, sleep regulatory, anti-epileptic, psychotonic, anti-depressant, anti-allergic, anti-secretory or anti-ulcerous effects.

23. A medicine acting as an antagonist of histamine H₃ receptors, containing, as active principle, a compound of the general formula 1A or 1B, according to any one of claims 1 to 8, possibly mixed with a therapeutically acceptable vehicle or excipient.

24. A medicine according to claim 23, containing as active principle, a compound selected from the group defined in claim 8.

25. Medicines controlling the synthesis of histamine, notably with sedative, sleep regulatory, anti-epileptic, psychotonic, anti-allergic, anti-secretory or anti-ulcerous effects, containing, as active principle, a compound of the general formula 1A or 1B according to any one of claims 1 to 8, possibly mixed with a therapeutically acceptable vehicle or excipient.

26. A medicine according to claim 25, containing as active principle a compound selected from the group defined in claim 8.

27. Use of a compound according to claim 9 for the manufacture of a medicine acting as an antagonist of hastamine H₃ receptors.

## Patentansprüche

1. Verbindungen der allgemeinen Formel IA oder IB 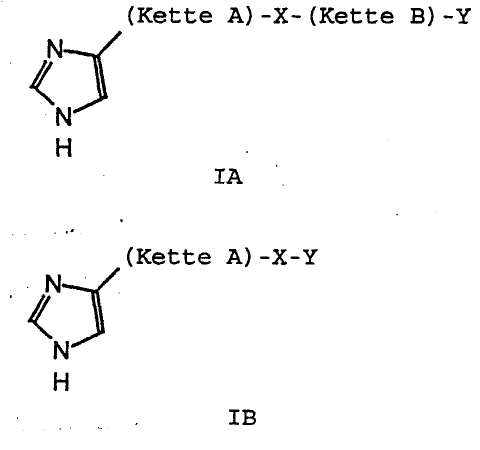 in der
- die Kette A eine gerade oder verzweigte, gesättigte oder ungesättigte Kohlenwasserstoffkette mit 1 bis 6 Kohlenstoffatomen darstellt, wobei die gesättigte Kohlenwasserstoffkette durch ein Schwefelatom unterbrochen sein kann,
- X ein Sauerstoff- oder Schwefelatom, -OCO-, -COO-, -OCONH-, OCON(C₁-C₃-Alkyl), -OCONHCO-, -CONH-, -SO-, -CO- oder -CHOH- darstellt,
- die Kette B eine gerade Alkylenkette -(CH₂)ₙ- darstellt, wobei n eine ganze Zahl zwischen 0 und 5 ist, oder eine verzweigte Alkylenkette mit 2 bis 8 Kohlenstoffatomen, wobei die Alkylenkette durch ein oder mehrere Sauerstoff- oder Schwefelatome unterbrochen sein kann, oder eine Kette -(CH₂)ₘ-O- oder -(CH₂)ₘ-S-, in der m eine ganze Zahl von gleich 1 oder 2 ist,
- Y folgendes darstellt: eine gerade oder verzweigte Alkylgruppe mit 1 bis 8 Kohlenstoffatomen; ein Cycloalkyl mit 3 bis 6 Kohlenstoffatomen; eine Bicycloalkylgruppe; eine Cycloalkenylgruppe; eine Naphtylgruppe; eine Phenylgruppe, die nicht substituiert oder mono- oder polysubstituiert ist durch ein Halo-genatom, eine Alkylgruppe mit höchstens 6 Kohlenstoffatomen, eine Gruppe CF₃, CN, COCH₃, COOR₁ oder OR₁, wobei R₁ eine Alkylgruppe mit höchstens 6 Kohlenstoffatomen, eine Gruppe NO₂ oder NR₂R₃ darstellt, wobei R₂ und R₃ ein Wasserstoffatom und/oder einen Alkylrest mit höchstens 6 Kohlenstoffatomen darstellt; einen heterocyclischen Rest mit 5 oder 6 Seitenketten mit einem oder zwei Heteroatomen, die aus den Stickstoff- und Schwefelatomen ausgewählt sind, wobei dieser heterocyclische Rest nicht substituiert oder durch eine Gruppe NO₂, CF₃, CH₃, NH₂, ein Halogenatom oder die Gruppe COOCH₃ mono- oder polysubstituiert ist; oder einen bicyclischen Rest, der durch Ansetzen eines Benzolrings an einen Heterocyclus der oben genannten Art gebildet ist,
- mit der Maßgabe, daß, wenn X ein Sauerstoffatom und die Kette A die Gruppe -(CH₂)₂- darstellt, Y in der Formel IB nicht eine substituierte Phenylgruppe darstellen kann;
- mit der Maßgabe, daß, wenn X die Gruppe -CONH- und die Kette A die Gruppe -(CH₂)₂- darstellt, Y in der Formel IB nicht eine nicht substituierte oder substituierte Phenylgruppe darstellen kann;
- mit der Maßgabe, daß, wenn X die Gruppe -CO- und die Kette A die Gruppe -(CH₂)₂- darstellt, Y in der Formel IB nicht folgendes darstellen kann: einen Naphtylrest oder einen nicht substituierten oder substituierten Phenylrest, einen nicht substituierten oder substituierten heterocyclischen Rest mit 5 Seitenketten, der Schwefel als Heteroatom hat, einen bicyclischen Rest, der sich aus dem Ansetzen eines Benzolrings an einen Heterocyclus mit 5 oder 6 Seitenketten ergibt, der als Heteroatome Stickstoff- und/oder Schwefelatome besitzt;
- mit der Maßgabe, daß, wenn X die Gruppe -CO- und A eine Methylengruppe darstellt, B und Y nicht gleichzeitig - C(CH₃)₂-(CH₂)ₚ-
(p = 0 oder 1) und eine Naphtylgruppe oder eine substituierte oder nicht substituierte Phenylgruppe darstellen können,
- sowie ihre pharmazeutisch akzeptablen Salze, ihre Hydrate, ihre hydratisierten Salze, die polymorphen kristallinen Strukturen und die tautomeren Formen dieser Verbindungen.

2. Verbindungen nach Anspruch 1, dadurch gekennzeichnet, daß die Kette A eine gerade Alkylenkette -(CH₂)ₙ- ist, wobei n eine ganze Zahl zwischen 1 und 6 ist.

3. Verbindungen nach einem der Ansprüche 1 und 2, dadurch gekennzeichnet, daß die Kette A eine durch einen oder mehrere Methyl- oder Ethylreste substituierte Alkylenkette ist.

4. Verbindungen nach einem der Ansprüche 1 bis 3, dadurch gekennzeichnet, daß Y eine Cycloalkylgruppe darstellt, die aus Cyclopentyl, Cyclohexyl oder einer Bicycloalkylgruppe ausgewählt ist.

5. Verbindungen nach einem der Ansprüche 1 bis 3, dadurch gekennzeichnet, daß Y eine mono- oder polysubstituierte Phenylgruppe darstellt.

6. Verbindungen nach einem der Ansprüche 1 bis 3, dadurch gekennzeichnet, daß Y einen heterocyclischen Rest darstellt, der ausgewählt ist aus einem Pyridylrest, einem Pyridyl-N-oxyd-Rest oder einem Pyrazinylrest, der nicht substituiert ist oder mono- oder polysubstituiert ist durch NO₂, CF₃, CH₃, NH₂, einem Halogen, COOCH3, oder einem Imidazolylrest oder einem Thiazylrest.

7. Verbindungen nach einem der Ansprüche 1 bis 3, dadurch gekennzeichnet, daß Y einen bicyclischen Rest darstellt, der sich durch Kondensation eines Benzolrings und eines Heterocyclus der in Anspruch 1 beschriebenen Art ergibt.

8. Verbindungen nach einem der Ansprüche 1 bis 7, dadurch gekennzeichnet, daß sie ausgewählt sind aus:
- N-(3-Phenylpropyl)-3-(1H-imidazol-4-yl)propanamid,
- 3-(1H-imidazol-4yl)propylester der 3-cyclopentylpropansäure,
- 3-(1H-Imidazol-4-yl)propylester der Benzoesäure,
- 3-(1H-Imidazol-4-yl)propylester der 4-iodobenzoesäure,
- 3-(1H-Imidazol-4-yl)propylester der 3-iodobenzoesäure,
- 3-(1H-Imidazol-4-yl)propylester der 3-iodo-4-methylbenzoesäure,
- 3-(1H-Imidazol-4-yl)propylester der 3-(4-iodophenyl)propylsäure,
- 3-(1H-Imidazol-4-yl)propylester der 4-amino-3,5-diiodobenzoesäure,
- 3-(1H-Imidazol-4-yl)propylester der 4-(4-iodophenyl)isobutansäure,
- 3-(1H-Imidazol-4-yl)propylester der 2-(4-iodophenyl)ethansäure,
- 3-(1H-Imidazol-4-yl)propylester der 4-phenylbutansäure,
- N-Benzyl-3-(1H-imidazol-4-yl)propyloxycarbamid,
- N-Cyclohexylmethyl-3-(1H-imidazol-4-yl)propyloxycarbamid,
- 3-(Cyclohexylpropyl)-3-(1H-imidazol-4-yl)propylether,
- 3-(3,4-Difluorphenyl)propyl-3-(1H-imidazol-4-yl)propylether,
- 3-(4-Bromphenyl)propyl-3-(1H-imidazol-4-yl)propylether,
- 3-(3-Trifluorethylphenyl)propyl-3-(1H-imidazol-4-yl)propylether,
- 1-Naphtylmethyl-3-(1H-imidazol-4-yl)propylether,
- (4-Iodophenyl)methyl-3-(1H-imidazol-4-yl)propylether,
- 4-Phenylbutyl-3-(1H-imidazol-4-yl)propylether,
- (3-Phenylpropyl)-3-(1H-imidazol-4-yl)propylether,
- (3-Phenylpropyl)-3-(1H-imidazol-4-yl)propylthioether,
- 4-[(4-Nitrobenzythio)methyl]-1H-imidazol,
- (3-Phenylpropyl-3-(1H-imidazol-4-yl)propylsulfoxid,
- 1-(1H-Imidazol-4-yl)-7-phenylheptan-4-on,
- 1-(1H-Imidazol-4-yl)-7-phenylheptan-4-ol,
- N-Benzoyl-3-(1H-imidazol-4-yl)propyloxycarbamid,
- N-Cyclobutylmethyl-3-(1H-imidazol-4-yl)propyloxycarbamid,
- N-Cyclopropylmethyl-3-(1H-imidazol-4-yl)propyloxycarbamid,
- (R)-(+)-N-1-Phenylethyl-3-(1H-imidazol-4-yl)propyloxycarbamid,
- (S)-(-)-1-Phenylethyl-3-(1H-imidazol-4-yl-)propyloxycarbamid,
- N-Cyclohexyl-3-(1H-imidazol-4-yl)propyloxycarbamid,
- N-Phenyl-3-(1H-imidazol-4-yl)propyloxycarbamid,
- N-(4-Methylphenyl)-3-(1H-imidazol-4-yl)propyloxycarbamid,
- N-(4-Trifluormethylphenyl)-3-(1H-imidazol-4-yl)propyloxycarbamid,
- N-(3-Trifluormethylphenyl)-3-(1H-imidazol-4-yl)propyloxycarbamid,
- N-(2-Trifluormethylphenyl)-3-(1H-imidazol-4-yl)propyloxycarbamid,
- N-(2-Phenylethyl)-2-(1H-imidazol-4-yl)ethoxycarbamid,
- N-(4-Nitrobenzyl)-3-(1H-imidazol-4-yl)propyloxycarbamid,
- N-(4-Aminobenzyl)-3-(1H-imidazol-4-yl)propyloxycarbamid,
- N-(3-Nitrophenyl)-3-(1H-imidazol-4-yl)propyloxycarbamid,
- 3-Cyclohexylpropylester der 3-(1H-Imidazol-4-yl)propansäure,
- N-(2-Nitrophenyl)-3-(1H-imidazol-4-yl)propyloxycarbamid,
- N-(4-Fluorphenyl)-3-(1H-imidazol-4-yl)propyloxycarbamid,
- N-2-(Phenylethyl)-3-(1H-imidazol-4-yl)propyloxycarbamid,
- N-(4-Fluorbenzyl)-3-(1H-imidazol-4-yl)propyloxycarbamid,
- N-(4-Bromphenyl)-3-(1H-imidazol-4-yl)propyloxycarbamid,
- N-(4-Chlorbenzyl)-3-(1H-imidazol-4-yl)propyloxycarbamid,
- N-(3-Iodophenyl)-3-(1H-imidazol-4-yl)propyloxycarbamid,
- N-(2-Iodophenyl)-3-(1H-imidazol-4-yl)propyloxycarbamid,
- N-(4-Iodophenyl)-3-(1H-imidazol-4-yl)propyloxycarbamid,
- N-(3-Phenylpropyl)-3-(1H-imidazol-4-yl)propyloxycarbamid,
- N-(4-Trifluormethylbenzyl)-3-(1H-imidazol-4-yl)propyloxycarbamid,
- N-Benzyl-N-methyl-3-(1H-imidazol-4-yl)propyloxycarbamid,
- N-Benzyl-N-isopropyl-3-(1H-imidazol-4-yl)propyloxycarbamid,
- 3-(4-Chlorphenyl)propyl-3-(1H-imidazol-4-yl)propylether,
- (4-Chlorophenyl)methyl-3-(1H-imidazol-4-yl)propylether,
- Cyclohexylmethyl-(1H-imidazol-4-yl)methylether,
- 3-(4-Fluorphenyl)propyl-3-(1H-imidazol-4-yl)propylether,
- p-Nitrophenyl-3-(1H-imidazol-4-yl)-2-transpropenoat,
- 2-[1H-Imidazol-4-yl]methylthio-5-nitropyridin,
- 2-(2-[1H-Imidazol-4-yl]ethylthio)-5-nitropyridin,
- 2-(2-[1H-Imidazol-4-yl]ethyl)-5-thiopyridin,
- 2-(2-[1H-Imidazol-4-yl]ethyl)-thio-1H-imidazol,
- 4-[2-(4-Nitrophenoxy)ethyl]-1H-imidazol,
- 4-[2-(4-Carbomethoxyphenoxy)ethyl]-1H-imidazol,
- 4-[2-(4-Cyanophenoxy)ethyl]-1H-imidazol,
- 4-[2-(4-Acetylphenoxy)ethyl]ethyl]-1H-imidazol,
- 4-[2-(4-Ethoxycarbonyl)phenoxy)ethyl]-1H-imidazol,
- 4-[2-(3-Nitrophenoxy)ethyl]-1H-imidazol,
- 4-[2-(4-Methoxyphenoxy)ethyl]-1H-imidazol,
- 4-[2-(4-Propylphenoxy)ethyl]-1H-imidazol,
- 4-[2-(4-Bromophenoxy)ethyl]-1H-imidazol,
- 4-[2-(3,5-Dichlorphenoxy)ethyl]-1H-imidazol,
- 4-[2-(2,3,4,5,6-Pentafluorphenoxy)ethyl]-1H-imidazol,
- 4-[2-(4-Trifluormethylphenoxy)ethyl]-1H-imidazol,
- 4-[2-(4-Ethylphenoxy)ethyl]-1H-imidazol,
- 4-[2-(3-Cyanophenoxy)ethyl]-1H-imidazol,
- 4-[2-(2-Cyanophenoxy)ethyl]-1H-imidazol,
- 4-[2-(2-Naphtyloxy)ethyl]-1H-imidazol,
- 4-[2-(1-Naphtyloxy)ethyl]-1H-imidazol,
- 4-[2-(4-Nitrophenylthio)ethyl]-1H-imidazol,
- 4-[3-(4-Fluorphenoxy)propyl-1H-imidazol,
- 4-[3-(4-Cyanophenoxy)propyl-1H-imidazol
- 4-[3-(4-Trifluormethylphenoxy)propyl-1H-imidazol.

9. 4-[2-(4-Benzoylphenoxy)ethyl]-1H-imidazol.

10. Verfahren zur Herstellung der Verbindungen nach Anspruch 1 der Formel IA oder IB, in denen X für -OCO- steht und die Kette A, die Kette B und Y die in Anspruch 1 angegebenen Bedeutungen haben, dadurch gekennzeichnet, daß man ein Säurechlorid der Formel
ClCO-(Kette B)-Y
oder
ClCO-Y
mit einem in Form von Chlorhydrat vorliegenden Alkohol der Formel 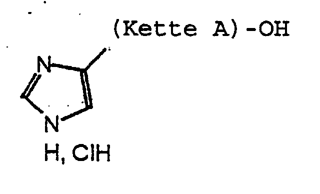 reagieren läßt.

11. Verfahren zur Herstellung der Verbindungen nach Anspruch 1 der Formel IA oder IB, in denen X für -CO-O- steht und die Kette A, die Kette B und Y die in Anspruch 1 angegebenen Bedeutungen haben, dadurch gekennzeichnet, daß man eine Säure der Formel 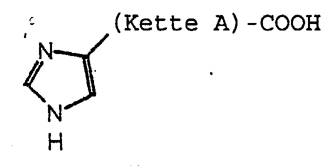 mit einem Alkohol der Formel
HO-(Kette B)-Y
oder
HO-Y
in Gegenwart von Thionylchlorid reagieren läßt.

12. Verfahren zur Herstellung der Verbindungen nach Anspruch 1 der Formel IA oder IB, in denen X für -OCONH-steht und die Kette A, die Kette B und Y die in Anspruch 1 angegebenen Bedeutungen haben, dadurch gekennzeichnet, daß man einen in Form von Chlorhydrat vorliegenden Alkohol der Formel 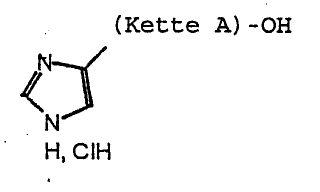 mit einem Isocyanat der Formel
OCN-(Kette B)-Y
oder
OCN-Y
in Gegenwart eines apolaren Lösungsmittels reagieren läßt.

13. Verfahren zur Herstellung der Verbindungen nach Anspruch 1 der Formel IA oder IB, in denen X für -O- steht und die Kette A, die Kette B und Y die in Anspruch 1 angegebenen Bedeutungen haben, dadurch gekennzeichnet, daß man einen Alkohol der Formel 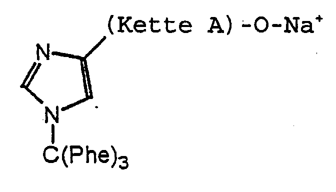 worin Phe den Phenylrest bezeichnet,
in Gegenwart eines neutralen Lösungsmittels
mit einer halogenierten Verbindung der Formel
Hal-(Kette B)-Y
oder
Hal-Y,
worin Hal ein Halogen bedeutet, reagieren läßt und anschließend die Gruppe -C(Phe)₃ durch eine saure Lösung abspaltet.

14. Verfahren zur Herstellung der Verbindungen nach Anspruch 1 der Formel IA oder IB, in denen X für -O- steht und die Kette A, die Kette B und Y die in Anspruch 1 angegebenen Bedeutungen haben, dadurch gekennzeichnet, daß man eine in Form von Chlorhydrat vorliegende halogenierte Verbindung der Formel 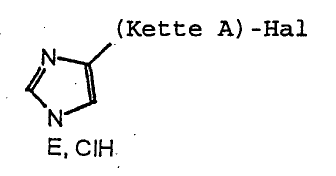 worin Hal ein Halogen bedeutet, mit einem Alkohol der Formel
HO-(Kette B)-Y
oder
HO-Y
reagieren läßt.

15. Verfahren zur Herstellung der Verbindungen nach Anspruch 1 der Formel IB, in der X für -O- steht, die Kette A die in Anspruch 1 angegebene Bedeutung hat und Y eine ggf. substituierte Phenylgruppe darstellt, dadurch gekennzeichnet, daß man einen Alkohol der Formel 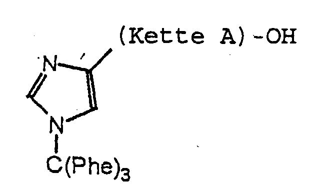 mit einer Phenolverbindung der Formel 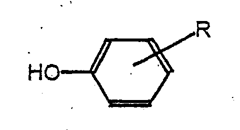 in der R den Substituent der Phenylgruppe in Y darstellt, in Gegenwart von Triphenylphosphin und Diethylazodicarboxylat in einem Lösungsmittel reagieren läßt und daß man die Gruppe -C(Phe)₃ durch Behandlung mit einer sauren Lösung abspaltet.

16. Verfahren zur Herstellung der Verbindungen nach Anspruch 1 der Formel IA oder IB, in denen X für -S- steht und die Kette A, die Kette B und Y die in Anspruch 1 angegebenen Bedeutungen haben, dadurch gekennzeichnet, daß man einen Isothioharnstoff der Formel 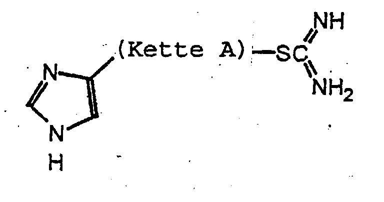 mit einer halogenierten Verbindung der Formel
Hal-(Kette B)-Y
oder
Hal-Y,
worin Hal ein Halogen bedeutet, in Gegenwart eines Lösungsmittels reagieren läßt.

17. Verfahren zur Herstellung der Verbindungen nach Anspruch 1, der Formel IA oder IB, in denen X für -S- steht und die Kette A, die Kette B und Y die in Anspruch 1 angegebenen Bedeutungen haben, dadurch gekennzeichnet, daß man einen Alkohol der Formel 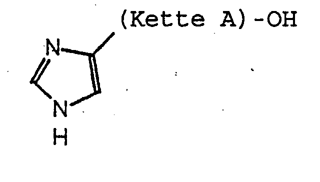 mit einer Verbindung der Formel
SH-(Kette B)-Y
oder
SH-Y
in Gegenwart eines halogenierten Hydrazids reagieren läßt oder eine halogenierte Verbindung 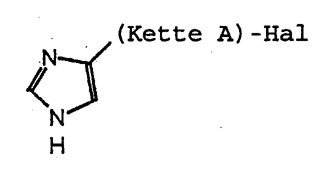 worin Hal ein Halogen bedeutet, mit einer Verbindung der Formel
SH-(Kette B)-Y
oder
SH Y
in Gegenwart einer Base reagieren läßt.

18. Verfahren zur Herstellung der Verbindungen nach Anspruch 1 der Formel IA oder IB, in denen X für -SO- steht und die Kette A, die Kette B und Y die in Anspruch 1 angegebenen Bedeutungen haben, dadurch gekennzeichnet, daß man eine Verbindung der Formel 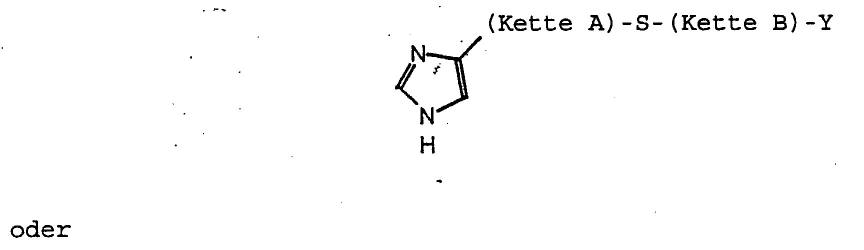 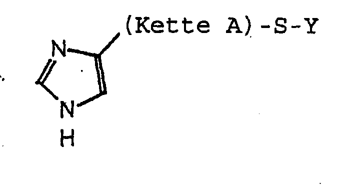 in Gegenwart eines Lösungsmittels mit einer Base und dann mit einem Oxidationsmittel behandelt.

19. Verfahren zur Herstellung der Verbindungen nach Anspruch 1 der Formel IA oder IB, in denen X für -CO- steht und die Kette A, die Kette B und Y die in Anspruch 1 angegebenen Bedeutungen haben, dadurch gekennzeichnet, daß man eine Verbindung der Formel 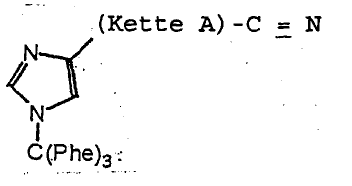 worin Phe den Phenylrest bedeutet, in Gegenwart eines Lösungsmittels mit
Hal-Mg-(Kette B)-Y
oder
Hal-Mg-Y,
worin Hal ein Halogen bedeutet, reagieren läßt und dann das erhaltene Produkt hydrolysiert.

20. Verwendung einer Verbindung der Formel IA oder IB 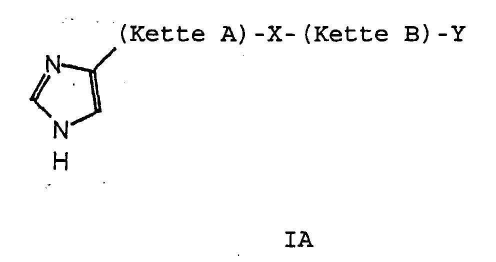 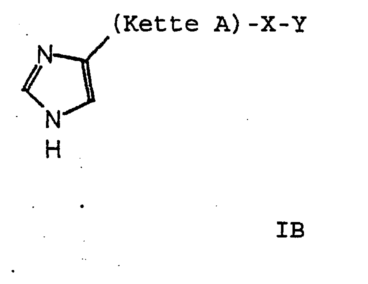 in der
- die Kette A eine gerade oder verzweigte, gesättigte oder ungesättigte Kohlenwasserstoffkette mit 1 bis 6 Kohlenstoffatomen darstellt, wobei die gesättigte Kohlenwasserstoffkette durch ein Schwefelatom unterbrochen sein kann,
- X ein Sauerstoff- oder Schwefelatom, -O-CO-, -CO-O-, -OCONH-, OCON-(C₁-C₃-Alkyl)-, -OCONH-CO-, -CONH-, -SO-, -CO- oder -CHOH- darstellt,
- die Kette B eine gerade Alkylenkette -(CH₂)ₙ- darstellt, wobei n eine ganze Zahl zwischen 0 und 5 ist, oder eine verzweigte Alkylenkette mit 2 bis 8 Kohlenstoffatomen, wobei die Alkylenkette durch ein oder mehrere Sauerstoff- oder Schwefelatome unterbrochen sein kann, oder eine Kette -(CH₂)ₘ-O- oder -(CH₂)ₘ-S-, in der m eine ganze Zahl von gleich 1 oder 2 ist,
- Y folgendes darstellt: eine gerade oder verzweigte Alkylgruppe mit 1 bis 8 Kohlenstoffatomen; ein Cycloalkyl mit 3 bis 6 Kohlenstoffatomen; eine Bicycloalkylgruppe; eine Cycloalkenylgruppe; eine Naphtylgruppe; eine Phenylgruppe, die nicht substituiert oder mono- oder polysubstituiert ist durch ein Halogenantom, eine Alkylgruppe mit höchstens 6 Kohlenstoffatomen, eine Gruppe CF₃, CN, COCH₃, COOR₁ oder OR₁, wobei R₁ eine Alkylgruppe mit höchstens 6 Kohlenstoffatomen, eine Gruppe NO₂ oder NR₂R₃ darstellt, wobei R₂ und R₃ ein Wasserstoffatom und/oder einen Alkylrest mit höchstens 6 Kohlenstoffatomen darstellt; einen heterocyclischen Rest mit 5 oder 6 Seitenketten mit einem oder zwei Heteroatomen, die aus den Stickstoff- und Schwefelatomen ausgewählt sind, wobei dieser heterocyclische Rest nicht substituiert oder durch eine Gruppe NO₂, CF₃, CH₃, NH₂, ein Halogenatom oder die Gruppe COOCH₃ mono- oder polysubstituiert ist; oder einen bicyclischen Rest, der durch Ansetzen eines Benzolrings an einen Heterocyclus der oben genannten Art gebildet ist,
- sowie ihrer pharmazeutisch akzeptablen Salze, ihrer Hydrate, ihrer hydrierten Salze, der polymorphen kristallinen Strukturen und der tautomeren Formen dieser Verbindungen
- für die Herstellung eines Arzneimittels, das als Antagonist der H₃-Rezeptoren des Histamins wirkt.

21. Verwendung nach Anspruch 20, dadurch gekennzeichnet, daß die Verbindung IA oder IB so beschaffen ist, wie in einem der Ansprüche 2 bis 8 definiert ist.

22. Verwendung einer Verbindung der allgemeinen Formel IA oder IB nach einem der Ansprüche 20 und 21 für Herstellung eines Arzneimittels zur Steuerung der Histaminsynthese insbesondere mit sedativer, schlafregulierender, antikonvulsiver, psychostimulierender, antidepressiver, antiallergischer, antisektretorischer oder antiulkeröser Wirkung.

23. Arzneimittel, das als Antagonist der H₃-Rezeptoren des Histamins wirkt und als Wirkstoff eine Verbindung der allgemeinen Formel IA oder IB gemäß einem der Ansprüche 1 bis 8 enthält, ggf. verbunden mit einem therapeutisch akzeptablen Träger oder Grundmaterial.

24. Arzneimittel nach Anspruch 23, das als Wirkstoff eine Verbindung enthält, die aus der in Anspruch 8 genannten Gruppe ausgewählt ist.

25. Arzneimittel zur Steuerung der Histaminsynthese insbesondere mit sedativer, schlafregulierender, antikonvulsiver, psychostimulierender, antiallergischer, antisekretorischer oder antiulkeröser Wirkung, die als Wirkstoff eine Verbindung der allgemeinen Formel IA oder IB nach einem der Ansprüche 1 bis 8 enthalten, ggf. verbunden mit einem therapeutische akzeptablem Träger oder Grundmaterial.

26. Arzneimittel nach Anspruch 25, das als Wirkstoff eine Verbindung enthält, die aus der in Anspruch 8 genannten Gruppe ausgewahlt ist.

27. Verwendung der Verbindung nach Anspruch 9 für die Herstellung eines Arzneimittels, das als Antagonist der H₃-Rezeptoren des Histamins wirkt.
